(19) Europäisches Patentamt
European Patent Office
Office européen des brevets

(11) Numéro de publication : **0 429 366 B1**

(12) # FASCICULE DE BREVET EUROPEEN

(45) Date de publication du fascicule du brevet : **16.03.94 Bulletin 94/11**

(51) Int. Cl.$^5$ : **C07D 209/44,** A61K 31/40, C07D 409/06, C07D 401/06, C07D 401/12, C07D 409/12

(21) Numéro de dépôt : **90403299.2**

(22) Date de dépôt : **22.11.90**

(54) **Nouveaux dérivés de l'isoindolone, leur préparation et les compositions pharmaceutiques qui les contiennent.**

(30) Priorité : **23.11.89 FR 8915406**

(43) Date de publication de la demande : **29.05.91 Bulletin 91/22**

(45) Mention de la délivrance du brevet : **16.03.94 Bulletin 94/11**

(84) Etats contractants désignés : **AT BE CH DE DK ES FR GB GR IT LI LU NL SE**

(56) Documents cités :
**DE-A- 2 259 498**
**US-A- 4 042 707**

(73) Titulaire : **RHONE-POULENC SANTE**
**20, avenue Raymond Aron**
**F-92165 Antony Cédex (FR)**

(72) Inventeur : **Dubroeucq, Marie-Christine**
**13, Villa Malleville**
**F-95880 Enghien Les Bains (FR)**
Inventeur : **Moutonnier, Claude**
**3, Rue Auguste Rodin**
**F-92350 Le Plessis Robinson (FR)**
Inventeur : **Peyronel, Jean-François**
**6, Parc d'Ardenay**
**F-91120 Palaiseau (FR)**
Inventeur : **Tabart, Michel**
**Tour Athènes, 75, Rue du Javelot**
**F-75013 Paris (FR)**
Inventeur : **Truchon, Alain**
**73, Rue Henri Gorjus**
**F-69004 Lyon (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 429 366 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés de l'isoindolone de formule générale :

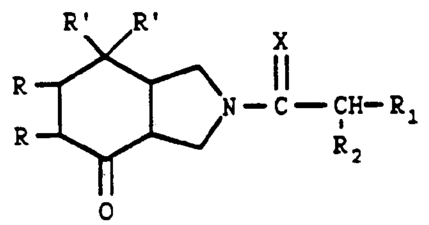

(I)

ainsi que leurs sels lorsqu'ils existent, qui antagonisent les effets de la substance P et sont de ce fait particulièrement intéressants dans les domaines thérapeutiques où cette substance est connue pour intervenir.

Dans le brevet américain 4 042 707 avaient été décrits des produits dérivés de l'isoindole de formule générale:

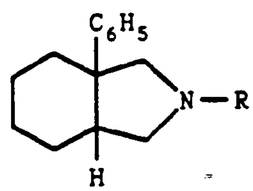

ayant une activité opiacée.

Ces produits n'ont pas d'activité vis à vis de la substance P. Jusqu'à présent, malgré les recherches mises en oeuvre et malgré l'intérêt suscité [M.R. Hanley, TINS, (5) 139 (1982)], il n'avait pas été découvert de produit agissant spécifiquement sur la substance P et ayant une structure non peptidique, c'est pourquoi les dérivés de l'isoindolone de formule générale (I) présentent un intérêt considérable.

Dans la formule générale (I) :

- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3
- le symbole X représente un atome d'oxygène, de soufre ou un radical N-$R_3$ pour lequel $R_3$ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces radicaux pouvant elles même porter un substituant dialcoylamino ou phényle) ou substitué par des radicaux phényle, phényle substitué (par halogène, alcoyle, alcoyloxy ou dialcoylamino), naphtyle, thiényle, furyle, pyridyle ou imidazolyle] ou un radical dialcoylamino,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 5 à 6 chaînons pouvant contenir un autre hétéroatome choisi parmi l'oxygène, le soufre ou l'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre et
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino.

Il est entendu que les radicaux alcoyle ou acyle cités ci-dessus contiennent 1 à 4 atomes de carbone en chaîne droite ou ramifiée.

Lorsque R' porte un substituant halogène, ce dernier peut être choisi parmi le chlore ou le fluor.

Lorsque $R_1$ ou $R_3$ contient un atome d'halogène, ce dernier peut être choisi parmi le chlore, le brome, le fluor ou l'iode.

Lorsque $R_1$ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle.

Par ailleurs, les produits de formule générale (I) présentent différentes formes stéréoisomères, il est entendu que les dérivés de l'isoindolone de forme (3aR,7aR) à l'état pur, ou sous forme de mélange des formes cis (3aRS,7aRS), entrent dans le cadre de la présente invention. De plus, lorsque le symbole $R_2$ est autre que l'atome d'hydrogène, la chaîne substituée sur l'isoindolone présente un centre chiral, il est entendu que les formes énantiomères et leurs mélanges entrent aussi dans le cadre de la présente invention.

Selon l'invention les dérivés de l'isoindolone de formule générale (I) peuvent être obtenus par action de l'acide de formule générale :

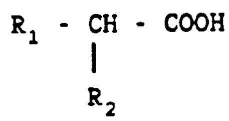

(II)

ou d'un dérivé réactif de cet acide, dans lequel $R_1$ et $R_2$ sont définis comme précédemment, sur un dérivé de l'isoindole de formule générale :

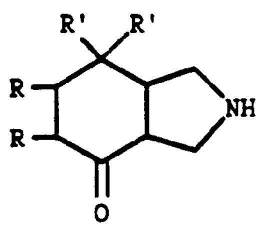

(III)

dans laquelle les symboles R et R'sont définis comme précédemment suivie le cas échéant de la transformation de l'amide obtenu en thioamide ou en une amidine pour laquelle X représente un radical N-$R_3$, $R_3$ ayant la définition donnée précédemment.

Il est entendu que, les radicaux amino, alcoylamino ou carboxy contenus dans $R_1$ et/ou $R_2$ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A.Wiley - Interscience Publication (1981), ou par Mc Omie, Protective Groups in Organic Chemistry, Plenum Press (1973).

A titre d'exemple,

- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque $R_2$ représente un radical hydroxy, il est préférable de protéger préalablement ce radical. La protection s'effectue par exemple par un radical acétoxy, trialcoylsilyle, benzyle, ou sous forme d'un carbonate par un radical -COORa dans lequel Ra est un radical alcoyle ou benzyle.

Lorsque l'on effectue la condensation du produit de formule générale (II) sous sa forme acide (dont le cas échéant les substituants amino, alkylamino, carboxy et/ou hydroxy sont préalablement protégés), on opère généralement en présence d'un agent de condensation tel qu'un carbodiimide [par exemple dicyclohexylcarbodiimide ou (diméthylamino-3 propyl)-1 éthyl-3 carbodiimide], le NN'-carbonyldiimidazole ou l'éthoxy-2 éthoxycarbonyl-1 dihydro-1,2 quinoléine, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacétamide, diméthylformamide par exemple), un nitrile (acétonitrile par exemple), une cétone (acétone par exemple) ou dans un hydrocarbure aromatique comme le toluène par exemple, à une température comprise entre -20 et 40°C, puis on transforme le cas échéant le produit obtenu en un thioamide ou une amidine, et élimine le cas échéant les radicaux protecteurs.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1, nitro-4 phényle, dinitro-2,4 phényle, pentachlorophényle ou phtalimido. La réaction s'effectue généralement à une température comprise entre -40 et +40°C, dans un solvant organique tel qu'un solvant chloré (dichlorométhane, dichloréthane, chloroforme par exemple), un éther (tétrahydrofuranne, dioxanne par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple), ou un carbodiimide, ou bien en milieu hydroorganique, en présence d'un agent alcalin de condensation comme le bicarbonate de sodium, puis on transforme le cas échéant l'amide obtenu en un thioamide ou en une amidine telle que définie précédemment.

La transformation de l'amide de formule générale (I) en un thioamide s'effectue par toute méthode de thionation qui n'altère pas le reste de la molécule.

On opère notamment par action du réactif de Lawesson [bis (méthoxy-4 phényl)-2,4 dithioxo-2,4 dithiadiphosphétane-1,3,2,4], ou par action du pentasulfure de phosphore, dans un solvant organique tel qu'un éther (tétrahydrofuranne, diméthoxy-1,2 éthane, dioxanne par exemple), un hydrocarbure aromatique (toluène par exemple), à une température comprise entre 0°C et la température de reflux du mélange réactionnel.

La transformation de l'amide de formule générale (I) en une amidine pour laquelle X est un radical N-$R_3$ s'effectue, soit directement, soit par l'intermédiaire du thioamide correspondant, en préparant le dérivé de l'isoindolium de formule générale :

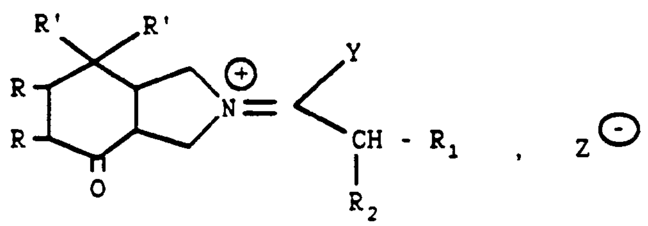

(IV)

dans laquelle R, R', $R_1$ et $R_2$ sont définis comme précédemment et, soit Y représente un atome de chlore, un radical méthoxy ou éthoxy et $Z^-$ représente un ion chlorure, tétrafluoroborate, fluorosulfonate, trifluorométhylsulfonate, méthylsulfate, ou éthylsulfate, soit Y représente un atome de chlore ou un radical méthylthio, éthylthio, benzylthio ou alcoyloxycarbonylméthylthio et $Z^-$ est défini comme ci-dessus ou représente un ion iodure ou bromure, puis en faisant agir une amine de formule générale :

$$R_3 - NH_2 \qquad (V)$$

dans laquelle $R_3$ est défini comme précédemment.

La préparation du dérivé de l'isoindolium de formule générale (IV) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La préparation du dérivé de l'isoindolium de formule générale (IV) dans laquelle Y est un atome de chlore, un radical méthyl (ou éthyl) thio, benzylthio, ou alcoyloxycarbonylméthylthio s'effectue à partir du dérivé de l'isoindolone de formule générale (I) dans laquelle X est un atome de soufre, par action d'un réactif tel que cité précédemment ou par action du bromure ou de l'iodure de méthyle, d'éthyle ou de benzyle. La réaction s'effectue dans un solvant chloré (dichlorométhane, dichloréthane par exemple) ou dans un hydrocarbure aromatique (toluène par exemple), à une température comprise entre 0°C et la température de reflux du mélange réactionnel. Lorsque l'on opère à partir du thioamide de formule générale (I), il est également possible d'utiliser des solvants tels que les éthers, les cétones, les esters ou les nitriles. L'action de l'amine de formule générale (V) sur le dérivé de formule générale (IV) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un

ester (par exemple acétate d'éthyle), dans un solvant aromatique (toluène par exemple) ou dans un mélange de ces solvants, à une température comprise entre -20°C et la température de reflux du mélange réactionnel. Il n'est pas indispensable d'avoir isolé le dérivé de l'isoindolium de formule générale (IV) pour le mettre en oeuvre dans cette réaction.

Selon l'invention les dérivés de l'isoindolone de formule générale (I) dans laquelle $R_1$ et $R_2$ sont définis comme précédemment, à l'exception de représenter ou de porter des substituants hydroxy, amino, alcoyamino, dialcoylamino, ou carboxy, et R, R', $R_3$ et X sont définis comme précédemment, peuvent également être obtenus à partir d'un dérivé de la cyclohexènone de formule générale :

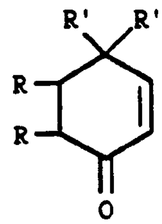

(VI)

dans laquelle R et R' sont définis comme précédemment par action de tristriméthylsilylméthyl-1,3,5 triazine-1,3,5 et d'un fluorure d'acide de formule générale :

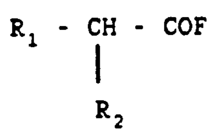

(VII)

dans laquelle $R_1$ et $R_2$ sont définis comme ci-dessus, suivie le cas échéant de la transformation de l'amide obtenu en un thioamide ou en une amidine pour laquelle X représente un radical N-$R_3$ ayant la définition donnée précédemment. La réaction s'effectue généralement dans un solvant organique tel qu'un solvant chloré (dichloréthane, trichloréthane, par exemple) ou dans un hydrocarbure aromatique (toluène, xylène par exemple) à une température comprise entre 80°C et la température de reflux du mélange réactionnel.

La transformation de l'amide en thioamide ou en amidine s'effectuent selon les méthodes décrites précédemment.

Selon l'invention les dérivés de l'isoindolone de formule générale (I) pour lesquels le symbole $R_1$ est un radical alcoyloxyphényle dont la partie alcoyle est substituée ou non et le symbole $R_2$ est autre qu'un radical hydroxy peuvent également être préparés à partir d'un dérivé de l'isoindole de formule générale (I) pour lequel $R_1$ est un radical hydroxyphényle, par action en milieu basique du dérivé halogéné correspondant, de formule générale :

$$Hal - R_4 \qquad (VIII)$$

dans laquelle Hal est un atome d'halogène et $R_4$ est un radical alcoyle éventuellement substitué par un radical hydroxy, ou dialcoylamino dont les parties alcoyle peuvent éventuellement former avec l'atome d'azote auquel elle sont rattachées, un hétérocycle tel que défini dans la formule générale (I).

La réaction s'effectue généralement en présence d'une base telle qu'un hydrure, un hydroxyde de métal alcalin, un alcoolate de métal alcalin, ou un carbonate de métal alcalin, dans un solvant organique tel qu'un amide (diméthyl formamide par exemple), un hydrocarbure aromatique (toluène par exemple), une cétone (butanone par exemple) ou dans un mélange de ces solvants, à une température comprise entre 20°C et la température de reflux du mélange réactionnel.

Lorsque le radical $R_4$ porte un substituant hydroxy, il est entendu que ce dernier est préalablement protégé. La protection et l'élimination du radical protecteur s'effectuent selon les méthodes décrites précédemment.

Selon l'invention, les dérivés de l'isoindolone de formule générale (I) pour lesquels X est un radical N-$R_3$ peuvent également être obtenus à partir du dérivé de l'isoindolone de formule générale (III), par action d'un produit de formule générale :

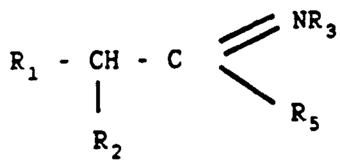 (IX)

éventuellement à l'état de sel, dans laquelle $R_1$, $R_2$ et $R_3$ sont définis comme précédemment et $R_5$ représente un radical alcoyloxy contenant 1 à 4 atomes de carbone en chaîne droite ou ramifiée ou un radical méthylthio, éthylthio, benzylthio ou alcoyloxycarbonylméthylthio, ou si $R_3$ est autre que l'atome d'hydrogène, $R_5$ représente un radical acyloxy contenant 1 à 4 atomes de carbone ou un atome de chlore.

La réaction est mise en oeuvre au moyen du dérivé de formule générale (IX) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile à une température comprise entre 0°C et la température de reflux du mélange réactionnel.

Il est entendu que, dans l'éventualité où les radicaux $R_1$, $R_2$ et/ou $R_3$ du produit de formule générale (IX) portent des substituants pouvant interférer avec la réaction, ces derniers doivent être préalablement protégés.

Les acides de formule générale (II) peuvent être préparés selon les méthodes décrites ci-après dans les exemples, ou par analogie avec ces méthodes.

Le dérivé de l'isoindole de formule générale (III) peut être obtenu à partir du dérivé correspondant de formule générale :

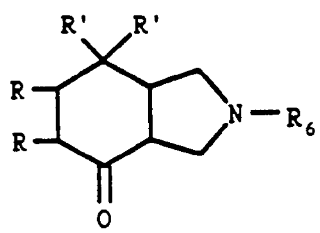 (X)

dans laquelle R et R' sont définis comme précédemment et $R_6$ représente un radical allyle ou un radical de structure - $CR_aR_bR_c$ dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non et les radicaux alcoyle contenant 1 à 4 atomes de carbone en chaîne droite ou ramifiée, par élimination du radical $R_5$, par toute méthode connue qui n'affecte pas le reste de la molécule.

Notamment, lorsque R est un atome d'hydrogène, et lorsque $R_5$ est autre qu'un radical allyle, le groupement $R_6$ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque $R_5$ est un radical benzhydryle ou trityle, l'élimination peut être effectuée par traitement en milieu acide, en opérant à une température comprise entre 0°C et la température de reflux du mélange réactionnel, dans un alcool, dans un éther, dans l'eau ou directement dans l'acide acétique, l'acide formique ou l'acide trifluoroacétique. Le groupement $R_6$ peut être également éliminé en faisant agir le chloroformiate de vinyle, le chloroformiate de chloro-1 éthyle ou le chloroformiate de phényle, en passant intermédiairement par un produit de formule générale :

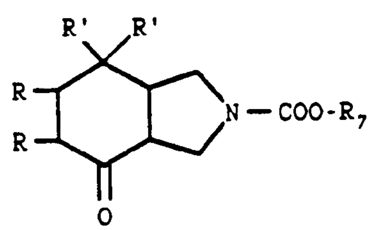 (XI)

dans laquelle R et R' sont définis comme précédemment, et $R_7$ est un radical vinyle, chloro-1 éthyle ou phényle, puis par élimination du radical $COOR_7$ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical $COOR_7$ est effectuée par traitement en milieu acide par exemple par l'acide trifluoroacétique, formique, méthane-sulfonique, p. toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux $COOR_7$ citées précédemment, le dérivé de l'isoindolone de formule générale (III) est obtenu à l'état de sel de l'acide employé qui peut être mis en oeuvre directement dans l'étape ultérieure.

Le dérivé de l'isoindolone de formule générale (X) peut être obtenu par réaction de cycloaddition par action d'un dérivé silylé de formule générale :

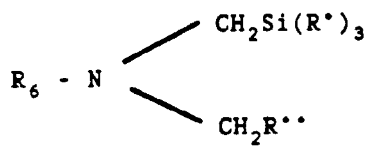

(XII)

dans laquelle $R_6$ est défini comme précédemment, $(R^{\circ})_3$ représente des radicaux alcoyle ou des radicaux alcoyle et phényle et $R^{\circ\circ}$ représente un radical alcoyloxy, cyano ou phénylthio, sur le dérivé de la cyclohexènone de formule générale (VI).

On opère en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

Le dérivé silylé de formule générale (XII) peut être obtenu selon les méthodes décrites par :

- Y. Terao et coll., Chem. Pharm. Bull., 33, 2762 (1985);
- A. Hosomi et coll., Chem. Lett., 1117 (1984) ;
- A. Padwa et coll., Chem. Ber., 119, 813 (1986) ou
- Tetrahedron, 41, 3529 (1985).

Il est entendu que les dérivés de l'isoindolone de formule générale (III) et (X) présentent plusieurs formes stéréoisomères. Lorsque l'on veut obtenir un produit de formule générale (I) de forme (3aR, 7aR), la séparation des formes isomères est mise en oeuvre de préférence au niveau du dérivé de formule générale (III). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique.

Les nouveaux dérivés de l'isoindolone de formule générale (I) peuvent être purifiés le cas échéant par des méthodes physiques telles que la cristallisation ou la chromatographie.

Le cas échéant, les nouveaux dérivés de formule générale (I) pour lesquels les symboles $R_1$ et/ou $R_2$ contiennent des substituants amino ou alcoylamino et/ou X représente un radical $NR_3$, peuvent être transformés en sels d'addition avec les acides. Comme exemples de sels d'addition avec des acides pharmaceutiquement acceptables, peuvent être cités les sels formés avec les acides minéraux (chlorhydrates, brombydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindolone de formule générale (I) peuvent aussi, le cas échéant, lorsque $R_2$ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou

lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les nouveaux dérivés de l'isoindolone selon la présente invention qui antagonisent les effets de la substance P peuvent trouver une application dans les domaines de l'analgésie, de l'inflammation, de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés se sont montrés actifs à des doses comprises entre 20 et 1000 nM.

La substance P est connue pour être impliquée dans un certain nombre de domaines pathologiques :

- Agonists and antagonists of substance P, A.S. Dutta, Drugs of the futur, 12 (8), 782 (1987);
- Substance P and pain : an updating, J.L. Henry, TINS, 3 (4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiovascular Pharmacology, 10 (suppl. 12), 5172 (1987).

Il a notamment été mis en évidence, par l'étude de plusieurs produits que les nouveaux dérivés de l'isoindolone manifestent une activité analgésique dans la technique de Siegmund E. et coll., Proc. Soc. Exp. Biol. Med., 95, 729 (1957).

| Produit de formule générale (I) | $DE_{50}$ mg/kg p.o. |
|---|---|
| Exemple 4 | 42 |
| Exemple 13 | 19 |
| Exemple 27 | 29 |
| Exemple 48 | 4 |
| Exemple 50 | 0,3 |
| Exemple 60 | 38 |

L'étude de plusieurs dérivés de l'isoindolone de formule générale (I) dans la technique de A. Saria et coll., Arch. Pharmacol.,324, 212-218 (1983) a permis de mettre en évidence chez le rat un effet inhibiteur de l'augmentation de la perméabilité capillaire entraîné par la substance P, ce qui témoigne d'une activité anti-inflammatoire :

| Produit de formule générale (I) | $DE_{50}$ mg/kg iv. |
|---|---|
| Exemple 50 | Env. 0,05 |
| Exemple 60 | Env. 1 |

Il a également été démontré au moyen de plusieurs produits que les nouveaux dérivés de l'isoindolone ont un impact au niveau du système nerveux central : ils antagonisent les effets comportementaux induits par un agoniste de la substance P (septide) administré par voie intratéchale chez le rat dans la technique de R.E. Rodriguez et coll., Neuropharmacology, 22, (2), 173-176 (1983) :

| Produit de formule générale (I) | % d'inhibition du comportement à la dose de 10 mg/kg sc |
|---|---|
| Exemple 50 | 50 |
| Exemple 60 | 80 |

L'injection de substance P chez l'animal provoque une hypotension. Les produits étudiés dans la technique de C.A. Maggi et coll., J. Auton. Pharmac., 7, 11-32 (1987) manifestent un effet antagoniste chez le rat vis-à-vis de cette hypotension. Notamment le produit de l'exemple 60 administré à la dose de 0,2 mg/kg i.v./mn, pendant 5 mn, provoque un antagonisme d'environ 50 % de l'hypotension induite par une injection i.v. de 250 µg de septide.
La substance P joue un rôle dans la modulation des processus immunitaires (J.P. Mc Gillis et coll., Fed. Proc., 46(1), 196-199 (1987). La substance P se lie à des récepteurs de cellules humaines en cultures : les lymphoblastes de lignée IM 9 [D.G. Payan et coll., J. of Immunology, 133(6), 3260-5 (1984)]. Les produits selon l'invention déplacent la substance P de ces sites.

| Produit de formule générale (I) | Binding Substance P -$H^3$ (concentration 1 nM) sur homogénats lavés de cellules IM-9 après congélation |
|---|---|
| Exemple 49 | 680 |
| Exemple 76 | 315 |

Enfin la technique de S. ROSELL, Substance P, page 83-88, Raven Press, New-York (1977) mise en oeuvre pour démontrer l'effet antagoniste vis-à-vis de la substance P, montre également une activité antagoniste vis-à-vis des spasmes induits par la substance P ou un agoniste de la substance P.

Par ailleurs, les dérivés de l'isoindolone selon la présente invention ne présentent pas de toxicité, ils se

sont montrés atoxiques chez la souris par voie sous cutanée à la dose de 40 mg/kg ou par voie orale à la dose de 100 mg/kg.

D'un interêt particulier sont les produits de formule générale (I) pour lesquels

- les radicaux R sont identiques et représentent des atomes d'hydrogène ou forment ensemble une liaison,
- les symboles R' sont identiques et représentent des radicaux phényle éventuellement substitués par un atome de fluor ou de chlore ou par un radical méthyle en position 2 ou 3,
- le symbole X représente un atome d'oxygène, de soufre ou un radical N-$R_3$ pour lequel $R_3$ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces radicaux pouvant elles même porter un substituant dialcoylamino ou phényle) ou substitué par des radicaux phényle, phényle substitué (par un atome de fluor ou de chlore, ou par un radical alcoyle, alcoyloxy ou dialcoylamino), naphtyle, thiényle-2, furyle-2, pyridyle ou imidazolyle] ou un radical dialcoylamino,
- le symbole $R_1$ représente un radical phényle éventuellement substitué par un ou plusieurs atomes de fluor, de chlore ou de brome ou radicaux hydroxy, alcoyle pouvant être éventuellement substitués (par des atomes d'halogène ou des radicaux amino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 6 chaînons pouvant contenir un autre atome d'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle saturé à 5 ou 6 chaînons, pouvant contenir en outre un autre atome d'oxygène, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'azote ou le soufre, et
- le symbole $R_2$ représente un atome d'hydrogène ou de fluor ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,

les radicaux alcoyle et acyle cités ci-dessus étant droits ou ramifiés et contenant 1 à 4 atomes de carbone.

Et parmi ces produits, plus spécialement intéressants sont les produits suivants :

l'[imino-1 (méthoxy-2 phényl)-2 éthyl]-2 diphényl-7,7 perhydroisoindolone-4 sous ses formes (3aR,7aR) ou (3aRS,7aRS);

la diphényl-7,7 [(diméthylamino-2 phényl)-2 acétyl]-2 perhydroisoindolone-4 sous ses formes (3aR,7aR) ou (3aRS,7aRS);

la diphényl-7,7 [(méthoxy-2 phényl)-2 propionyl-(R)]-2 perhydroisoindolone-4 sous ses formes (3aR,7aR) ou (3aRS,7aRS);

la {[(diméthylamino-3 propoxy)-2 phényl] acétyl}-2 diphényl-7,7 perhydroisoindolone-4 sous ses formes (3aR,7aR) ou (3aRS,7aRS);

la [(S)-α-carboxy benzylimino-1 (méthoxy-2 phényl)-2 éthyl]-2 diphényl-7,7 perhydroisoindolone-4 sous ses formes (3aR,7aR) ou (3aRS,7aRS).

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

## EXEMPLE 1

A une solution de 1,34 g d'acide phénylacétique dans 30 $cm^3$ de dichlorométhane sec, refroidie à +5°C, on ajoute 1,7 g de N,N'-carbonyidiimidazole. On agite 1 heure à +5°C puis on ajoute une solution de 3,27 g de chlorhydrate de diphényl-7,7 perhydroisoindolone-4 et de 1,7 $cm^3$ de triéthylamine dans 30 $cm^3$ de dichlorométhane. Le mélange réactionnel est agité 1 heure à +5°C, puis 1 heure à 20°C. Le mélange réactionnel est lavé par 50 $cm^3$ d'une solution aqueuse saturée en hydrogénocarbonate de sodium, séché sur sulfate de magnésium, filtré et concentré à sec sous pression réduite (2,7 kPa). Le résidu est cristallisé dans 15 $cm^3$ d'acétonitrile. Les cristaux sont essorés, lavés par 10 $cm^3$ d'oxyde d'isopropyle et séchés. On obtient 2,7 g de diphényl-7,7 (phénylacétyl)-2 perhydroisoindolone-4-(3aRS,7aRS) fondant à 216°C .

Le chlorhydrate de diphényl-7,7 perhydroisoindolone-4-(3aRS,7aRS) peut être préparé selon l'une des méthodes suivantes :

a) A 15 g de palladium sur charbon à 10%, on ajoute 150 g de benzyl-2 diphényl-7,7 perhydroisoindolone-4 (3aRS,7aRS), 1500 $cm^3$ de méthanol et 450 $cm^3$ d'acide chlorhydrique 1N ; le mélange réactionnel est hydrogéné, sous agitation, à température ambiante et sous pression atmosphérique. Après 5 heures de

réaction le volume théorique d'hydrogène a été absorbé ; le mélange réactionnel est filtré, puis concentré à sec sous pression réduite (2,7 kPa) ; le résidu est cristallisé dans 200 $cm^3$ d'éthanol ; les cristaux obtenus sont essorés, lavés par 50 $cm^3$ d'éthanol et séchés. On obtient 110 g de chlorhydrate de diphényl-7,7 perhydroisoindolone-4-(3aRS,7aRS) fondant à 270°C avec décomposition.

Spectre de RMN du proton :

2,03 (Mt, 1H, 1H du H en 5 ou 6) ; 2,3 (Mt, 1H, 1H du - H en 5 ou 6) ; 2.48 (DD, en partie masqué, 1H du $-CH_2-$ en 1) ; 2,69 (DD, 1H, 1H du $-CH_2-$ en 1); 2,8 (Mt, 2H, $-CH_2-$ en 6 ou 5) ; 3,34 (DD, en partie masqué, 1H du $-CH_2-$ en 3) ; 3,5 (Mt, 1H, -CH- en 3a) ; 3,82 (DD, 1H, 1H du $-CH_2-$ en 3) ; 3,95 (Mt, 1H, -CH- en 7a) ; 7,15 à 7,65 (Mt, 10H, aromatiques) ; 9,43 (Mf, 2H, $-NH_2-Cl$).

Spectre infra-rouge (KBr) bandes caractéristiques en $cm^{-1}$:

3600-3300, 3100-3000, 3000-2850, 3100-2400, 1715, 1595, 1580, 1495, 1470, 1445, 775, 750, 705.

b) A une solution de 193 g de benzyl-2 diphényl-7,7 perhydroisoindolone-4 (3aRS,7aRS) dans 1225 $cm^3$ de dichloro-1,2 éthane refroidie à +5°C, on ajoute goutte à goutte en 10 minutes 56 $cm^3$ de chloroformiate de vinyle. Après agitation 30 minutes entre 10 et 20°C le mélange réactionnel est chauffé au reflux pendant 90 minutes, refroidi et concentré à sec sous pression réduite (2,7 kPa puis 1 kPa). La masse cristalline obtenue est battue avec 200 $cm^3$ d'oxyde d'isopropyle froid. Les cristaux obtenus sont essorés, lavés 2 fois par 100 $cm^3$ d'oxyde d'isopropyle et séchés. On obtient 177 g de diphényl-7,7 vinyloxycarbonyl-2 perhydroisoindolone-4-(3aRS,7aRS) fondant à 178°C.

On traite 177 g de diphényl-7,7 vinyloxycarbonyl-2 perhydroisoindolone-4-(3aRS,7aRS) par 1000 $cm^3$ d'une solution 5,7 N d'acide chlorhydrique dans le dioxanne sec pendant 30 minutes à 20°C. La solution est concentrée à sec sous pression réduite (2,7 kPa) et le résidu repris dans 500 $cm^3$ d'éthanol et le mélange réactionnel est agité à 60°C pendant 30 minutes puis refroidi à + 5°C. Les cristaux obtenus sont essores, lavés par 50 $cm^3$ d'éthanol et séchés. On obtient 130 g de chlorhydrate de diphényl-7,7 perhydroisoindolone-4-(3aRS,7aRS) fondant à 270°C.

La benzyl-2 diphényl-7,7 perhydroisoindolone-4-(3aRS,7aRS) peut être préparée de la manière suivante :

A une solution de 155 g de diphényl-4,4 cyclohexène-2 one-1 et de 202 $cm^3$ de N-butoxyméthyl N-triméthylsilylméthyl benzylamine dans 1000 $cm^3$ de dichlorométhane sec, on ajoute 5 gouttes d'acide trifluoroacétique et chauffe le mélange réactionnel ou reflux pendant 45 minutes. On ajoute 50 $cm^3$ de N-butoxyméthyl N-triméthylsilylméthyl benzylamine et 3 gouttes d'acide trifluoroacétique et agite encore 45 minutes au reflux avant d'ajouter de nouveau 25 $cm^3$ de N-butoxyméthyl N-triméthylsilylméthyl benzylamine et 3 gouttes d'acide trifluoroacétique. Le mélange réactionnel est agité au reflux 45 minutes puis traité par 50 g de carbonate de potassium, filtré et concentré à sec sous pression réduite (2,7 kPa). Le résidu est dissous dans 200 $cm^3$ d'oxyde d'isopropyle et la solution refroidie à 0°C pendant 1 heure. Les cristaux sont essorés, lavés 2 fois par 15 $cm^3$ d'oxyde d'isopropyle et séchés pour donner 193 g de benzyl-2 diphényl-7,7 perpydroisoindolone-4-(3aRS,7aRS) sous la forme de cristaux blancs fondant à 132°C.

La N-butoxyméthyl N-triméthylsilylméthyl benzylamine peut être préparée selon la méthode de Y. Terao et coll., Chem. Pharm. Bull., 33, 2762 (1985).

## EXEMPLE 2

A une solution de 10 g de chlorhydrate de diphényl-7,7 perhydroisoindolone-4-(3aR,7aR) dans 80 $cm^3$ de dichlorométhane sec, refroidie à +5°C, on ajoute goutte à goutte une solution de 4 $cm^3$ de chlorure de phénylacétyle et de 8,6 $cm^3$ de triéthylamine dans 10 $cm^3$ de dichlorométhane. Le mélange réactionnel est agité 2 heures à +5°C, puis 20 heures à 20°C. Le mélange réactionnel est traité par 30 $cm^3$ d'une solution aqueuse saturée en hydrogénocarbonate de sodium ; la phase organique est lavée par 50 $cm^3$ d'eau distillée (2 fois), séchée sur sulfate de magnésium, filtrée et concentrée à sec sous pression réduite (2,7 kPa). Le résidu est cristallisé dans 15 $cm^3$ d'acétonitrile. Les cristaux sont essorés, lavés par 10 $cm^3$ d'acétonitrile, par 10 $cm^3$ d'oxyde d'isopropyle et séchés. On obtient 5,7 g de diphényl-7,7 (phénylacétyl)-2 perhydroisoindolone-4-(3aR,7aR), fondant à 173°C ;

$[\alpha]_D^{20}$= -282° (c=1, méthanol).

Le chlorhydrate de diphényl-7,7 perhydroisoindolone-4 (3aR,7aR) peut être préparé de la manière suivante :

A une suspension de 200 g de chlorhydrate de diphényl-7,7 perhydroisoindolone-4 (3aRS,7aRS) dans 2000 $cm^3$ d'acétate d'éthyle, on ajoute lentement, sous agitation, 500 $cm^3$ de soude aqueuse 4N ; l'agitation est poursuivie jusqu'à dissolution du produit de départ. La solution organique est lavée par 250 $cm^3$ d'eau distillée, par 250 $cm^3$ d'une solution aqueuse saturée en chlorure de sodium, séchée sur sulfate de magnésium et filtrée. A la solution ainsi obtenue, on ajoute, sous agitation, une solution de 92,8 g d'acide L-(+) mandélique dans 1000 $cm^3$ d'acétate d'éthyle ; après 4 heures d'agitation, les cristaux obtenus sont essorés, lavés par

250 $cm^3$ d'acétate d'éthyle (2 fois) et séchés. Les cristaux sont repris par 2000 $cm^3$ d'eau distillée ; le mélange est chauffé au reflux, sous agitation, pendant 15 minutes ; les cristaux insolubles sont essorés, lavés par 100 $cm^3$ d'eau distillée (2 fois ) et séchés. Ils sont recristallisés dans un mélange de 1100 $cm^3$ d'acétonitrile et de 500 $cm^3$ d'eau distillée; les cristaux obtenus sont essorés, lavés par 40 $cm^3$ d'acétonitrile (3 fois) et séchés. On obtient 80 g de (L)- mandélate de diphényl-7,7 perhydroisoindolone-4 (3aR,7aR);
$[\alpha]_D^{20}$ = -164° (c=1, méthanol).

A 80 g de (L)-mandélate de diphényl-7,7 perhydroisoindolone-4 (3aR,7aR), on ajoute 400 $cm^3$ de soude aqueuse 1N et 600 $cm^3$ d'acétate d'éthyle ; le mélange est agité à température ambiante jusqu'à dissolution du produit de départ ; la solution organique est lavée par 250 $cm^3$ d'eau distillée, par 250 $cm^3$ d'une solution aqueuse saturée en chlorure de sodium, séchée sur sulfate de magnésium et filtrée ; elle est acidifiée, sous agitation, par addition de 30 $cm^3$ d'acide chlorhydrique 9N ; les cristaux obtenus sont essorés, lavés par 50 $cm^3$ d'acétate d'éthyle (2 fois), par 50 $cm^3$ d'oxyde d'isopropyle et séchés. On obtient 52,3 g de chlorhydrate de diphényl-7,7 perhydroisoindolone-4 (3aR,7aR), fondant à 270°C avec décomposition;
$[\alpha]_D^{20}$= -282° (c=0,5, méthanol).

EXEMPLE 3

A une solution de 4 g de diphényl-7,7 phénylacétyl-2 perhydroisoindolone-4-(3aRS,7aRS) dans 160 $cm^3$ de tétrahydrofuranne, on ajoute 3,95 g de bis(méthoxy-4 phényl)-2,4 dithioxo-2,4 dithiadiphosphétane-1,3,2,4 ; le mélange réactionnel est laissé sous agitation 20 heures à température ambiante.Il est traité par 10 $cm^3$ d'une solution aqueuse d'ammoniaque à 20% et par 30 $cm^3$ d'eau distillée, puis extrait par 150 $cm^3$ d'acétate d'éthyle (2 fois); les phases organiques sont réunies et lavées par 200 $cm^3$ d'eau distillée (2 fois), par 100 $cm^3$ d'une solution aqueuse saturée en chlorure de calcium, séchées sur sulfate de magnésium et concentrées à sec sous pression réduite (2,7 kPa). Le résidu est chromatographié sur une colonne de gel de silice (0,06-0,2 mm, diamètre 3 cm, hauteur 30 cm) en éluant par un mélange de cyclohexane et d'acétate d'éthyle (50/50 en volumes) et en recueillant des fractions de 25 $cm^3$. Les fractions 1 à 6 sont réunies et concentrées à sec sous pression réduite (2,7 kPa). Les cristaux obtenus sont lavés par 20 $cm^3$ d'oxyde d'isopropyle, essorés, et séchés. On obtient 1,5 g de diphényl-7,7 (phényl-2 thioacétyl)-2 perhydroisoindolone-4-(3aRS,7aRS), fondant à 221°C.

EXEMPLE 4

A une solution de 20 g de diphényl-7,7 phénylacétyl-2 perhydroisoindolone-4-(3aRS,7aRS) dans 50 $cm^3$ de dichlorométhane, on ajoute 10,45 g de tétrafluoroborate de triéthyloxonium. Le mélange réactionnel est laissé sous agitation 20 heures à température ambiante. Le précipité obtenu est essoré, lavé par 10 $cm^3$ de dichlorométhane anhydre, par 10 $cm^3$ d'éther anhydre et séché ; on obtient 11,1 g de tétrafluoroborate d'[(éthoxy-1 phényl-2) éthylidène]-2 oxo-4 diphényl-7,7 perhydroisoindolium-(3aRS,7aRS) sous la forme d'une poudre blanche utilisée à l'état brut pour les manipulations suivantes.

A une suspension agitée et refroidie à -20°C de 3,75 g de tetrafluoroborate d'[(éthoxy-1 phényl-2) éthylidène]-2 oxo-4 diphényl-7,7 perhydroisoindolium-(3aRS,7aRS) dans 30 $cm^3$ de dichlorométhane anhydre, on ajoute 8,8 $cm^3$ d'une solution de dichlorométhane 0,8 N en ammoniac. On laisse le mélange réactionnel revenir à température ambiante et on poursuit l'agitation 5 heures. Le mélange réactionnel est traité par 30 $cm^3$ d'une solution aqueuse à 10% de carbonate de potassium. Le précipité présent est éliminé par filtration, puis la phase organique est lavée par 15 $cm^3$ d'eau distillée, par 15 $cm^3$ d'une solution aqueuse saturée en chlorure de sodium, séchée sur sulfate de magnésium et concentrée à sec sous pression réduite (2,7 kPa). Le résidu est recristallisé dans 22 $cm^3$ d'acétonitrile. Les cristaux obtenus sont essorés et séchés. On obtient 1,3 g d'($\alpha$-iminophénéthyl)-2 diphényl-7,7 perhydroisoindolone-4-(3aRS,7aRS ) fondant à 202°C.

EXEMPLE 5

A une suspension agitée de 3 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 5 $cm^3$ de dichlorométhane anhydre, on ajoute 0,43 $cm^3$ de méthylamine. Le mélange réactionnel est laissé sous agitation 3 heures à température ambiante ; il est dilué par 40 $cm^3$ de dichlorométhane et traité par 15 $cm^3$ d'une solution aqueuse contenant 22 g de carbonate de potassium. Le précipité présent est éliminé par filtration, puis la phase organique est lavée par 15 $cm^3$ d'eau distillée, par 10 $cm^3$ d'une solution aqueuse saturée en chlorure de sodium, séchée sur sulfate de magnésium et concentrée à sec sous pression réduite (2,7 kPa). Le résidu est chromatographié sur une colonne de gel d'alumine neutre Péchiney CBT1 (diamètre 3 cm, hauteur 23,5 cm), en éluant par 750 $cm^3$ d'un

mélange de cyclohexane et d'acétate d'éthyle (10/90 en volumes), par 250 $cm^3$ d'acétate d'éthyle et par 500 $cm^3$ d'un mélange d'acétate d'éthyle et de méthanol (95/5 en volumes) et en recueillant des fractions de 25 $cm^3$. Les fractions 41 à 65 sont réunies et concentrées à sec sous pression réduite (2,7 kPa). Le résidu est cristallisé dans 0,5 $cm^3$ d'acétonitrile ; les cristaux obtenus sont essorés, lavés par 0,5 $cm^3$ d'acétonitrile et séchés. On obtient 0,5 g d'(α-méthyliminophénéthyl)-2 diphényl-7,7 perhydroisoindolone-4-(3aRS,7aRS), fondant à 110°C.

EXEMPLE 6

A une suspension agitée de 3 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 5 $cm^3$ de dichlorométhane anhydre, on ajoute 0,3 $cm^3$ d'éthylamine. Le mélange réactionnel est laissé sous agitation 20 heures à température ambiante. Le précipité obtenu est essoré, lavé par 30 $cm^3$ d'éther éthylique et séché. Il est dissous dans 40 $cm^3$ de dichlorométhane, et la solution est lavée par 15 $cm^3$ d'une solution aqueuse contenant 44 g de carbonate de potassium, par 30 $cm^3$ d'eau distillée et par 15 $cm^3$ d'une solution aqueuse saturée en chlorure de sodium ; elle est séchée sur sulfate de magnésium et concentrée à sec sous pression réduite (2,7 kPa). Le résidu est recristallisé dans 15 $cm^3$ d'isopropanol; les cristaux obtenus sont essorés et séchés. On obtient 1,15 g d'(α-éthyliminophénéthyl)-2 diphényl-7,7 perhydroisoindolone-4-(3aRS,7aRS), fondant à 108°C.

EXEMPLE 7

A une suspension agitée et refroidie à +5°C de 3,5 g de tetrafluoroborate d'[(éthoxy-1 phényl-2) éthylidène]-2 oxo-4 diphényl-7,7 perhydroisoindolium-(3aRS,7aRS) préparé selon l'exemple 4 dans 30 $cm^3$ de dichlorométhane anhydre, on ajoute 0,6 $cm^3$ de propylamine.Le mélange réactionnel est laissé sous agitation 20 heures à température ambiante ; il est traité par 30 $cm^3$ d'une solution aqueuse à 10% de carbonate de potassium. Le précipité présent est éliminé par filtration, puis la phase organique est lavée par 15 $cm^3$ d'eau distillée, par 15 $cm^3$ d'une solution aqueuse saturée en chlorure de sodium, séchée sur sulfate de magnésium et concentrée à sec sous pression réduite (2,7 kPa). Le résidu est recristallisé dans 5 $cm^3$ d'acétonitrile. Les cristaux obtenus sont essorés et séchés. On obtient 0,5 g d'(α-propyliminophénéthyl)-2 diphényl-7,7 perhydroisoindolone-4-(3aRS,7aRS), fondant à 86°C.

EXEMPLE 8

A une suspension agitée et refroidie à +5°C de 3,75 g de tetrafluoroborate d'[(éthoxy-1 phényl-2) éthylidène]-2 oxo-4 diphényl-7,7 perhydroisoindolium-(3aRS,7aRS) préparé selon l'exemple 4 dans 30 $cm^3$ de dichlorométhane anhydre, on ajoute 0,77 $cm^3$ de N,N-diméthylamino-2 éthylamine. Le mélange réactionnel est laissé sous agitation 20 heures à température ambiante; il est traité par 30 $cm^3$ d'une solution aqueuse à 10% de carbonate de potassium. Le précipité présent est éliminé par filtration, puis la phase organique est lavée par 15 $cm^3$ d'eau distillée, par 15 $cm^3$ d'une solution aqueuse saturée en chlorure de sodium, séchée sur sulfate de magnésium et concentrée à sec sous pression réduite (2,7 kPa). Le résidu est cristallisé dans 5 $cm^3$ d'acétonitrile. Les cristaux obtenus sont essorés et séchés. Ils sont recristallisés dans 6 $cm^3$ d'acétonitrile. Les cristaux obtenus sont essorés et séchés. On obtient 1,6 g d'[α-(N,N-diméthylamino-2 éthylimino) phénéthyl]-2 diphényl-7,7 perhydroisoindolone-4-(3aRS,7aRS), fondant à 134°C.

EXEMPLE 9

A une suspension de 2,1 g de tétrafluoroborate d'[(éthoxy-1 phényl-2) éthylidène]-2 oxo-4 diphényl-7,7 perhydroisoindolium-(3aRS,7aRS) dans 15 $cm^3$ de dichlorométhane anhydre, on ajoute 0,63 g d'acétylamino-6 hexylamine. Le mélange réactionnel est agité 16 heures à 20°C puis dilué par 50 $cm^3$ d'une solution aqueuse à 20% de carbonate de potassium et 50 $cm^3$ de dichlorométhane, agité puis filtré. La phase organique est lavée par 50 $cm^3$ de solution saturée de chlorure de sodium, séchée sur sulfate de magnésium et concentrée à sec sous pression réduite (2,7 kPa). Le résidu est chromatographié sur une colonne d'alumine CBT1 (diamètre 3 cm, hauteur 24 cm) en éluant sous une pression de 0,5 bar par 1 litre d'acétate d'éthyle puis par un mélange de dichloro-1,2 éthane et d'éthanol (90/10 en volumes) et en recueillant des fractions de 75 $cm^3$. Les fractions 15 à 23 sont réunies et concentrées à sec sous pression réduite (2,7 kPa) pour donner 1,2 g d'[(acétamido-6 hexyl-1)imino-1 phényl-2 éthyl]-2 diphényl-7,7 perhydroisoindolone-4-(3aRS,7aRS) sous la forme d'une meringue blanche.
Spectre de RMN du proton (DMSO-$d_6$):

1,18 (m, 4H, =N-$CH_2$-$CH_2$-<u>$CH_2$</u>-<u>$CH_2$</u>-$CH_2$-$CH_2$-NHAc) ; 1,32 (m, 4H, =N-$CH_2$-<u>$CH_2$</u>-$CH_2$-$CH_2$-<u>$CH_2$</u>-$CH_2$-NHAc ) ; 1,8 (s,3H, -$COCH_3$) ; 1,95 à 2,30 (m , 2H, -$CH_2$- en 5) ; 2,55 à 2,95 (m, 4H, -$CH_2$- en 6 et en 1) ; 2,96 (m, 2H, -<u>$CH_2$</u>-NHAc) ; 3,05 (t, J=7, =N-$CH_2$-) ; 3,24 (m, 1H, H en 3a) ; 3,34 (dd, J=11 et 6, 1H, 1H en 3) ; 3,69 (AB, 2H, $ArCH_2$) ; 4 (m, 1H, H en 7a) ; 4,12 (d, J=11, 1H, 1H en 3) ; 7 à 7,7 (m, 15H, aromatiques) ;7,8 (t, J=5, 1H, -NHAc).
Spectre infra-rouge (bandes caractéristiques en $cm^{-1}$): 3290, 3100-3000, 3000-2825, 1715, 1655, 1615, 1600, 1580, 1495, 1550, 750, 700.

EXEMPLE 10

A une solution de 20 g de diphényl-7,7 (phényl-2 thioacétyl)-2 perhydroisoindolone-4 -(3aRS,7aRS) dans 150 $cm^3$d'acétone on ajoute une solution de 20 g d'iodométhane dans 200 $cm^3$d'acétone . Le mélange réactionnel est agité à +50°C pendant 20 heures . Le précipité obtenu est essoré , lavé à l'oxyde d'isopropyle et séché . On obtient 23,7 g d'iodure de [(méthylthio-1 phényl-2)éthylidène]-2 oxo-4 diphényl-7,7 perpydroisoindolium-(3aRS,7aRS) sous forme de solide blanc.
A une solution de 5 g d'iodure de [(méthylthio-1 phényl-2)éthylidène]-2 oxo-4 diphényl-7,7 perhydroisoindolium-(3aRS,7aRS) dans 50 $cm^3$ de tétrahydrofuranne anhydre, on ajoute goutte à goutte à température ambiante une solution de 0,65 g d'isobutylamine dans 5 $cm^3$ de tétrahydrofuranne. Le mélange réactionnel est laissé sous agitation 20 heures à température ambiante. Le précipité obtenu est essoré et séché. On obtient 4,3 g d'iodhydrate de diphényl-7,7 [α-(isobutylimino)-phénéthyl]-2 perhydroisoindolone-4-(3aRS,7aRS) sous forme de cristaux blancs, fondant à 241°C.

EXEMPLE 12

A une solution de 1,2 g de chlorhydrate de glycinate de méthyle dans 40 $cm^3$ de dichlorométhane anhydre, on ajoute 1,2 $cm^3$ de triéthylamine, puis 5 g de tétrafluoroborate d'[(éthoxy-1 phényl-2) éthylidène]-2 oxo-4 diphényl-7,7 perhydroisoindolium-(3aRS,7aRS) préparé selon l'exemple 4. Le mélange réactionnel est laissé sous agitation 20 heures à température ambiante; il est lavé par 50 $cm^3$ d'eau distillée (2 fois), séché sur sulfate de magnésium et concentré à sec sous pression réduite (2,7 kPa). Le résidu est chromatographié sur une colonne de gel d'alumine neutre Péchiney CBT1 (diamètre 3 cm, hauteur 23,5 cm), en éluant par un mélange de cyclohexane et d'acétate d'éthyle (30/70 en volumes) et en recueillant des fractions de 60 $cm^3$. Les fractions 9 à 23 sont réunies et concentrées à sec sous pression réduite (2,7 kPa). On obtient 0,4 g d'(α-méthoxycarbonylméthyliminophénéthyl)-2 diphényl-7,7 perhydroisoindolone-4-(3aRS,7aRS), fondant à 147°C.

EXEMPLE 13

A une suspension agitée de 6,5 g de tétrafluoroborate d'[(éthoxy-1 phényl-2)éthylidène]-2 oxo-4 diphényl-7,7 perhydroisoindolium-(3aRS,7aRS) préparé selon l'exemple 4 dans 25 $cm^3$ de dichlorométhane anhydre, on ajoute 1,3 $cm^3$ de benzylamine. Le mélange réactionnel est laissé sous agitation 20 heures à température ambiante; il est dilué par 30 $cm^3$ de dichlorométhane et traité par 30 $cm^3$ d'une solution aqueuse contenant 44 g de carbonate de potassium. Le précipité présent est éliminé par filtration, puis la phase organique est lavée par 30 $cm^3$ d'eau distillée (2 fois), par 30 $cm^3$ d'une solution aqueuse saturée en chlorure de sodium, séchée sur sulfate de magnésium et concentrée à sec sous pression réduite (2,7 kPa). Le résidu est chromatographié sur une colonne de gel d'alumine neutre Péchiney CBT1 (diamètre 4,5 cm, hauteur 28 cm), en éluant par de l'acétate d'éthyle et en recueillant des fractions de 60 $cm^3$. Les fractions 2 à 4 sont réunies et concentrées à sec sous pression réduite (2,7 kPa). Le résidu est cristallisé dans 10 $cm^3$ d'acétonitrile ; les cristaux obtenus sont essorés, lavés par 5 $cm^3$ d'acétonitrile et séchés. On obtient 2,5 g d'(α-benzyliminophénéthyl)-2 diphényl-7,7 perhydroisoindolone-4-(3aRS,7aRS), fondant à 165°C.

EXEMPLE 14

A une suspension agitée et refroidie à +5°C de 3,75 g de tetrafluoroborate d'[(éthoxy-1 phényl-2) éthylidène]-2 oxo-4 diphényl-7,7 perhydroisoindolium-(3aRS,7aRS) préparé selon l'exemple 4 dans 40 $cm^3$ de dichlorométhane anhydre, on ajoute 0,9 $cm^3$ de phénéthylamine. Le mélange réactionnel est laissé sous agitation 20 heures à température ambiante ; il est traité par 30 $cm^3$ d'une solution aqueuse à 10% de carbonate de potassium. Le précipité présent est éliminé par filtration, puis la phase organique est lavée par 15 $cm^3$ d'eau distillée, par 10 $cm^3$ d'une solution aqueuse saturée en chlorure de sodium, séchée sur sulfate de magnésium et concentrée à sec sous pression réduite (2,7 kPa). Le résidu est recristallisé dans 100 $cm^3$ d'acétonitrile. Les cristaux obtenus sont essorés et séchés. On obtient 2,05 g d'(α-phénéthyliminophénéthyl)-2 diphényl-7,7 perhydroisoindolone-4-(3aRS,7aRS), fondant à 188°C.

EXEMPLE 15

A une suspension agitée et refroidie à +5°C de 5,25 g de tetrafluoroborate d'[(éthoxy-1 phényl-2) éthylidène]-2 oxo-4 diphényl-7,7 perhydroisoindolium-(3aRS,7aRS) préparé selon l'exemple 4 dans 30 $cm^3$ de dichlorométhane anhydre, on ajoute 1,15 $cm^3$ de phényl-3 propylamine. On laisse ensuite le mélange réactionnel revenir à température ambiante et on poursuit l'agitation 20 heures. Le mélange réactionnel est traité par 20 $cm^3$ d'une solution aqueuse à 10% de carbonate de potassium. Le précipité présent est éliminé par filtration, puis la phase organique est lavée par 25 $cm^3$ d'eau distillée, par 25 $cm^3$ d'une solution aqueuse saturée en chlorure de sodium, séchée sur sulfate de magnésium et concentrée à sec sous pression réduite (2,7 kPa). Le résidu est cristallisé dans 5 $cm^3$ d'acétonitrile. Les cristaux obtenus sont essorés et séchés. Ils sont recristallisés dans 15 $cm^3$ d'acétonitrile ; les cristaux obtenus sont essorés et séchés. On obtient 1,9 y de diphényl-7,7 [α-(phényl-3 propylimino) phénéthyl]-2 perhydroisoindolone-4-(3aRS,7aRS), fondant à 144°C.

EXEMPLE 16

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,7 $cm^3$ d'aminodiphénylméthane. Le mélange réactionnel est laissé sous agitation 20 heures à température ambiante, puis traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est chromatographié sur une colonne de gel d'alumine neutre Péchiney CBT1 (diamètre 3,4 cm, hauteur 28 cm), en éluant par du dichloro-1,2 éthane et en receuillant des fractions de 60 $cm^3$. Les fractions 8 à 10 sont réunies et concentrées à sec sous pression réduite (2,7 kPa). Le résidu est cristallisé dans 2 $cm^3$ d'acétonitrile ; les cristaux obtenus sont essorés, lavés par 2 $cm^3$ d'acétonitrile et séchés. On obtient 0,17 g de diphényl-7,7 (α-diphénylméthyliminophénéthyl)-2 perhydroisoindolone4-(3aRS,7aRS), fondant à 172°C.

EXEMPLE 17

A une suspension agitée de 4,5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1 $cm^3$ de (fluoro-2) benzylamine. Le mélange réactionnel est laissé sous agitation 20 heures à température ambiante;il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans un mélange de 10 $cm^3$ d'acétonitrile et de 5 $cm^3$ d'oxyde d'isopropyle ; les cristaux obtenus sont essorés, lavés par 5 $cm^3$ d'acétonitrile et séchés. On obtient 1,3 g d'[α-(fluoro-2 benzyl) iminophénéthyl]-2 diphényl-7,7 perhydroisoindolone-4-(3aRS,7aRS), fondant à 160°C.

EXEMPLE 18

A une suspension agitée de 4,2 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 0,95 $cm^3$ de (fluoro-3) benzylamine. Le mélange réactionnel est laissé sous agitation 20 heures à température ambiante; il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 10 $cm^3$ d'acétonitrile ; les cristaux obtenus sont essorés, lavés par 5 $cm^3$ d'acétonitrile et séchés. On obtient 1,7 g d'[α-(fluoro-3 benzyl) iminophénéthyl]-2 diphényl-7,7 perhydroisoindolone-4-(3aRS,7aRS), fondant à 156°C.

EXEMPLE 19

A une suspension agitée de 4,2 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 0,95 $cm^3$ de (fluoro-4) benzylamine. Le mélange réactionnel est laissé sous agitation 20 heures à

température ambiante; il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sul fate de magnésium et concentrée à sec sous pression réduite (2,7 kPa). Le résidu est cristallisé dans 10 $cm^3$ d'acétonitrile ; les cristaux obtenus sont essorés, lavés par 5 $cm^3$ d'acétonitrile et séchés. On obtient 2,7 g d'[α-(fluoro-4 benzyl) iminophénéthyl]-2 diphényl-7,7 perhydroisoindolone-4-(3aRS,7aRS), fondant à 180°C.

EXEMPLE 20

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,2 $cm^3$ de (chloro-2) benzylamine. Le mélange réactionnel est laissé sous agitation 20 heures à température ambiante ; il est traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 5 $cm^3$ d'acétonitrile ; les cristaux obtenus sont essorés, lavés par 5 $cm^3$ d'acétonitrile et séchés. On obtient 2,2 g d'[α-(chloro-2 benzyl) iminophénéthyl]-2 diphényl-7,7 perhydroisoindolone-4-(3aRS,7aRS), fondant à 192°C.

EXEMPLE 21

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,2 $cm^3$ de (chloro-4) benzylamine. Le mélange réactionnel est laissé sous agitation 20 heures à température ambiante ; il est traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 20 $cm^3$ d'acétonitrile ; les cristaux obtenus sont essorés, lavés par 10 $cm^3$ d'acétonitrile et séchés. On obtient 2,9 g d'[α-(chloro-4 benzyl) iminophénéthyl]-2 diphényl-7,7 perhydroisoindolone-4-(3aRS,7aRS), fondant à 198°C.

EXEMPLE 22

A une suspension agitée de 4,1 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1 $cm^3$ de (méthoxy-2) benzylamine. Le mélange réactionnel est laissé sous agitation 20 heures à température ambiante ; il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans un mélange de 5 $cm^3$ d'acétonitrile et de 10 $cm^3$ d'oxyde d'isopropyle ; les cristaux obtenus sont essorés, lavés par 5 $cm^3$ d'acétonitrile et séchés. On obtient 1,6 g d'[α-(méthoxy-2 benzyl) iminophénéthyl]-2 diphényl-7,7 perhydroisoindolone-4-(3aRS,7aRS), fondant à 146°C.

EXEMPLE 23

A une suspension agitée de 4,1 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1 $cm^3$ de (méthoxy-3) benzylamine. Le mélange réactionnel est laissé sous agitation 20 heures à température ambiante ; il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 15 $cm^3$ d'acétonitrile ; les cristaux obtenus sont essorés, lavés par 5 $cm^3$ d'acétonitrile et séchés. On obtient 2,7 g d'[α-(méthoxy-3 benzyl) iminophénéthyl]-2 diphényl-7,7 perhydroisoindolone-4-(3aRS,7aRS), fondant à 127°C.

EXEMPLE 24

A une suspension agitée de 4,5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,1 $cm^3$ de (méthoxy-4) benzylamine.Le mélange réactionnel est laissé sous agitation 20 heures à

température ambiante ; il est dilué par 20 $cm^3$ de dichlorométhane et traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 15 $cm^3$ d'acétonitrile ; les cristaux obtenus sont essorés, lavés par 5 $cm^3$ d'acétonitrile et séchés. On obtient 2,7 g d'[α-(méthoxy-4 benzyl) iminophénéthyl]-2 diphényl-7,7 perhydroisoindolone-4-(3aRS,7aRS), fondant à 158°C.

EXEMPLE 25

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,4 g de diméthylamino-4 benzylamine. Le mélange réactionnel est laissé sous agitation 20 heures à température ambiante, puis traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 5 $cm^3$ d'acétonitrile ; les cristaux obtenus sont essorés, lavés par 5 $cm^3$ d'acétonitrile et séchés. On obtient 3,5 g d' [α-(diméthylamino-4 benzylimino) phénéthyl]-2 diphényl-7,7 perhydroisoindolone-4-(3aRS,7aRS), fondant à 186°C.

EXEMPLE 26

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1,4 $cm^3$ d'aminométhyl-1 naphtalène. Le mélange réactionnel est laissé sous agitation 20 heures à température ambiante, puis traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 15 $cm^3$ d'acétonitrile ; les cristaux obtenus sont essorés, lavés par 10 $cm^3$ d'acétonitrile et séchés. On obtient 3,2 g d' [α-(naphtyl-1 méthyl) iminophénéthyl]-2 diphényl-7,7 perhydroisoindolone-4-(3aRS,7aRS), fondant à 192°C.

EXEMPLE 27

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 1 $cm^3$ d'aminométhyl-2 thiophène. Le mélange réactionnel est laissé sous agitation 20 heures à température ambiante, puis traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 5 $cm^3$ d'acétonitrile ; les cristaux obtenus sont essorés, lavés par 5 $cm^3$ d'acétonitrile et séchés. On obtient 2,5 g de diphényl-7,7 [α-(thiényl-2 méthyl) iminophénéthyl]-2 perhydroisoindolone-4(3aRS,7aRS), fondant à 114°C.

EXEMPLE 28

A une suspension agitée de 5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 0,85 $cm^3$ de furfurylamine. Le mélange réactionnel est laissé sous agitation 20 heures à température ambiante, puis traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans 100 $cm^3$ d'éther de pétrole ; les cristaux obtenus sont essorés et séchés. Les cristaux sont recristallisés dans 10 $cm^3$ d'acétonitrile ; les cristaux obtenus sont essorés, lavés par 10 $cm^3$ d'acétonitrile et séchés. On obtient 1,7 g d' [α-(furyl-2 -méthyl)iminophénéthyl]-2 diphényl-7,7 perhydroisoindolone-4-(3aRS,7aRS), fondant à 128°C.

EXEMPLE 29

A une suspension agitée de 4,5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 0,9 $cm^3$ d'aminométhyl-2 pyridine. Le mélange réactionnel est laissé sous laissé sous agitation 20 heures à température ambiante ; il est traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans un mélange de 3 $cm^3$ d'acétonitrile et de 5 $cm^3$ d'oxyde d'isopropyle ; les cristaux obtenus sont essorés, lavés par 5 $cm^3$ d'acétonitrile et séchés. On obtient 2 g de diphényl-7,7 [α-(pyridyl-2 méthyl) iminophénéthyl]-2 perhydroisoindolone-4-(3aRS,7aRS), fondant à 180°C.

EXEMPLE 30

A une suspension agitée de 4,5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 10 $cm^3$ de dichlorométhane anhydre, on ajoute 0,9 $cm^3$ d'aminométhyl-3 pyridine. Le mélange réactionnel est laissé sous laissé sous agitation 20 heures à température ambiante ; il est traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans un mélange de 3 $cm^3$ d'acétonitrile et de 5 $cm^3$ d'oxyde d'isopropyle ; les cristaux obtenus sont essorés, lavés par 5 $cm^3$ d'acétonitrile et séchés. On obtient 1,7 g de diphényl-7,7 [α-(pyridyl-3 méthyl)iminophénéthyl]-2 perhydroisoindolone-4-(3aRS,7aRS), fondant à 102°C.

EXEMPLE 31

A une suspension agitée de 4,5 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 20 $cm^3$ de dichlorométhane anhydre, on ajoute 0,9 $cm^3$ d'aminométhyl-4 pyridine. Le mélange réactionnel est laissé sous laissé sous agitation 20 heures à température ambiante ; il est traité par 20 $cm^3$ d'une solution aqueuse saturée en carbonate de potassium. Le précipité présent est éliminé par filtration, puis la phase organique est séchée sur sulfate de magnésium et concentrée à sec sous pression réduite (2,7 kPa). Le résidu est cristallisé dans un mélange de 5 $cm^3$ d'acétonitrile et de 5 $cm^3$ d'oxyde d'isopropyle ; les cristaux obtenus sont essorés, lavés par 5 $cm^3$ d'acétonitrile et séchés. Ils sont recristallisés dans 5 $cm^3$ d'acétonitrile. Les cristaux obtenus sont essorés et séchés. On obtient 2,3 g de diphényl-7,7 [α-(pyridyl-4 méthyl) iminophénéthyl]-2 perhydroisoindolone-4-(3aRS,7aRS), fondant à 150°C.

EXEMPLE 32

A une suspension agitée de 3,75 g de tétrafluoroborate d'[(éthoxy-1 phényl-2) éthylidène]-2 oxo-4 diphényl-7,7 perhydroisoindolium-(3aRS,7aRS) préparé selon l'exemple 4 dans 40 $cm^3$ de dichlorométhane anhydre, refroidie à +5°C, on ajoute 0,53 $cm^3$ de N,N-diméthylhydrazine. Le mélange réactionnel est agité 1 heure à +5°C, puis 20 heures à température ambiante ; il est traité par 30 $cm^3$ d'une solution aqueuse saturée en carbonate de potassium. Le précipité présent est éliminé par filtration, puis la phase organique est lavée par 30 $cm^3$ d'eau distillée, séchée sur sulfate de magnésium et concentrée à sec sous pression réduite (2,7 kPa). Le résidu est chromatographié sur une colonne de gel d'alumine neutre Péchiney CBT1 (diamètre 3 cm, hauteur 30 cm), en éluant par un mélange de cyclohexane et d'acétate d'éthyle (50/50 en volumes) et en recueillant des fractions de 60 $cm^3$. La fraction 2 est concentrée à sec sous pression réduite (2,7 kPa). Le résidu est recristallisé dans 10 $cm^3$ d'acétonitrile ; les cristaux obtenus sont essorés. Les eaux de recristallisation sont concentrées à sec sous pression réduite (2,7 kPa) ; le résidu est cristallisé dans 5 $cm^3$ d'oxyde d'isopropyle ; les cristaux sont essorés et séchés. On obtient 0,22 g de [(N,N-diméthylhydrazono-1 phényl-2) éthyl]-2 diphényl-7,7 perhydroisoindolone-4-(3aRS,7aRS), fondant à 142°C.

EXEMPLE 33

A une solution de 1,08 g d'acide fluoro-2 phénylacétique dans 30 $cm^3$ de dichlorométhane sec, refroidie à +5°C, on ajoute 1,14 g de N,N'-carbonyldiimidazole. On agite 20 minutes à +5°C puis on ajoute une solution de 2,3 g de chlorhydrate de diphényl-7,7 perhydroisoindolone-4-(3aRS,7aRS) et de 1,98 $cm^3$ de triéthylamine dans 20 $cm^3$ de dichlorométhane. Le mélange réactionnel est agité à 20°C pendant 72 heures, puis lavé par 150 $cm^3$ d'eau (2 fois), séché sur sulfate de magnésium, filtré et concentré à sec sous pression réduite (2,7 kPa). Le solide obtenu est cristallisé dans un mélange de 30 $cm^3$ d'acétonitrile et 50 $cm^3$ d'oxyde d'isopropyle. Les cristaux sont essorés, lavés à l'oxyde d'isopropyle et séchés. On obtient 1,53 g de diphényl-7,7 [(fluoro-2 phényl) acétyl]-2 perhydroisoindolone-4-(3aRS,7aRS) sous la forme de cristaux blancs, fondant à 186°C.

EXEMPLE 34

A une solution de 1,08 g d'acide (fluoro-4 phényl)-2 acétique dans 30 $cm^3$ de dichlorométhane sec, refroidie à +5°C, on ajoute 1,13 g de N,N'-carbonyldiimidazole. On agite 1 heure à +5°C puis on ajoute une solution de 2,3 g de chlorhydrate de diphényl-7,7 perhydroisoindolone-4 et de 0,98 $cm^3$ de triéthylamine dans 40 $cm^3$ de dichlorométhane. Le mélange réactionnel est agité 1 heure à +5°C puis 16 heures à 20°C. Le mélange réactionnel est lavé par 50 $cm^3$ d'eau distillée (3 fois), séché sur sulfate de magnésium, filtré et concentré à sec sous pression réduite (2,7 kPa). Le résidu est recristallisé dans 5 $cm^3$ d'acétonitrile. Les cristaux sont essorés, lavés par 10 $cm^3$ d'oxyde d'isopropyle et séchés. On obtient 2 g de [(fluoro-4 phényl) acétyl]-2 diphényl-7,7 perhydroisoindolone-4-(3aRS,7aRS), fondant à 210°C.

EXEMPLE 35

A une solution de 1,2 g d'acide (difluoro-2,6 phényl)-2 acétique dans 30 $cm^3$ de dichlorométhane sec, refroidie à +5°C, on ajoute 1,13 g de N,N'-carbonyldiimidazole. On agite 45 minutes à +5°C puis on ajoute une solution de 2,3 g de chlorhydrate de diphényl-7,7 perhydroisoindolone-4 (3aRS,7aRS) et de 0,98 $cm^3$ de triéthylamine dans 40 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est cristallisé dans 10 $cm^3$ d'acétate d'éthyle. Les cristaux obtenus sont essorés , lavés avec 10 $cm^3$ d'oxyde d'isopropyle et séchés. On obtient 2,1 g de [(difluoro-2,6 phényl) acétyl]-2 diphényl-7,7 perhydroisoindolone-4-(3aRS,7aRS), fondant à 218°C.

EXEMPLE 36

A une solution de 1,19 g d'acide chloro-2 phénylacétique dans 30 $cm^3$ de dichlorométhane sec, refroidie à +5°C, on ajoute 1,13 g de N,N'-carbonyldiimidazole. On agite 15 minutes à +5°C puis on ajoute une solution de 2,28 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité à 20°C pendant 2,5 heures, puis lavé par 150 $cm^3$ d'eau (2 fois), séché sur sulfate de magnésium, filtré et concentré à sec sous pression réduite (2,7 kPa). Le solide obtenu est cristallisé dans un mélange de 25 $cm^3$ d'acétonitrile et 25 $cm^3$ d'oxyde d'isopropyle. Les cristaux sont essorés, lavés à l'oxyde d'isopropyle et séchés. On obtient 1,9 g de diphényl-7,7 [(chloro-2 phényl) acétyl]-2 perhydroisoindolone-4-(3aRS,7aRS) sous la forme de cristaux blancs, fondant à 206°C.

EXEMPLE 37

A une solution de 1,19 g d'acide chloro-3 phénylacétique dans 30 $cm^3$ de dichlorométhane sec, refroidie à +5°C, on ajoute 1,13 g de N,N'-carbonyldiimidazole. On agite 15 minutes à +5°C puis on ajoute une solution de 2,28 g de chlorhydrate de diphényl-7,7 perhydroisoindolone-4(3aRS,7aRS) et de 1,96 $cm^3$ de triéthylamine dans 20 $cm^3$ de dichlorométhane. Le mélange réactionnel est agité à 20°C pendant 16 heures, puis lavé par 150 $cm^3$ d'eau (2 fois), séché sur sulfate de magnésium, filtré et concentré à sec sous pression réduite (2,7 kPa). La meringue obtenue est cristallisée dans un mélange de 30 $cm^3$ d'acétonitrile et 100 $cm^3$ d'oxyde d'isopropyle. Les cristaux sont essorés, lavés à l'oxyde d'isopropyle et séchés. On obtient 2,2 g de diphényl-7,7 [(chloro-3 phényl) acétyl]-2 perhydroisoindolone-4-(3aRS,7aRS) sous la forme de cristaux blancs, fondant à 179°C.

EXEMPLE 38

A une solution de 1,36 g d'acide (chloro-4 phényl)-2 acétique dans 30 $cm^3$ de dichlorométhane sec, refroidie à +5°C, on ajoute 1,3 g de N,N'-carbonyldiimidazole. On agite 1 heure à +5°C puis on ajoute une solution de 2,62 g de chlorhydrate de diphényl-7,7 perhydroisoindolone-4 (3aRS,7aRS) et de 1,12 $cm^3$ de triéthylamine dans 20 $cm^3$ de dichlorométhane. Le mélange réactionnel est agité 2 heures à +5°C et 16 heures à 20°C ; il est lavé par 50 $cm^3$ d'eau distillée (3 fois), séché sur sulfate de magnésium, filtré et concentré à sec sous pression réduite (2,7 kPa). Le résidu est cristallisé dans un mélange de 5 $cm^3$ d'acétonitrile et de 10 $cm^3$ d'oxyde d'isopropyle. Les cristaux obtenus sont essorés, et séchés. Le résidu est recristallisé dans 40 $cm^3$ d'acétonitrile. Les cristaux obtenus sont essorés, lavés par 10 $cm^3$ d'oxyde d'isopropyle et séchés. On obtient 2,4 g de [(chloro-4 phényl) acétyl]-2 diphényl-7,7 perhydroisoindolone-4-(3aRS,7aRS), fondant à 213°C.

EXEMPLE 39

A une solution de 1,43 g d'acide (dichloro-2,6 phényl)-2 acétique dans 30 $cm^3$ de dichlorométhane sec, refroidie à +5°C, on ajoute 1,13 g de N,N'-carbonyldiimidazole. On agite 30 minutes à +5°C puis on ajoute une solution de 2,3 g de chlorhydrate de diphényl-7,7 perhydroisoindolone-4 (3aRS,7aRS) et de 0,98 $cm^3$ de triéthylamine dans 40 $cm^3$ de dichlorométhane. Le mélange réactionnel est agité à 20°C pendant 20 heures, puis lavé par 50 $cm^3$ d'eau distillée (3 fois), séché sur sulfate de magnésium, filtré et concentré à sec sous pression réduite (2,7 kPa). Le résidu est chromatographié sur une colonne de gel de silice (0,2-0,063 mm, diamètre 1,8 cm, hauteur 23,5 cm ) en éluant par du dichlorométane et en recueillant des fractions de 15 $cm^3$. Les fractions 1 et 2 sont réunies et concentrées à sec sous pression réduite (2,7 kPa). Le résidu est cristallisé dans un mélange de 5 $cm^3$ d'acétonitrile et de 4 $cm^3$ d'oxyde d'isopropyle. Les cristaux obtenus sont essorés, lavés par 10 $cm^3$ d'oxyde d'isopropyle et séchés. On obtient 0,62 g de [(dichloro-2,6 phényl) acétyl]-2 diphényl-7,7 perhydroisoindolone-4-(3aRS,7aRS), fondant à 201°C.

EXEMPLE 40

A une solution de 1,5 g d'acide (bromo-2 phényl)-2 acétique dans 30 $cm^3$ de dichlorométhane sec, refroidie à +5°C, on ajoute 1,13 g de N,N'-carbonyldiimidazole. On agite 45 minutes à +5°C puis on ajoute une solution de 2,3 g de chlorhydrate de diphényl-7,7 perhydroisoindolone-4-(3aRS,7aRS) et de 0,98 $cm^3$ de triéthylamine dans 40 $cm^3$ de dichlorométhane. Le mélange réactionnel est agité 4 heures à 20°C. Le mélange réactionnel est lavé par 50 $cm^3$ d'eau distillée (3 fois), séché sur sulfate de magnésium, filtré et concentré à sec sous pression réduite (2,7 kPa). Le résidu est recristallisé dans 74 $cm^3$ d'acétonitrile. Les cristaux sont essorés, lavés par 10 $cm^3$ d'oxyde d'isopropyle et séchés. On obtient 2,04 g de [(bromo-2 phényl) acétyl]-2 diphényl-7,7 perpydroisoindolone-4-(3aRS,7aRS), fondant à 217°C.

EXEMPLE 41

A une solution de 1,06 g d'acide hydroxy-2 phénylacétique dans 30 $cm^3$ de dichlorométhane sec, refroidie à +5°C, on ajoute 1,14 g de N,N'-carbonyldiimidazole. On agite 30 minutes à +5°C puis on ajoute une solution de 2,23 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité à 20°C pendant 16 heures, puis lavé par 100 $cm^3$ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 4 cm, hauteur 40 cm) en éluant sous une pression de 0,7 bar d'azote par un mélange de cyclohexane et d'acétate d'éthyle (70/30 en volumes) et en recueillant des fractions de 125 $cm^3$. Les fractions 4 à 11 sont réunies et concentrées à sec sous pression réduite (2,7 kPa). Le résidu est cristallisé dans un mélange de 15 $cm^3$ d'acétonitrile et 30 $cm^3$ d'oxyde d'isopropyle. Les cristaux sont essorés, lavés à l'oxyde d'isopropyle et séchés. On obtient 0,8 g de diphényl-7,7 [(hydroxy-2 phényl) acétyl]-2 perhydroisoindolone-4-(3aRS,7aRS) sous la forme de cristaux blancs, fondant à 232°C.

EXEMPLE 42

A une solution de 1,05 g d'acide (méthyl-2 phényl)-2 acétique dans 30 $cm^3$ de dichlorométhane sec, refroidie à +5°C, on ajoute 1,13 g de N,N'-carbonyldiimidazole. On agite 40 minutes à +5°C puis on ajoute une solution de 2,3 g de chlorhydrate de diphényl-7,7 perhydroisoindolone-4-(3aRS,7aRS) et de 0,98 $cm^3$ de triéthylamine dans 20 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 2 cm, hauteur 16 cm) en éluant avec de l'acétate d'éthyle et en recueillant des fractions de 45 $cm^3$. Les fractions 2 à 4 sont réunies et concentrées à sec sous pression réduite (2,7 kPa). Le résidu est recristallisé dans un mélange de 8 $cm^3$ d'acétonitrile et de 7,5 $cm^3$ d'oxyde d'isopropyle. Les cristaux sont essorés, lavés par 10 $cm^3$ d'oxyde d'isopropyle et séchés. On obtient 1,25 g de [(méthyl-2 phényl) acétyl]-2 diphényl-7,7 perhydroisoindolone-4-(3aRS,7aRS), fondant à 196°C.

EXEMPLE 43

A une solution de 1,05 g d'acide (méthyl-3 phényl)-2 acétique dans 30 $cm^3$ de dichlorométhane sec, refroidie à +5°C, on ajoute 1,13 g de N,N'-carbonyldiimidazole. On agite 35 minutes à +5°C puis on ajoute une

solution de 2,3 g de chlorhydrate de diphényl-7,7 perhydroisoindolone-4-(3aRS,7aRS) et de 0,98 $cm^3$ de triéthylamine dans 20 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est cristallisé par dissolution dans 10 $cm^3$ d'acétate d'éthyle bouillant. Les cristaux sont essorés, séchés, et recristallisés dans un mélange de 8 $cm^3$ d'acétonitrile et de 7,5 $cm^3$ d'oxyde d'isopropyle. Les cristaux sont essorés, lavés par 10 $cm^3$ d'oxyde d'isopropyle et séchés. On obtient 1,37 g de [(méthyl-3 phényl) acétyl]-2 diphényl-7,7 perhydroisoindolone-4-(3aRS,7aRS), fondant à 184°C.

EXEMPLE 44

A une solution de 1,43 g d'acide trifluorométhyl-3 phénylacétique dans 30 $cm^3$ de dichlorométhane sec, refroidie à +5°C, on ajoute 1,13 g de N,N'-carbonyldiimidazole. On agite 15 minutes à +5°C puis on ajoute une solution de 2,28 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité à 20°C pendant 2 heures, puis lavé par 150 $cm^3$ d'eau (2 fois), séché sur sulfate de magnésium, filtré et concentré à sec sous pression réduite (2,7 kPa). La meringue obtenue est cristallisée dans un mélange de 30 $cm^3$ d'acétonitrile et 30 $cm^3$ d'oxyde d'isopropyle. Les cristaux sont essorés, lavés à l'oxyde d'isopropyle et séchés. On obtient 2 g de diphényl-7,7 [(trifluorométhyl-3 phényl) acétyl]-2 perhydroisoindolone-4-(3aRS,7aRS) sous la forme de cristaux blancs, fondant à 184°C.

EXEMPLE 45

A une solution de 1,42 g d'acide (trifluorométhyl-2 phényl)-2 acétique dans 30 $cm^3$ de dichlorométhane sec, refroidie à +5°C, on ajoute 1,13 g de N,N'-carbonyldiimidazole. On agite 1 heure 45 minutes à +5°C puis on ajoute une solution de 2,3 g de chlorhydrate de diphényl-7,7 perhydroisoindolone-4 (3aRS,7aRS) et de 0,98 $cm^3$ de triéthylamine dans 40 $cm^3$ de dichlorométhane. Le mélange réactionnel est agité à 20°C pendant 18 heures, puis lavé par 50 $cm^3$ d'eau distillée (3 fois), séché sur sulfate de magnésium, filtré et concentré à sec sous pression réduite (2,7 kPa). Le résidu est cristallisé dans 10 $cm^3$ d'acétonitrile. Les cristaux obtenus sont essorés, lavés par 10 $cm^3$ d'oxyde d'isopropyle et séchés. On obtient 1,68 g de diphényl-7,7 [(trifluorométhyl-2 phényl) acétyl]-2 perhydroisoindolone-4-(3aRS,7aRS), fondant à 207°C.

EXEMPLE 46

A une solution de 0,6 g d'acide (tert-butoxycarbonylaminométhyl-2 phényl acétique dans 20 $cm^3$ de dichlorométhane sec, refroidie à +5°C, on ajoute 0,37 g de N,N'-carbonyldiimidazole. On agite 30 minutes à +5°C puis on ajoute une solution de 0,75 g de chlorhydrate de diphényl-7,7 perhydroisoindolone-4-(3aRS,7aRS) et de 0,6 $cm^3$ de triéthylamine dans 20 $cm^3$ de dichlorométhane. Le mélange réactionnel est agité à 20°C pendant 16 heures, puis lavé par 100 $cm^3$ d'eau (2 fois), séché sur sulfate de magnésium, filtré et concentré à sec sous pression réduite (2,7 kPa). On obtient 1,05 g de [(tert-butoxycarbonylaminométhyl-2 phényl) acétyl]-2 diphényl-7,7 perhydroisoindolone-4-(3aRS,7aRS) sous la forme d'une meringue blanche.

On traite 1 g de [(tert-butoxycarbonylaminométhyl-2 phényl) acétyl]-2 diphényl-7,7 perhydroisoindolone-4-(3aRS,7aRS) par 10 $cm^3$ d'une solution 5,7 N d'acide chlorhydrique dans le dioxanne sec à 20°C pendant 16 heures. Le mélange réactionnel est concentré à sec sous pression réduite (2,7 kPa) et le résidu est concrété par grattage avec de l'oxyde d'isopropyle et précipité à nouveau par refroidissement d'une solution dans 20 $cm^3$ d'acétonitrile bouillant. Le solide est essoré, lavé par de l'oxyde d'isopropyle et séché. Le solide est dissous dans l'eau, la solution est filtrée, alcalinisée à pH 10 par action d'une solution décinormale de soude et extraite par de l'acétate d'éthyle. La phase organique est séchée sur sulfate de magnésium, filtrée et traitée par 1 $cm^3$ de solution 3 N d'acide chlorhydrique dans le dioxanne. Le précipité est essoré, lavé par de l'oxyde d'isopropyle et séché. On obtient 0,5 g de chlorhydrate d'[(aminométhyl-2 phényl) acétyl]-2 diphényl-7,7 perhydroisoindolone-4-(3aRS,7aRS), sous la forme d'un solide blanc crème.

Spectre RMN du proton :

A température ambiante, on observe le mélange des deux rotamères : 2 à 2,35 (Mt, 2H, $-CH_2-$ en 5 ou 6) ; 2,65 à 3,15 (Mt, 4H, $-CH_2-$ en 1 et $-CH_2-$ en 6 ou 5) ; 3,20 à 4,5 (Mt, $-CH_2-$ en 3, -CH - en 3a, -CH - en 7, $-N-CO-CH_2-$ et $-CH_2N-$); 7,05 à 7,7 (Mt, 14 H, aromatiques).

Spectre Infra-rouge (KBr) bandes caractéristiques ($cm^{-1}$):

3600-3250, 3100-3000, 3000-2800, 2750-2250, 1715, 1620, 1600, 1580, 1495, 1475, 1445, 1440, 755, 703.

L'acide (tert-butoxycarbonylaminométhyl-2 phényl) acétique est préparé selon la méthode décrite dans la demande de brevet japonnais 74 24 975.

EXEMPLE 47

A une solution de 1,16 g d'acide méthoxy-2 phénylacétique dans 30 $cm^3$ de dichlorométhane sec, refroidie à +5°C, on ajoute 1,13 g de N,N'-carbonyldiimidazole. On agite 15 minutes à +5°C puis on ajoute une solution de 2,28 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité à 20°C pendant 16 heures, puis lavé par 150 $cm^3$ d'eau (2 fois), séché sur sulfate de magnésium, filtré et concentré à sec sous pression réduite (2,7 kPa). La meringue obtenue est cristallisée dans un mélange de 30 $cm^3$ d'acétonitrile et 30 $cm^3$ d'oxyde d'isopropyle. Les cristaux sont essorés, lavés par 25 $cm^3$ oxyde d'isopropyle et séchés. On obtient 2 g de diphényl-7,7 [(méthoxy-2 phényl) acétyl]-2 perhydroisoindolone-4-(3aRS,7aRS) sous la forme de cristaux blancs, fondant à 163°C.

EXEMPLE 48

A une solution de 5 g de diphényl-7,7 [(méthoxy-2 phényl) acétyl]-2 perhydroisoindolone-4-(3aRS,7aRS) dans 10 $cm^3$ de dichlorométhane, on ajoute 2,35 g de tétrafluoroborate de triéthyloxonium. Le mélange réactionnel est laissé sous agitation 20 heures à température ambiante ; il est alors traité par 100 $cm^3$ d'éther, et le précipité obtenu est essoré, lavé par 100 $cm^3$ d'éther, et séché. On obtient 5,75 g tétrafluoroborate d'[(éthoxy-1 (méthoxy-2 phényl)-2) éthylidène]-2 oxo-4 diphényl-7,7 perhydroisoindolium-(3aRS,7aRS) sous forme d'une poudre jaune utilisée à l'état brut pour la manipulation suivante.

A une suspension agitée et refroidie à -10°C de 5,7 g de tétrafluoroborate d'[(éthoxy-1 (méthoxy-2 phényl)-2) éthylidène]-2 oxo-4 diphényl-7,7 perhydroisoindolium(3aRS,7aRS) dans 15 $cm^3$ de dichlorométhane anhydre, on ajoute 1,3 $cm^3$ d'une solution éthanolique d'ammoniac 5,4N. On laisse ensuite le mélange réactionnel revenir à température ambiante et on poursuit l'agitation 20 heures. Le mélange réactionnel est dilué par 20 $cm^3$ de dichlorométhane et traité par 20 $cm^3$ d'une solution aqueuse à 10% de carbonate de potassium. Le précipité présent est éliminé par filtration, puis la phase organique est lavée par 25 $cm^3$ d'eau distillée, par 25 $cm^3$ d'une solution aqueuse saturée en chlorure de sodium, séchée sur sulfate de magnésium et concentrée à sec sous pression réduite (2,7 kPa). Le résidu est cristallisé dans 10 $cm^3$ d'acétonitrile. Les cristaux obtenus sont essorés, lavés par 10 $cm^3$ d'acétonitrile et séchés. On obtient 1 g d' [imino-1 (méthoxy-2 phényl)-2 éthyl]-2 diphényl-7,7 perhydroisoindolone-4-(3aRS,7aRS) fondant à une température supérieure à 260°C.

EXEMPLE 49

A une solution de 1 g d'acide méthoxy-2 phénylacétique dans 30 $cm^3$ de dichlorométhane sec, refroidie à +5°C, on ajoute 1 g de N,N'-carbonyldiimidazole. On agite 40 minutes à +5°C puis on ajoute une solution de 2 g de chlorhydrate de diphényl-7,7 perhydroisoindolone-4-(3aR,7aR) et de 1,7 $cm^3$ de triéthylamine dans 40 $cm^3$ de dichlorométhane. Le mélange réactionnel est agité à 20°C pendant 16 heures, puis lavé par 50 $cm^3$ d'eau (2 fois), séché sur sulfate de magnésium filtré et concentré à sec sous pression réduite (2,7 kPa). Le résidu est chromatographié sur une colonne de gel de silice (0,2-0,063 mm, diamètre 1,8 cm, hauteur 13 cm) en éluant par de l'acétate d'éthyle et en recueillant des fractions de 25 $cm^3$. Les fractions 3 à 5 sont réunies et concentrées à sec sous pression réduite (2,7 kPa). Le résidu est cristallisé dans un mélange de 5 $cm^3$ d'acétonitrile et 10 $cm^3$ d'oxyde d'isopropyle. Les cristaux sont essorés, lavés par 25 $cm^3$ oxyde d'isopropyle et séchés. On obtient 1,7 g de (-)-diphényl-7,7 [(méthoxy-2 phényl) acétyl]-2 perhydroisoindolone-4-(3aR,7aR) sous la forme de cristaux blancs, fondant à 200°C ;
$[\alpha]_D^{20}$= - 274° (c=0,49 , acide acétique).

EXEMPLE 50

A une solution de 7,7 g de diphényl-7,7 [(méthoxy-2 phényl) acétyl]-2 perhydroisoindolone-4-(3aR,7aR) dans 13 $cm^3$ de dichlorométhane anhydre, on ajoute 4 g de tétrafluoroborate de triéthyloxonium. Le mélange réactionnel est laissé sous agitation 20 heures à température ambiante ; on refoidit ensuite le mélange à -15°C, puis on ajoute 2,6 $cm^3$ d'une solution éthanolique d'ammoniac 5,4N. On laisse le mélange réactionnel revenir à température ambiante et on poursuit l'agitation 5 heures et demi. Le mélange réactionnel est traité par 20 $cm^3$ d'une solution aqueuse de carbonate de potassium à 10% ; le précipité formé est essoré et lavé par 10 $cm^3$ de dichlorométhane. Les phases organiques sont réunies, séchées sur sulfate de magnésium et concentrées à sec sous pression réduite (2,7 kPa). Le résidu est cristallisé dans 10 $cm^3$ d'acétonitrile; les cristaux obtenus sont essorés, lavés par 5 $cm^3$ d'acétonitrile, par 10 $cm^3$ d'oxyde d'isopropyle et séchés. Ils sont ensuite chromatographiés sur une colonne de gel d'alumine neutre Péchiney CBT1 (diamètre 4,5 cm, hauteur 28 cm),

en éluant par 200 $cm^3$ d'un mélange de dichloro-1,2 éthane et de méthanol (98/2 en volumes), puis par un mélange de dichloro-1,2 éthane et de méthanol (90/10 en volumes) et en recueillant des fractions de 25 $cm^3$. Les fractions 8 à 31 sont réunies et concentrées à sec sous pression réduite (2,7 kPa). Le résidu est cristallisé dans 10 $cm^3$ d'acétonitrile ; les cristaux obtenus sont essorés et séchés. On obtient 1,6 g d'[imino-1 (méthoxy-2phényl)-2 éthyl]-2 diphényl-7,7 perhydroisoindolone-4-(3aR,7aR), fondant à 190°C ; $[\alpha]_D^{20}$ = -254° (c=1, méthanol).

EXEMPLE 52

A une solution de 0,63 g d'acide (diméthoxy-2,6 phényl)-2 acétique dans 20 $cm^3$ de dichlorométhane sec, refroidie à +5°C, on ajoute 0,52 g de N,N'-carbonyldiimidazole. On agite 35 minutes à +5°C puis on ajoute une solution de 1,05 g de chlorhydrate de diphényl-7,7 perhydroisoindolone-4-(3aRS,7aRS) et de 0,44 $cm^3$ de triéthylamine dans 25 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est cristallisé dans un mélange de 16 $cm^3$ d'acétonitrile et de 15 $cm^3$ d'oxyde d'isopropyle. Les cristaux sont essorés, lavés par 10 $cm^3$ d'oxyde d'isopropyle et séchés. On obtient 0,89 g de [(diméthoxy-2,6 phényl) acétyl]-2 diphényl-7,7 perhydroisoindolone-4-(3aRS,7aRS), fondant à 224°C.

L'acide (diméthoxy-2,6 phényl)-2 acétique peut être préparé de la manière suivante :

Un mélange de 2,88 g de N-[(diméthoxy-2,6 phényl)-2 thioacétyl] morpholine et de 40 $cm^3$ d'une solution aqueuse de potasse à 10% est chauffé au reflux pendant 12 heures. Après retour à température ambiante, le mélange réactionnel est extrait par 100 $cm^3$ d'acétate d'éthyle ; la phase organique est acidifiée par addition de 25 $cm^3$ d'acide chlorhydrique 4N. La phase aqueuse est extraite par 100 $cm^3$ d'acétate d'éthyle (2 fois). Les phases organiques sont réunies et lavées par 100 $cm^3$ d'eau distillée (3 fois), séchées sur sulfate de magnésium, filtrées et concentrées à sec sous pression réduite (2,7 kPa). Le résidu est recristallisé dans un mélange de 20 $cm^3$ de cyclohexane et de 10 $cm^3$ d'acétate d'éthyle. Les cristaux obtenus sont essorés, lavés par 15 $cm^3$ (2 fois) d'oxyde d'isopropyle et séchés. On obtient 0,63 g d'acide (diméthoxy-2,6 phényl)-2 acétique, fondant à 156°C.

La N-[(diméthoxy-2,6 phényl)-2 thioacétyl] morpholine peut être préparée de la manière suivante :

Un mélange de 39,5 g de diméthoxy-2,6 acétophénone, de 19 $cm^3$ de morpholine et de 7 g de soufre est chauffé au reflux, sous agitation, pendant 24 heures. Après retour à température ambiante, le mélange réactionnel est versé sur de la glace. Le mélange est extrait par 100 $cm^3$ d'acétate d'éthyle ; la solution organique est lavée par 100 $cm^3$ (3 fois) d'eau distillée, séchée sur sulfate de magnésium et concentrée à sec sous pression réduite (2,7 kPa). Le résidu est chromatographié sur une colonne de gel de silice (0,2-0,063 mm, diamètre 4,3 cm, hauteur 48 cm) en éluant par un mélange de cyclohexane et d'acétate d'éthyle (90/10 en volumes) et en recueillant des fractions de 100 $cm^3$. Les fractions 61 à 72 sont réunies et concentrées à sec sous pression réduite (2,7 kPa). On obtient 2,88 g de N[(diméthoxy-2,6 phényl-2 thioacétyl] morpholine utilisé à l'état brut pour les synthèses ultérieures.

EXEMPLE 53

A une solution de 1,34 g d'acide diméthoxy-2,5 phénylacétique dans 30 $cm^3$ de dichlorométhane sec, refroidie à +5°C, on ajoute 1,14 g de N,N'-carbonyldiimidazole. On agite 30 minutes à +5°C puis on ajoute une solution de 2,28 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité à 20°C pendant 16 heures, puis lavé par 200 $cm^3$ d'eau (2 fois), séché sur sulfate de magnésium, filtré et concentré à sec sous pression réduite (2,7 kPa). La meringue obtenue est cristallisée dans un mélange de 20 $cm^3$ d'acétonitrile et 80 $cm^3$ d'oxyde d'isopropyle. Les cristaux sont essorés, lavés par 25 $cm^3$ oxyde d'isopropyle et séchés. On obtient 1,5 g de diphényl-7,7 [(diméthoxy-2,5 phényl) acétyl]-2 perhydroisoindolone-4-(3aRS,7aRS) sous la forme de cristaux blancs, fondant à 209°C.

EXEMPLE 54

A une solution de 0,87 g d'acide méthylthio-2 phénylacétique dans 30 $cm^3$ de dichlorométhane sec, refroidie à +5°C, on ajoute 0,77 g de N,N'-carbonyldiimidazole. On agite 15 minutes à +5°C puis on ajoute une solution de 1,56 g de chlorhydrate de diphényl-7,7 perhydroisoindolone-4-(3aRS,7aRS) et de 1,34 $cm^3$ de triéthylamine dans 30 $cm^3$ de dichlorométhane. Le mélange réactionnel est agité à 20°C pendant 3,5 heures, puis lavé par 100 $cm^3$ d'eau (3 fois), séché sur sulfate de magnésium, filtré et concentré à sec sous pression réduite (2,7 kPa). Le résidu est chromatographié sur une colonne de gel de silice (0,04-0,063 mm, diamètre 5 cm, hauteur 50 cm) en éluant sous une pression de 0,5 bar d'azote par un mélange de cyclohexane et d'acétate d'éthyle (60/40 en volumes) et en recueillant des fractions de 50 $cm^3$. Les fractions 1 à 14 sont réunies et concentrées à sec sous pression réduite (2,7 kPa). Le solide obtenu est recristallisé dans 5 $cm^3$ d'acétonitrile, les cristaux sont essorés, lavés à l'oxyde d'isopropyle et séchés. On obtient 1,29 g de diphényl-7,7 [(méthylthio-2 phényl)acétyl]-2 perhydroisoindolone-4-(3aRS,7aRS) sous la forme de cristaux jaune pâle, fondant à 188°C.

L'acide méthylthio-2 phénylacétique est préparé selon G. Kompa et St. Weckmann (J. Prak. Chem. [2], 138, 123 (1933))

EXEMPLE 55

A une solution de 2,51 g d'acide (tert-butoxycarbonylamino-2 phényl) acétique dans 30 $cm^3$ de dichlorométhane sec, refroidie à +5°C, on ajoute 1,62 g de N,N'-carbonyldiimidazole. On agite 45 minutes à +5°C puis on ajoute une solution de 3,27 g de chlorhydrate de diphényl-7,7 perhydroisoindolone-4-(3aRS,7aRS) et de 2,8 $cm^3$ de triéthylamine dans 30 $cm^3$ de dichlorométhane. Le mélange réactionnel est agité à 20°C pendant 16 heures, puis lavé par 100 $cm^3$ d'eau (3 fois), séché sur sulfate de magnésium, filtré et concentré à sec sous pression réduite (2,7 kPa). Le résidu est chromatographié sur une colonne de gel de silice (0,2-0,063 mm, diamètre 4 cm, hauteur 40 cm) en éluant sous une pression de 0,7 bar d'azote par un mélange de cyclohexane et d'acétate d'éthyle (60/40 en volumes) et en recueillant des fractions de 125 $cm^3$. Les fractions 22 à 34 sont réunies et concentrées à sec sous pression réduite (2,7 kPa) pour donner 1,5 g de [(tert-butoxycarbonylamino-2 phényl) acétyl]-2 diphényl-7,7 perhydroisoindolone-4-(3aRS,7aRS) sous la forme d'une meringue jaune.

On traite 1,5 g de [(tert-butoxycarbonylamino-2 phényl) acétyl]-2 diphényl-7,7 perhydroisoindolone-4-(3aRS,7aRS) par 15 $cm^3$ d'une solution 5,7 N d'acide chlorhydrique dans le dioxanne sec à 20°C pendant 4 heures. Le mélange réactionnel est concentré à sec sous pression réduite (2,7 kPa) et le résidu purifié par dissolution dans 20 $cm^3$ d'acétonitrile et précipitation par 30 $cm^3$ d'oxyde d'isopropyle. Le solide est essoré, lavé par de l'oxyde d'isopropyle et séché. On obtient 1,1 g de chlorhydrate d'[(amino-2 phényl) acétyl]-2 diphényl-7,7 perhydroisoindolone-4-(3aRS,7aRS), sous la forme d'un solide légèrement rosé.

Spectre RMN du proton :

A température ambiante, on observe le mélange des deux rotamères.

2,1 et 2,27 (2Mt, respectivement 1H chacun, $-CH_2-$ en 5 ou 6) ; 2,65 à 3,35 (Mt, 4H, $-CH_2-$ en 6 ou 5 et $-CH_2-$ en 1) ; 3,4 à 3,75 (Mt, 1H du $-CH_2-$ en 3 et -CH- en 3a) ; 3,55 et 3,84 (2S, $-N-CO-CH_2-$) ; 3,9 à 4,2 (Mt, -CH- en 7a) ; 4,15 à 4,4 (Mt, 1H du $-CH_2-$ en 3 ; 7 à 7,7 (Mt, 14H, aromatiques).

Spectre infra-rouge (KBr) bandes caractéristiques ($cm^{-1}$):

3430, 3085, 3055, 3025, 3000-1900, 2965, 2880, 1715, 1630-1520, 1625, 1595, 1580, 1492, 1455, 1445, 755, 703

L'acide (tert-butoxycarbonylamino-2 phényl) acétique peut être obtenu de la manière suivante :

Une solution de 18,1 g d'acide (nitro-2 phényl) acétique dans 120 $cm^3$ de solution normale de soude est hydrogénée en autoclave sous une pression de 5 bars en 2,5 heures à 20°C en présence de 1,5 g de palladium à 3 % sur noir de carbone. La solution du sel de sodium de l'acide (amino-2 phényl) acétique ainsi obtenu est refroidie à +5°C puis traitée par une solution 26,16 g de dicarbonate de ditert-butyle dans 100 $cm^3$ de tétrahydrofuranne puis par 80 $cm^3$ de solution normale de soude. Le mélange réactionnel est agité à 20°C pendant 80 heures, concentré partiellement sous pression réduite (2,7 kPa), dilué par 200 $cm^3$ d'eau et lavé 3 fois par 200 $cm^3$ d'éther éthylique. La phase aqueuse est acidifiée à pH 3 par addition d'acide chlorhydrique 4 N et extraite par de l'acétate d'éthyle (2 fois 200 $cm^3$). Les phases organiques réunies sont lavées par 150 $cm^3$ d'eau (2 fois), séchées sur sulfate de magnésium, filtrées et concentrées à sec sous pression réduite (2,7 kPa). On obtient 25 g d'acide (tert-butoxycarbonylamino-2 phényl) acétique sous la forme d'un solide blanc crème.

EXEMPLE 56

A une solution de 1,85 g d'acide (N-tert-butoxycarbonyl N-méthyl amino-2 phényl) acétique dans 30 $cm^3$ de dichlorométhane sec, refroidie à +5°C, on ajoute 1,13 g de N,N'-carbonyldiimidazole. On agite 30 minutes à +5°C puis on ajoute une solution de 2,29 g de chlorhydrate de diphényl-7,7 perhydroisoindolone-4-(3aRS,7aRS) et de 1,9 $cm^3$ de triéthylamine dans 20 $cm^3$ de dichlorométhane. Le mélange réactionnel est agité à 20°C pendant 2 heures, puis lavé par 200 $cm^3$ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 4 cm, hauteur 50 cm) en éluant sous une pression de 0,7 bar d'azote par un mélange de cyclohexane et d'acétate d'éthyle (30/70 en volumes) et en recueillant des fractions de 125 $cm^3$. Les fractions 22 à 34 sont réunies et concentrées à sec sous pression réduite (2,7 kPa) pour donner 2,8 g de [(N-tert-butoxycarbonyl N-méthyl amino-2 phényl) acétyl]-2 diphényl-7,7 perhydroisoindolone-4-(3aRS,7aRS) sous la

forme d'une meringue blanche.

On traite 2,8 g de [(N-tert-butoxycarbonyl N-méthyl amino-2 phényl) acétyl]-2 diphényl-7,7 perhydroisoindolone-4-(3aRS,7aRS) par 30 $cm^3$ d'une solution 5,7 N d'acide chlorhydrique dans le dioxanne sec à 20°C pendant 4 heures. On ajoute 100 $cm^3$ d'oxyde d'isopropyle, le solide est essoré, lavé par de l'oxyde d'isopropyle et séché. On obtient 2,1 g de chlorhydrate de [(méthylamino-2 phényl) acétyl]-2 diphényl-7,7 perhydroisoindolone-4-(3aRS,7aRS), sous la forme d'un solide blanc.

Spectre RMN du proton :

A température ambiante, on observe le mélange des deux rotamères.

2,1 à 2,27 (2Mt, respectivement 1H chacun, $-CH_2-$ en 5 ou 6) ; 2,65 à 3,35 (Mt, $-CH_2-$ en 6 ou 5 et $-CH_2-$ en 1) ; 2,82 et 2,87 (2S, $-NCH_3$) ; 3,4 à 3,75 (Mt, 1H du $-CH_2-$ en 3 et -CH- en 3a) ; 3,55 et 3,85 (2S, $-N-CO-CH_2-$) ; 3,9 à 4,2 (Mt, -CH- en 7a) ; 4,15 à 4,45 (Mt, 1H du $-CH_2-$ en 6 ou 5) ; 7 à 7,7 (Mt, 14H, aromatiques).

Spectre infra-rouge (KBr) bandes caractéristiques ($cm^{-1}$):

3600-3300, 3100-3000, 3000-2850, 3100-2200, 1712, 1640- 1610, 1595, 1495, 1475-1410, 1445, 755, 702.

L'acide (N-tert-butoxycarbonyl N-méthyl amino-2 phényl) acétique peut être préparé de la manière suivante :

Une solution de 3,5 g de (N-tert-butoxycarbonyl N-méthyl amino-2 phényl) acétate de méthyle dans 50 $cm^3$ d'éthanol est traitée par 15 $cm^3$ de solution de soude normale à 80°C pendant 4 heures. Le mélange réactionnel est concentré sous pression réduite (2,7 kPa). Le résidu est repris par 100 $cm^3$ d'eau, et la solution acidifiée à pH 1 par action d'acide chlorhydrique 4N est extraite par 100 $cm^3$ d'acétate d'éthyle (2 fois).Les phases organiques réunies sont séchées sur sulfate de magnésium, filtrées et concentrées à sec sous pression réduite (2,7 kPa) pour donner 2,85 g d'acide (N-tert-butoxycarbonyl N-méthyl amino-2 phényl) acétique sous la forme d'un solide blanc.

Le (N-tert-butoxycarbonyl N-méthyl amino-2 phényl) acétate de méthyle peut être préparé de la manière suivante :

Une solution de 5 g d'acide (tert-butoxycarbonylamino-2 phényl) acétique dans 50 $cm^3$ de diméthylformamide sec est ajoutée à une suspension de 1,2 g d'hydrure de sodium (dispersion à 80 % dans l'huile) dans 20 $cm^3$ de diméthylformamide sec. Le mélange réactionnel est chauffé à 80°C pendant 2 heures, refroidi à 20°C. On ajoute 2,51 $cm^3$ d'iodure de méthyle et agite à 20°C pendant 16 heures. On dilue par 200 $cm^3$ d'eau et extrait par de l'acétate d'éthyle (2 fois 200 $cm^3$). Les phases organiques réunies sont lavées par 100 $cm^3$ d'eau, séchées sur sulfate de magnésium, filtrées et concentrées à sec sous pression réduite (2,7 kPa). Le résidu est chromatographié sur une colonne de gel de silice (0,2-0,063 mm, diamètre 4 cm, hauteur 48 cm) en éluant sous une pression de 0,7 bar d'azote par un mélange de cyclohexane et d'acétate d'éthyle (90/10 en volumes) et en recueillant des fractions de 125 $cm^3$. Les fractions 9 à 17 sont réunies et concentrées à sec sous pression réduite (2,7 kPa) pour donner 3,5 g de (N-tert-butoxycarbonyl N-méthyl amino-2 phényl) acétate de méthyle sous la forme d'une huile jaune.

## EXEMPLE 57

A une solution de 1,4 g d'acide (N-tert-butoxycarbonyl N-éthyl amino-2 phényl) acétique dans 30 $cm^3$ de dichlorométhane sec, refroidie à +5°C, on ajoute 0,81 g de N,N'-carbonyldiimidazole. On agite 30 minutes à +5°C puis on ajoute une solution de 1,63 g de chlorhydrate de diphényl-7,7 perhydroisoindolone-4-(3aRS,7aRS) et de 1,4 $cm^3$ de triéthylamine dans 20 $cm^3$ de dichlorométhane. Le mélange réactionnel est agité à 20°C pendant 16 heures, puis lavé par 100 $cm^3$ d'eau (2 fois), séché sur sulfate de magnésium filtré et concentré à sec sous pression réduite (2,7 kPa). Le résidu est chromatographié sur une colonne de gel de silice (0,2-0,063 mm, diamètre 4 cm, hauteur 35 cm) en éluant sous une pression de 0,7 bar d'azote par un mélange de cyclohexane et d'acétate d'éthyle (60/40 en volumes) et en recueillant des fractions de 125 $cm^3$. Les fractions 19 à 29 sont réunies et concentrées à sec sous pression réduite (2,7 kPa) pour donner 1,9 g de [(N-tert-butoxycarbonyl N-éthyl amino-2 phényl) acétyl]-2 diphényl-7,7 perhydroisoindolone-4-(3aRS,7aRS) sous la forme d'une meringue blanche.

On traite 1,8 g de [(N-tert-butoxycarbonyl N-éthyl amino-2 phényl) acétyl]-2 diphényl-7,7 perhydroisoindolone-4-(3aRS,7aRS) par 18 $cm^3$ d'une solution 5,7 N d'acide chlorhydrique dans le dioxanne sec à 20°C pendant 4 heures. Le mélange réactionnel est concentré à sec sous pression réduite (2,7 kPa), le solide est agité avec 100 $cm^3$ d'oxyde d'isopropyle, essoré, lavé par de l'oxyde d'isopropyle et séché. On obtient 1,54 g de chlorhydrate d'[(éthylamino-2 phényl) acétyl]-2 diphényl-7,7 perhydroisoindolone-4-(3aRS,7aRS) que l'on agite avec un mélange de 40 $cm^3$ de solution normale de soude et de 30 $cm^3$ d'acétate d'éthyle. Le solide est essoré, lavé 2 fois par 10cm³ d'eau et 3 fois par 10 $cm^3$ d'acétate d'éthyle et séché. On obtient 0,85 g d'[(éthylamino-2 phényl) acétyl]-2 diphényl-7,7 perhydroisoindolone-4-(3aRS,7aRS) sous la forme d'un solide blanc.

Spectre RMN du proton :

A température ambiante, on observe le mélange des deux rotamères.
1,16 (T, 3H, $-CH_3$ de l'éthyl amino) ; 2,05 et 2,23 (2Mt, respectivement 1H chacun, $-CH_2-$ en 5 ou 6) ; 2,6 à 3,6 (Mt, $-CH_2-$ en 6 ou 5, $-CH_2-$ en 1, $-NCH_2-$ éthyl, $-NCOCH_2-$,1H du $-CH_2-$ en 3 et -CH- en 3a) ; 3,9 à 4,2 (Mt, -CH- en 7a) ; 4,10 à 4,45 (Mt, 1H du $-CH_2-$ en 3) ; 6,3 à 7,7 (Mt, 14H, aromatiques).
Spectre Infra-rouge (KBr) bandes caractéristiques ($cm^{-1}$):
3600-3300, 3465, 3105-3000, 3000-2870, 1715, 1625, 1595, 1525, 1495, 1475, 1445, 1410, 755, 705.

L'acide (N-tert-butoxycarbonyl N-éthyl amino-2 phényl) acétique peut être préparé de la manière suivante :
Une solution de 4,7 g de (N-tert-butoxycarbonyl N-éthyl amino-2 phényl) acétate d'éthyle dans 50 $cm^3$ d'éthanol est traitée par 20 $cm^3$ de solution de soude normale pendant 4 heures au reflux. Le mélange réactionnel est concentré sous pression réduite (2,7 kPa). Le résidu est repris par 50 $cm^3$ d'eau et 50 $cm^3$ d'acétate d'éthyle, la phase aqueuse est lavée 2 fois par 100 $cm^3$ d'acétate d'éthyle puis acidifiée à pH 1 par action d'acide chlorhydrique 4N et extraite par 100 $cm^3$ d'acétate d'éthyle (2 fois). Les phases organiques réunies sont séchées sur sulfate de magnésium, filtrées et concentrées à sec sous pression réduite (2,7 kPa) pour donner 4 g d'acide (N-tert-butoxycarbonyl N-éthyl amino-2 phényl) acétique sous la forme d'une huile jaune.

Le (N-tert-butoxycarbonyl N-éthyl amino-2 phényl) acétate d'éthyle peut être préparé de la manière suivante :
Une solution de 5 g d'acide (tert-butoxycarbonylamino-2 phényl) acétique dans 50 $cm^3$ de diméthylformamide sec est ajoutée à une suspension de 1,2 g d'hydrure de sodium (dispersion à 80 % dans l'huile) dans 20 $cm^3$ de diméthylformamide sec. Le mélange réactionnel est chauffé à 80°C pendant 2 heures, refroidi à 20°C. On ajoute 3,25 $cm^3$ d'iodure d'éthyle et agite à 20°C pendant 16 h. On dilue par 200 $cm^3$ d'eau et extrait par de l'acétate d'éthyle (2 fois 200 $cm^3$). Les phases organiques réunies sont lavées par 100 $cm^3$ d'eau, séchées sur sulfate de magnésium, filtrées et concentrées à sec sous pression réduite (2,7 kPa). Le résidu est chromatographié sur une colonne de gel de silice (0,2-0,063 mm, diamètre 4 cm, hauteur 40 cm) en éluant sous une pression de 0,7 bar d'azote par un mélange de cyclohexane et d'acétate d'éthyle (80/20 en volumes) et en recueillant des fractions de 125 $cm^3$. Les fractions 5 à 7 sont réunies et concentrées à sec sous pression réduite (2,7 kPa) pour donner 4,7 g de (N-tert-butoxycarbonyl N-éthyl amino-2 phényl) acétate d'éthyle sous la forme d'une huile jaune.

EXEMPLE 58

A une solution de 0,9 g d'acide (N-tert-butoxycarbonyl N-propylamino-2 phényl) acétique dans 25 $cm^3$ de dichlorométhane sec, refroidie à +5°C, on ajoute 0,5 g de N,N'-carbonyldiimidazole. On agite 30 minutes à +5°C puis on ajoute une solution de 1 g de chlorhydrate de diphényl-7,7 perhydroisoindolone-4-(3aRS,7aRS) et de 0,87 $cm^3$ de triéthylamine dans 15 $cm^3$ de dichlorométhane. Le mélange réactionnel est agité à 20°C pendant 3 heures, puis dilué par 75 $cm^3$ d'eau et 50 $cm^3$ d'acétate d'éthyle. La phase organique est lavée par 50 $cm^3$ d'eau et 75 $cm^3$ de solution saturée de chlorure de sodium, séchée sur sulfate de magnésium, filtrée et concentrée à sec sous pression réduite (2,7 kPa). Le résidu est chromatographié sur une colonne de gel de silice (0,2-0,063 mm, diamètre 2,2 cm, hauteur 25 cm) en éluant par un mélange de cyclohexane et d'acétate d'éthyle (50/50 en volumes) et en recueillant des fractions de 50 $cm^3$. Les fractions 5 à 10 sont réunies et concentrées à sec sous pression réduite (2,7 kPa) pour donner 1,4 g de [(N-tert-butoxycarbonyl N-propyl amino-2 phényl) acétyl]-2 diphényl-7,7 perhydroisoindolone-4-(3aRS,7aRS) sous la forme d'une huile jaune.

On traite 1,4 g de [(N-tert-butoxycarbonyl N-propyl amino-2 phényl) acétyl]-2 diphényl-7,7 perhydroisoindolone-4(3aRS,7aRS) par 15 $cm^3$ d'une solution 5,7 N d'acide chlorhydrique dans le dioxanne sec à 20°C pendant 4 heures. Le mélange réactionnel est concentré à sec sous pression réduite (2,7 kPa) et le résidu est dissous dans un mélange de 50 $cm^3$ d'acétate d'éthyle et de 50 $cm^3$ d'eau. Les cristaux formés sont essorés, lavés par 15 $cm^3$ d'eau, 25 $cm^3$ d'acétate d'éthyle et 2 fois 50 $cm^3$ l'oxyde d'isopropyle et séchés. On obtient 0,75 g de diphényl-7,7 [(propylamino-2 phényl) acétyl-2 perhydroisoindolone-4-(3aRS,7aRS) sous la forme de cristaux blancs, fondant à 238°C.

L'acide (N-tert-butoxycarbonyl N-propyl amino-2 phényl) acétique peut être préparé de la manière suivante :
Une solution de 1,1 g de (N-tert-butoxycarbonyl N-propylamino-2 phényl) acétate de propyle dans 25 $cm^3$ d'éthanol est traitée par 3,3 $cm^3$ de solution de soude normale pendant 3 heures au reflux. Le mélange réactionnel est concentré sous pression réduite (2,7 kPa). Le résidu est repris par 50 $cm^3$ d'eau et 50 $cm^3$ d'acétate d'éthyle et acidifié à pH 1 par de l'acide chlorhydrique 4N, la phase aqueuse est extraite par 50 $cm^3$ d'acétate d'éthyle (2 fois). Les phases organiques réunies sont lavées par 50 $cm^3$ d'eau et 50 $cm^3$ de solution saturée de chlorure de sodium, séchées sur sulfate de magnésium, filtrées et concentrées à sec sous pression réduite (2,7 kPa) pour donner 0,9 g d'acide (N-tert-butoxycarbonyl N-propyl amino-2 phényl) acétique sous la forme d'une huile jaune.

Le (N-tert-butoxycarbonyl N-propyl amino-2 phényl) acétate de propyle peut être préparé de la manière suivante :
Une solution de 3 g d'acide (tert-butoxycarbonylamino-2 phényl) acétique dans 30 $cm^3$ de diméthylformamide sec est ajoutée à une suspension de 0,72 g d'hydrure de sodium (dispersion à 80 % dans l'huile) dans 20 $cm^3$ de diméthylformamide sec. Le mélange réactionnel est chauffé à 80°C pendant 2 heures, refroidi à 20°C. On ajoute 2,33 $cm^3$ d'iodure de propyle et agite à 20°C pendant 20 heures. On dilue par 100 $cm^3$ d'eau et extrait par de l'acétate d'éthyle (2 fois 100 $cm^3$). Les phases organiques réunies sont lavées par 100 $cm^3$ d'eau (2 fois) et par 100 $cm^3$ de solution saturée de chlorure de sodium, séchées sur sulfate de magnésium, filtrées et concentrées à sec sous pression réduite (2,7 kPa). Le résidu est chromatographié sur une colonne de gel de silice (0,2-0,063 mm, diamètre 2,6 cm, hauteur 27 cm) en éluant sous une pression de 0,5 bar d'azote par un mélange de cyclohexane et d'acétate d'éthyle (97/3 en volumes) et en recueillant des fractions de 35 $cm^3$. Les fractions 10 à 20 sont réunies et concentrées à sec sous pression réduite (2,7 kPa) pour donner 1,1 g de (N-tert-butoxycarbonyl N-propyl amino-2 phényl) acétate de propyle sous la forme d'une huile jaune.

EXEMPLE 59

A une solution de 1,1 g d'acide diméthylamino-2 phénylacétique, dans 30 $cm^3$ de dichlorométhane sec, refroidie à +5°C, on ajoute 1 g de N,N'-carbonyldiimidazole. On agite 30 minutes à +5°C puis on ajoute une solution de 2,03 g de chlorhydrate de diphényl-7,7 perhydroisoindolone-4-(3aRS,7aRS) et de 1,68 $cm^3$ de triéthylamine dans 20 $cm^3$ de dichlorométhane. Le mélange réactionnel est agité à 20°C pendant 24 heures, puis lavé par 200 $cm^3$ d'eau (3 fois), séché sur sulfate de magnésium, filtré et concentré à sec sous pression réduite (2,7 kPa).

Le résidu est chromatographié sur une colonne de gel de silice (0,2-0,063 mm, diamètre 3 cm, hauteur 25 cm) en éluant sous une pression de 0,7 bar d'azote, par un mélange de cyclohexane et d'acétate d'éthyle (40/60 en volumes) et en recueillant des fractions de 125 $cm^3$. Les fractions 8 à 26 sont réunies et concentrées à sec sous pression réduite (2,7 kPa). Le résidu est cristallisé dans 40 $cm^3$ d'oxyde d'isopropyle. Les cristaux sont essorés, lavés à l'oxyde d'isopropyle et séchés. On obtient 0,9 g de diphényl-7,7 [(diméthylamino-2 phényl)-2 acétyl]-2 perhydroisoindolone-4-(3aRS,7aRS) sous la forme de cristaux blancs, fondant à 150°C.

L'acide diméthylamino-2 phénylacétique est préparé selon la méthode de D-U. Lee, K.K. Mayer et W. Wiegrebe (Arch. Pharm. (Weinheim), 321, 303 (1988)).

EXEMPLE 60

A une solution de 2,68 g d'acide diméthylamino-2 phénylacétique, dans 50 $cm^3$ de dichlorométhane sec, refroidie à +5°C, on ajoute 2,43 g de N,N'-carbonyldiimidazole. On agite 90 minutes à +5°C puis on ajoute une solution de 4,9 g de chlorhydrate de diphényl-7,7 perhydroisoindolone-4-(3aR,7aR) et de 4,2 $cm^3$ de triéthylamine dans 50 $cm^3$ de dichlorométhane. Le mélange réactionnel est agité à 20°C pendant 16 heures, puis lavé par 100 $cm^3$ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 5 cm, hauteur 50 cm) en éluant sous une pression de 0,7 bar d'azote, par un mélange de cyclohexane et d'acétate d'éthyle (40/60 en volumes) et en recueillant des fractions de 125 $cm^3$. Les fractions 5 à 20 sont réunies et concentrées à sec sous pression réduite (2,7 kPa). Le résidu est cristallisé dans un mélange de 40 $cm^3$ d'acétonitrile et 200 $cm^3$ d'oxyde d'isopropyle. Les cristaux sont essorés, lavés à l'oxyde d'isopropyle et séchés. On obtient 2,58 g de diphényl-7,7 [(diméthylamino-2 phényl)-2 acétyl]-2 perhydroisoindolone-4-(3aR,7aR) sous la forme de cristaux blancs, fondant à 190°C;
$[\alpha]_D^{20}$= - 242° (c=1,18, chloroforme)

EXEMPLE 61

A une solution de 1,1 g d'acide diéthylamino-2 phénylacétique, dans 30 $cm^3$ de dichlorométhane sec, refroidie à +5°C, on ajoute 0,86 g de N,N'-carbonyldiimidazole. On agite 30 minutes à +5°C puis on ajoute une solution de 1,73 g de chlorhydrate de diphényl-7,7 perhydroisoindolone-4-(3aRS,7aRS) et de 1,48 $cm^3$ de triéthylamine dans 40 $cm^3$ de dichlorométhane. Le mélange réactionnel est agité à 20°C pendant 24 heures, puis lavé par 150 $cm^3$ d'eau (2 fois), séché sur sulfate de magnésium, filtré et concentré à sec sous pression réduite (2,7 kPa). Le résidu est chromatographié sur une colonne de gel de silice (0,2-0,063 mm, diamètre 3 cm, hauteur 40 cm) en éluant sous une pression de 0,7 bar d'azote, par un mélange de cyclohexane et d'acétate d'éthyle (30/80 en volumes) et en recueillant des fractions de 125 $cm^3$. Les fractions 12 à 20 sont réunies et concentrées à sec sous pression réduite (2,7 kPa). Le résidu est cristallisé dans un mélange de 15 $cm^3$ d'acétonitrile

et de 50 $cm^3$ d'oxyde d'isopropyle d'oxyde d'isopropyle. Les cristaux sont éssorés, lavés à l'oxyde d'isopropyle et séchés. On obtient 1,6 g de diphényl-7,7 [(diéthylamino-2 phényl)-2 acétyl]-2 perhydroisoindolone-4-(3aRS,7aRS) sous la forme de cristaux blancs, fondant à 160°C.

L'acide diéthylamino-2 phénylacétique est préparé par hydrogènation d'une suspension de 9 g d'acide nitro-2 phénylacétique dans 40 $cm^3$ d'éthanol, sous une pression de 7 bars pendant 4 heures à 25°C en présence de 7 $cm^3$ d'acétaldéhyde et de 1 g de palladium à 10 % sur noir de carbone. Le mélange réactionnel est filtré, concentré à sec sous pression réduite (2,7 kPa) et le résidu chromatographié sur une colonne de silice (0,2-0,063 mm, diamètre 5 cm, hauteur 50 cm) en éluant sous une pression de 0,7 bar d'azote par un mélange de cyclohexane et d'acétate d'éthyle (70/30 en volumes) et en recueillant des fractions de 125 $cm^3$. Les fractions 38 à 48 sont concentrées à sec sous pression réduite (2,7 kPa) pour donner 1,1 g d'acide diéthylamino-2 phénylacétique sous la forme d'une huile jaune.

EXEMPLE 62

A une solution de 1,97 g d'acide (diméthylamino-2 fluoro-6 phényl)acétique,dans 50 $cm^3$ de dichlorométhane sec, refroidie à +5°C, on ajoute 1,62 g de N,N'-carbonyldiimidazole. On agite 90 minutes à +5°C puis on ajoute une solution de 3,27 g de chlorhydrate de diphényl-7,7 perhydroisoindolone-4-(3aRS,7aRS) et de 2,8 $cm^3$ de triéthylamine dans 50 $cm^3$ de dichlorométhane. Le mélange réactionnel est agité à 20°C pendant 16 heures, puis lavé par 100 $cm^3$ d'eau (2 fois), séché sur sulfate de magnésium, filtré et concentré à sec sous pression réduite (2,7 kPa). Le résidu est chromatographié sur une colonne de gel de silice (granulométrie 0,04-0,06 mm, diamètre 4 cm, hauteur 25 cm) en éluant sous une pression de 0,5 bar d'azote, par un mélange de cyclohexane et d'acétate d'éthyle (70/30 en volumes) et en recueillant des fractions de 50 $cm^3$. Les fractions 6 à 19 sont réunies et concentrées à sec sous pression réduite . Le résidu est trituré dans de l'éther éthylique. Les cristaux sont essorés et séchés. On obtient 3,6 g de [(diméthylamino-2 fluoro-6 phényl)acétyl]-2 diphényl-7,7 perhydroisoindolone-4-(3aRS,7aRS) sous la forme de cristaux blancs fondant à 210°C.

L'acide (diméthylamino-2 fluoro-6 phényl)acétique est préparé de la façon suivante :

A une solution de 10 g d'acide (fluoro-6 nitro-2 phényl)acétique et de 25 $cm^3$ de solution aqueuse de formaldéhyde à 37% dans 45 $cm^3$ d'éthanol, dans un autoclave de 250 $cm^3$ , on ajoute 1,0 g de palladium sur charbon à 10%, puis on place l'autoclave sous une pression de 30 bars d'hydrogène, et on l'agite à température ambiante pendant 45 minutes. Le mélange réactionnel est ensuite filtré et concentré à sec sous pression réduite (2,7 kPa). Le résidu est chromatographié sur colonne de gel de silice Merck (granulométrie 0,06-0,04 mm, diamètre 4,0 cm, hauteur 40 cm), en éluant par un mélange de cyclohexane et d'acétate d'éthyle (75/25 en volumes) et en recueillant des fractions de 60 $cm^3$. Les fractions 11 à 25 sont réunies et concentrées à sec sous pression réduite (2,7 kPa) . On obtient 7,0 g d'acide (diméthylamino-2 fluoro-6 phényl)-acétique sous la forme de cristaux blancs, fondant à 94°C.

L'acide (fluoro-6 nitro-2 phényl)-acétique est obtenu selon la méthode décrite dans le brevet CS 194005.

EXEMPLE 63

A une solution de 2 g d'acide (diméthylamino-2 fluoro-4 phényl)acétique, dans 50 $cm^3$ de dichlorométhane sec, refroidie à +5°C, on ajoute 1,64 g de N,N'-carbonyldiimidazole. On agite 90 minutes à +5°C puis on ajoute une solution de 3,3 g de chlorhydrate de diphényl-7,7 perhydroisoindolone-4-(3aRS,7aRS) et de 2,8 $cm^3$ de triéthylamine dans 50 $cm^3$ de dichlorométhane. Le mélange réactionnel est agité à 20°C pendant 16 heures, puis lavé par 100 $cm^3$ d'eau (2 fois), séché sur sulfate de magnésium, filtré et concentré à sec sous pression réduite (2,7 kPa). Le résidu est chromatographié sur une colonne de gel de silice (granulométrie 0,04-0,06 mm, diamètre 4 cm, hauteur 25 cm) en éluant sous une pression de 0,4 bar d'azote, par un mélange de cyclohexane et d'acétate d'éthyle (50/50 en volumes) et en recueillant des fractions de 50 $cm^3$. Les fractions 5 à 12 sont réunies et concentrées à sec sous pression réduite. Le résidu est trituré dans de l'éther éthylique. Les cristaux sont essorés, et séchés. On obtient 3,1 g de [(diméthylamino-2 fluoro-4 phényl)acétyl]-2 diphényl-7,7 perhydroisoindolone-4-(3aRS,7aRS) sous la forme de cristaux blancs fondant à 164°C.

L'acide (diméthylamino-2 fluoro-4 phényl)acétique est préparé de la façon suivante : à une solution de 5,5 g d'acide (fluoro-4 nitro-2 phényl)acétique et de 15 $cm^3$ de solution aqueuse de formaldéhyde à 37% dans 130 $cm^3$ d'éthanol, dans un autoclave de 250 $cm^3$, on ajoute 0,6 g de palladium sur charbon à 10% , puis on place l'autoclave sous une pression de 47 bars d'hydrogène, et on l'agite à température ambiante pendant 48 heures. Le mélange réactionnel est ensuite filtré et concentré à sec sous pression réduite (2,7 kPa). Le résidu est chromatographié sur colonne de gel de silice Merck (granulométrie 0,06-0,04 mm, diamètre 4,2 cm, hauteur 50 cm), en éluant par un mélange de cyclohexane et d'acétate d'éthyle (80/20 en volumes) et en recueillant des fractions de 50 $cm^3$. Les fractions 9 à 23 sont réunies et concentrées à sec sous pression réduite (2,7 kPa). On

obtient 3,9 g d'acide (diméthylamino-2 fluoro-4 phényl)-acétique sous forme d'une huile incolore.
L'acide (fluoro-4 nitro-2 phényl)acétique est obtenu selon la méthode décrite dans le brevet CS 194005.

EXEMPLE 64

A une solution de 1,42 g d'acide [(pyrrolydinyl-1)-2 phényl]-2 acétique dans 30 $cm^3$ de dichlorométhane sec, refroidie à +5°C, on ajoute 1,13 g de N,N'-carbonyldiimidazole. On agite 40 minutes à +5°C puis on ajoute une solution de 2,3 g de chlorhydrate de diphényl-7,7 perhydroisoindolone-4-(3aRS,7aRS) et de 0,98 $cm^3$ de triéthylamine dans 20 $cm^3$ de dichlorométhane. Le mélange réactionnel est agité 1 heure à +5°C, puis 1 heure à 20°C. il est lavé par 20 $cm^3$ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 2 cm, hauteur 25 cm) en éluant avec un mélange d'acétate d'éthyle et de cyclohexane (70/30 en volumes) et en recueillant des fractions de 100 $cm^3$. Les fractions 3 à 8 sont réunies et concentrées à sec sous pression réduite (2,7 kPa). Le résidu est recristallisé dans un mélange de 5 $cm^3$ d'acétonitrile et de 5 $cm^3$ d'oxyde d'isopropyle. Les cristaux sont essorés, lavés par 10 $cm^3$ d'oxyde d'éthylène et séchés. On obtient 2,2 g de diphényl-7,7 [[(pyrrolidinyl-1)-2 phényl]-2 acétyl]-2 perhydroisoindolone-4-(3aRS,7aRS), fondant à 178°C.

Un mélange de 10,7 g d'acide (bromo-2 phényl)-2 acétique, de 20 $cm^3$ de pyrrolidine et de 1,66 g d'acétate de cuivre est chauffé 2 heures, sous agitation, à 90°C ; après retour à température ambiante, le mélange réactionnel est concentré à sec sous pression réduite (2,7 kPa). Le résidu est chromatographié sur une colonne de gel de silice (0,2-0,063 mm, diamètre 5 cm, hauteur 55 cm) en éluant par 1000 $cm^3$ de dichloro-1,2 éthane, puis par des mélanges de dichloro-1,2 éthane et de méthanol (proportions en volumes) : 1000 $cm^3$ (98/2), 2000 $cm^3$ (97/3), 1000 $cm^3$ (96/4) et 1000 $cm^3$ (95/5) et en recueillant des fractions de 250 $cm^3$. Les fractions 21 à 29 sont réunies et concentrées à sec sous pression réduite (2,7 kPa). On obtient 2 g d'acide [(pyrrolidinyl-1)-2 phényl]-2 acétique sous forme d'une huile orangée.

EXEMPLE 65

A une solution de 0,48 g d'acide [(morpholinyl-4)-2 phényl]-2 acétique dans 25 $cm^3$ de dichlorométhane sec, refroidie à +5°C, on ajoute 0,35 g de N,N'-carbonyldiimidasole. On agite 75 minutes à +5°C puis on ajoute une solution de 0,71 g de chlorhydrate de diphényl-7,7 perhydroisoindolone-4-(3aRS,7aRS) et de 0,6 $cm^3$ de triéthylamine dans 20 $cm^3$ de dichlorométhane. Le mélange réactionnel est agité 1 heure à +5°C, puis 8 heures à 20°C. Il est lavé par 100 $cm^3$ d'eau distillée (3 fois) et par 100 $cm^3$ de solution saturée de chlorure de sodium, séché sur sulfate de magnésium, filtré et concentré à sec sous pression réduite (2,7 kPa). Le résidu est chromatographié sur une colonne de gel de silice (0,04-0,063 mm, diamètre 2,2 cm, hauteur 12 cm) en éluant avec un mélange d'acétate d'éthyle et de cyclohexane (70/30 en volumes) sous une pression de 0,7 bar d'azote et en recueillant des fractions de 50 $cm^3$. Les fractions 4 et 5 sont réunies et concentrées à sec sous pression réduite (2,7 kPa). Le résidu est recristallisé dans 2 $cm^3$ d'oxyde d'éthylène. Les cristaux sont essorés, lavés par 1 $cm^3$ d'oxyde d'isopropyle et séchés. On obtient 0,27 g de diphényl-7,7 [[(morpholinyl-4)-2 phényl]-2 acétyl]-2 perhydroisoindolone-4-(3aRS,7aRS), fondant à 167°C.

L'acide [(morpholinyl-4)-2 phényl]-2 acétique peut être préparé de la manière suivante :
Une solution de 2,78 g de N-[(morpholinyl-4)-2 phényl] thioacétyl] morpholine dans un mélange de 25 $cm^3$ d'acide acétique et de 25 $cm^3$ d'acide chlorhydrique à 37 % est chauffée au reflux pendant 8 heures puis concentrée à sec sous pression réduite (2,7 kPa). Le résidu est repris par 20 $cm^3$ d'acétate d'éthyle et 20 $cm^3$ de solution normale de soude. La phase aqueuse est acidifiée à pH 5 par de l'acide acétique puis extraite par 20 $cm^3$ d'acétate d'éthyle. Cette phase organique est lavée à l'eau (2 fois 4 $cm^3$), séchées sur sulfate de magnésium et concentré à sec sous pression réduite (2,7 kPa). On obtient 0,48 g d'acide [(morpholinyl-4)-2 phényl]-2 acétique, fondant à 128°C.

La N-[(morpholinyl-4)-2 phényl) thioacétyl] morpholine peut être préparée de la manière suivante :
Un mélange de 4,6 g de (morpholinyl-4)-2 acétophénone, de 3,9 $cm^3$ de morpholine et de 1,79 g de soufre est chauffé au reflux, sous agitation, pendant 5 heures puis le mélange réactionnel chaud est dilué par 17 $cm^3$ d'éthanol bouillant. Après refroidissement le solide est essoré et chromatographié sur une colonne de gel de silice (0,04-0,063 mm, diamètre 3,9 cm, hauteur 30 cm) en éluant sous une pression de 0,6 bar d'azote par un mélange de cyclohexane et d'acétate d'éthyle (60/40 en volumes) et en recueillant des fractions de 100 $cm^3$. Les fractions 7 à 28 sont réunies et concentrées à sec sous pression réduite (2,7 kPa) pour donner 3,82 g de N-[(morpholinyl-4)-2 phényl] thioacétyl] morpholine, fondant à 140°C.

La (morpholino-4)-2 acétophénone peut être préparée de la manière suivante :
Un mélange de 19,9 g de fluoro-2 acétophènone, 21 $cm^3$ de morpholine et 27,6 g de carbonate de potassium dans 32 $cm^3$ de diméthylsulfoxyde est chauffé à 110°C pendant 30 heures. Le mélange réactionnel est dilué

par 100 $cm^3$ d'eau et extrait 4 fois par 100 $cm^3$ d'acétate d'éthyle; la solution organique est lavée par 100 $cm^3$ d'eau distillée, séchée sur sulfate de magnésium et concentrée à sec sous pression réduite (2,7 kPa). Le résidu est chromatographié sur une colonne de gel de silice (0,04-0,063 mm, diamètre 9 cm, hauteur 40 cm) en éluant par un mélange de cyclohexane et d'acétate d'éthyle (80/20 en volumes) et en recueillant des fractions de 250 $cm^3$. Les fractions 24 à 44 sont réunies et concentrées à sec sous pression réduite (2,7 kPa). On obtient 4,72 g de (morpholinyl-4)-2 acétophénone utilisée telle quelle pour les synthèses ultérieures.

EXEMPLE 66

A une solution de 1,3 g d'acide (naphtyl-1)-2 acétique dans 30 $cm^3$ de dichlorométhane sec, refroidie à +5°C, on ajoute 1,13 g de N,N'-carbonyldiimidasole. On agite 25 minutes à + 5°C puis on ajoute une solution de 2,3 g de chlorhydrate de diphényl-7,7 perhydroisoindolone-4-(3aRS,7aRS) et de 0,98 $cm^3$ de triéthylamine dans 40 $cm^3$ de dichlorométhane. Le mélange réactionnel est agité à 20°C pendant 4 heures, puis lavé par 50 $cm^3$ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 1,8 cm, hauteur 19 cm ) en éluant avec du dichlorométhane et en recueillant des fractions de 30 $cm^3$. Les fractions 3 à 8 sont réunies et concentrées à sec sous pression réduite (2,7 kPa). Le résidu est cristallisé dans 14 $cm^3$ d'acétonitrile. Les cristaux sont essorés, lavés par 10 $cm^3$ d'oxyde d'isopropyle et séchés. On obtient 1,4 g de (naphtyl-1 acétyl)-2 diphényl-7,7 perhydroisoindolone-4-(3aRS,7aRS), fondant à 207 °C.

EXEMPLE 67

A une solution de 0,71 g d'acide (thiényl-2)-2 acétique dans 20 $cm^3$ de dichlorométhane sec, refroidie à +5°C, on ajoute 0,81 g de N,N'-carbonyldiimidasole. On agite 1 heure à +5°C puis on ajoute une solution de 1,6 g de chlorhydrate de diphényl-7,7 perhydroisoindolone-4 et de 0,7 $cm^3$ de triéthylamine dans 20 $cm^3$ de dichlorométhane. Le mélange réactionnel est agité 2 heures à +5°C, 16 heures à 20°C puis lavé par 50 $cm^3$ d'eau distillée (3 fois), séché sur sulfate de magnésium, filtré et concentré à sec sous pression réduite (2,7 kPa). Le résidu est recristallisé dans 10 $cm^3$ d'acétonitrile. Les cristaux sont essorés, lavés par 10 $cm^3$ d'oxyde d'isopropyle et séchés. On obtient 1,5 g de diphényl-7,7 [(thiényl-2) acétyl]-2 perhydroisoindolone-4-(3aRS,7aRS), fondant à 206°C

EXEMPLE 68

A une solution de 0,99 g d'acide (thiényl-3)-2 acétique dans 30 $cm^3$ de dichlorométhane sec, refroidie à +5°C, on ajoute 1,13 g de N,N'-carbonyldiimidazole. On agite 15 minutes à +5°C puis on ajoute une solution de 2,24 g de chlorhydrate de diphényl-7,7 perhydroisoindolone-4-(3aRS,7aRS) et de 1,96 $cm^3$ de triéthylamine dans 20 $cm^3$ de dichlorométhane. Le mélange réactionnel est agité 16 heures à 20°C. puis lavé par 50 $cm^3$ d'eau distillée (3 fois), séché sur sulfate de magnésium filtré et concentré à sec sous pression réduite (2,7 kPa). Le résidu est recristallisé dans un mélanges de 30 $cm^3$ d'acétonitrile et de 40 $cm^3$ d'oxyde d'isopropyle. Les cristaux sont essorés, lavés par de l'oxyde d'isopropyle et séchés . On obtient 2,2 g de diphényl-7,7 [(thiényl-3)-2 acétyl]-2 perhydroisoindolone-4-(3aRS,7aRS), fondant à 224°C.

EXEMPLE 69

A une solution de 1,23 g d'acide (dithiine-1,3 yl-5)-2 acétique dans 30 $cm^3$ de dichlorométhane sec, refroidie à +5°C, on ajoute 1,13 g de N,N'-carbonyldiimidazole. On agite 20 minutes à +5°C puis on ajoute une solution de 2,3 g de chlorhydrate de diphényl-7,7 perhydroisoindolone-4-(3aRS,7aRS) et de 0,98 $cm^3$ de triéthylamine dans 40 $cm^3$ de dichlorométhane. Le mélange réactionnel est agité 16 heures à 20°C. Le mélange réactionnel est lavé par 50 $cm^3$ d'eau distillée (3 fois), séché sur sulfate de magnésium, filtré et concentré à sec sous pression réduite (2,7 kPa). Le résidu est cristallisé dans 80 $cm^3$ d'acétonitrile. Les cristaux sont essorés, lavés par 10 $cm^3$ d'oxyde d'isopropyle et séchés. On obtient 1,94 g de diphényl-7,7 [(dithiine-1,3-yl-5)-2 acétyl]-2 perhydroisoindolone-4-(3aRS,7aRS), fondant à 211°C.

EXEMPLE 70

A une suspension de 1,73 g du chlorhydrate de l'acide (pyridyl-2)-2 acétique dans 30 $cm^3$ de dichlorométhane sec on ajoute 1,4 $cm^3$ de triéthylamine. La solution est refroidie à +5°C puis traitée par 1,62 g de N,N'-carbonyldiimidazole. On agite 15 minutes à +5°C puis on ajoute une solution de 3,27 g de chlorhydrate de diphényl-7,7 perhydroisoindolone-4-(3aRS,7aRS) et de 2,8 $cm^3$ de triéthylamine dans 20 $cm^3$ de dichlorométhane. Le mélange réactionnel est agité à 20°C pendant 2 heures, puis concentré à sec sous pression réduite (2,7 kPa). Le résidu est repris dans un mélange de 100 $cm^3$ d'acétate d'éthyle et de 75 $cm^3$ d'eau. La phase organique est lavée par 50 $cm^3$ d'eau, séchée sur sulfate de magnésium, filtrée et concentrée à sec sous pression réduite (2,7 kPa). Le résidu est recristallisé dans un mélange de 30 $cm^3$ d'acétonitrile et de 30 $cm^3$ d'oxyde d'isopropyle. On obtient 1,6 g de diphényl-7,7 [(pyridyl-2)-2 acétyl]-2 perhydroisoindolone-4-(3aRS,7aRS) sous la forme de cristaux blancs, fondant à 195°C.

EXEMPLE 71

A une solution de 0,685 g d'acide (pyridyl-3)-2 acétique dans 15 $cm^3$ de dichlorométhane sec, refroidie à +5°C, on ajoute 0,81 g de N,N'-carbonyldiimidazole. On agite 30 minutes à +5°C puis on ajoute une solution de 1,6 g de chlorhydrate de diphényl-7,7 perhydroisoindolone-4-(3aRS,7aRS) et de 0,7 $cm^3$ de triéthylamine dans 10 $cm^3$ de dichlorométhane. Le mélange réactionnel est agité à 20°C pendant 16 heures, puis concentré à sec sous pression réduite (2,7 kPa). Le résidu est repris dans un mélange de 100 $cm^3$ d'acétate d'éthyle et de 75 $cm^3$ d'eau. La phase organique est lavée par 50 $cm^3$ de solution saturée de chlorure de sodium, séchée sur sulfate de magnésium, filtrée et concentrée à sec sous pression réduite (2,7 kPa). Le résidu est chromatographié sur une colonne d'alumine basique (diamètre 2 cm, hauteur 15 cm) en éluant par de l'acétate d'éthyle et en recueillant des fractions de 50 $cm^3$. Les fractions 4 à 24 sont concentrées à sec sous pression réduite (2,7kPa). Les cristaux obtenus sont recristallisés dans 15 $cm^3$ d'acétonitrile pour donner 0,9 g de diphényl-7,7 [(pyridyl-3)-2 acétyl]-2 perhydroisoindolone-4-(3aRS,7aRS) sous la forme de cristaux blancs, fondant à 208°C.

EXEMPLE 72

A une solution de 4,37 g d'acide (indolyl-3)-2 acétique dans 100 $cm^3$ de dichlorométhane sec, refroidie à +5°C, on ajoute 4,05 g de N,N'-carbonyldiimidazole. On agite 1 heure à +5°C puis on ajoute 7,3 g sde diphényl-7,7 perhydroisoindolone-4-(3aRS,7aRS). Le mélange réactionnel est agité à 20°C pendant 20 heures, puis lavé par 50 $cm^3$ d'eau distillée (3 fois), séché sur sulfate de magnésium, filtré et concentré à sec sous pression réduite (2,7 kPa). Le résidu est chromatographié sur une colonne de gel de silice (0,2-0,063 mm, diamètre 7 cm, hauteur 40 cm) en éluant par un mélange d'acétate d'éthyle et de cyclohexane (70/30 en volumes) et en recueillant des fractions de 100 $cm^3$. Les fractions 6 à 11 sont concentrées à sec sous pression réduite (2,7 kPa). Le résidu est recristallisé dans 50 $cm^3$ d'acétonitrile. Les cristaux obtenus sont essorés , lavés par 10 $cm^3$ d'oxyde d'isopropyle et séchés. On obtient 5 g de diphényl-7,7 [(indolyl-3)-2 acétyl]-2 perhydroisoindolone-4-(3aRS,7aRS), fondant à 250°C.

A une solution de 16,6 g de chlorhydrate de diphényl-7,7 perhydroisoindolone-4-(3aRS,7aRS) dans 250 $cm^3$ de dichlorométhane on ajoute, sous agitation, 100 $cm^3$ de soude aqueuse 4N ; la phase organique est séchée sur sulfate de magnésium, filtrée et concentrée à sec sous pression réduite (4 kPa). On obtient 13 g de diphényl-7,7 perhydroisoindolone-4-(3aRS,7aRS) sous forme d'une meringue blanche.

Spectre RMN du proton :

2,15 et 2,4 (2 Mt, respectivement 1H chacun, $-CH_2-$ en 5 ou 6) ; 2,75 (Mt, 4H, $-CH_2-$ en 1 et $-CH_2-$ en 6 ou 5) ; 3,3 à 3,6 (Mt, 2H, 1H du $-CH_2-$ en 3 et -CH- en 3a) ; 3,95 à 4,2 (Mt, 2H, 1H du $-CH_2-$ en 3 et -CH- en 7a) ; 7 à 7,5 (Mt, 10H, aromatiques).

Spectre infra-rouge ($CHBr_3$) bandes caractéristiques ($cm^{-1}$):

3350, 3100-3000, 3000-2800, 1705, 1600, 1580, 1495, 1460, 1445, 755.

EXEMPLE 73

A une solution de chlorure de l'acide α-fluoro phénylacétique-(S) [préparé à partir de 1 g d'acide selon la méthode décrite par S. Hamman et coll., J. Fluorine Chem., 37, 85 (1987)] dans 20 $cm^3$ de dichlorométhane on ajoute à 0°C une solution de 2,12 g de chlorhydrate de diphényl-7,7 perhydroisoindolone-4-(3aR,7aR), 0,9 $cm^3$ de triéthylamine et 0,5 $cm^3$ de pyridine dans 30 $cm^3$ de dichlorométhane. Le mélange réactionnel est agité 2 heures en laissant revenir la température à 20°C puis concentré à sec sous pression réduite (2,7 kPa). Le résidu est repris dans 100 $cm^3$ d'acétate d'éthyle. La solution est lavée 2 fois par 100 $cm^3$ d'eau puis par 50 $cm^3$ de solution saturée de bicarbonate de sodium et par 50 $cm^3$ de solution saturée de chlorure de sodium, séchée sur sulfate de magnésium, filtrée et concentrée à sec sous pression réduite (2,7 kPa). Le résidu est chromatographié sur une colonne de gel de silice (granulométrie 0,2-0,063 mm, diamètre 3 cm, hauteur 40 cm) en éluant sous une pression de 0,7 bar d'azote par de l'acétate d'éthyle et en recueillant des fractions de 100 $cm^3$ pour donner 1 g de [α-fluoro phénylacétyl-(S)]-2 diphényl-7,7 perhydroisoindolone-4-(3aR,7aR) sous

la forme d'une meringue blanche.
Spectre de RMN du proton (DMSO-$d_6$):
A température ordinaire on observe le mélange des deux rotamères.
2 à 2,4 (m, 2H, $-CH_2-$ en 5) ; 2,45 à 3 (m, 4H, $-CH_2-$ en 6 et $-CH_2-$ en 1) ; 3,11 à 3,28 (2m, 1H, 1H en 3) ; 3,38 (m, 1H, 1H en 3a) ; 3,94 et 4,05 (2m, 1H, H en 7a) ; 4,2 et 4,38 (2m, J=11, 1H , 1H en 3) ; 5,90 et 6,28 (2d, J=47, 1H, -CHF-) ; 7 à 7,7 (m, 15H, aromatiques).
Spectre infrarouge (bandes caractéristiques en $cm^{-1}$): 3100-3000, 3000-2875, 1715, 1655, 1600, 1580, 1495, 1450, 750, 700.

EXEMPLE 74

A une solution de 0,62 g d'acide phényl-2 propionique-(S) dans 30 $cm^3$ de dichlorométhane sec, refroidie à +5°C, on ajoute 0,66 g de N,N'-carbonyldiimidazole. On agite 40 minutes à +5°C puis on ajoute une solution de 1,35 g de chlorhydrate de diphényl-7,7 perhydroisoindolone-4-(3aR,7aR) et de 0,57 $cm^3$ de triéthylamine dans 40 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 1,8 cm, hauteur 15 cm) en éluant par de l'acétate d'éthyle et en recueillant des fractions de 15 $cm^3$. La première fraction est concentrée à sec sous pression réduite (2,7 kPa). On obtient 1 g de diphényl-7,7 [phényl-2 propionyl-(S)]-2 perhydroisoindolone-4-(3aR,7aR) sous la forme d'une meringue blanche.
$[\alpha]_D^{20}$ = -231° (c=1 , méthanol).
Spectre RMN du proton :
A température ambiante, on observe le mélange des deux rotamères.
1,16 et 1,26 (2D, 3H en totalité, $-CH_3$) ; 1,95 à 2,3 (Mt, 2H, $-CH_2-$ en 5 ou 6) ; 2,65 à 2,9 (Mt, 4H, $-CH_2-$ en 6 ou 5 et $-CH_2-$ en 1) ; 3,05 à 3,35 (Mt, 2H, 1H du $-CH_2-$ en 3 et -CH- en 3a) ; 3,4 et 3,8 à 4 (Mt, -N-CO-CH- et -CH- en 7a) ; 4,2 à 4,4 (Mt, 1H, 1H du $-CH_2-$ en 3) ; 6,9 à 7,6 (Mt, 15H, aromatiques).
Spectre infra-rouge (KBr) bandes caractéristiques ($cm^{-1}$):
3600-3300, 3100-3000, 3000-2870, 1715, 1640, 1600, 1580, 1495, 1455, 1445, 1420, 1370, 755, 700.

EXEMPLE 75

A une solution de 0,62 g d'acide phényl-2 propionique-(R) dans 30 $cm^3$ de dichlorométhane sec, refroidie à +5°C, on ajoute 0,66 g de N,N'-carbonyldiimidazole. On agite 30 minutes à +5°C puis on ajoute une solution de 1,35 g de chlorhydrate de diphényl-7,7 perhydroisoindolone-4-(3aR,7aR) et de 0,57 $cm^3$ de triéthylamine dans 40 $cm^3$ de dichlorométhane. Le mélange réactionnel est agité à 20°C pendant 16 heures, puis concentré à sec sous pression réduite (2,7 kPa). Le résidu est dissous dans 100 $cm^3$ d'acétate d'éthyle et la solution lavée par 50 $cm^3$ d'eau (2 fois), séchée sur sulfate de magnésium, filtrée et concentrée à sec sous pression réduite (4 kPa). Le résidu est chromatographié sur une colonne de gel de silice (0,2-0,04 mm, diamètre 2,8 cm, hauteur 15 cm) en éluant sous une pression de 0,5 bar d'azote par un mélange de cyclohexane et d'acétate d'éthyle (60/40 en volumes) et en recueillant des fractions de 50 $cm^3$. Les fractions 4 à 8 sont concentrées à sec sous pression réduite (4 kPa) pour donner 1,4 g de diphényl-7,7 [phényl-2 propionyl-(R)]-2 perhydroisoindolone-4-(3aR,7aR) sous la forme d'une meringue blanche dont le spectre infra-rouge est identique à celui du produit de l'exemple 68 ; $[\alpha]_D^{20}$ = -278° (c=1 , méthanol).
Spectre RMN du proton :
1,2 et 1,28 (2D, 3H en totalité, $-CH_3$) ; 1,9 à 2,3 (Mt, 2H, $-CH_2-$ en 5 ou 6) ; 2,50 à 3,15 (Mt, 4H, $-CH_2-$ en 6 ou 5 et $-CH_2-$ en 1) ; 3,15 à 4,O5 (Mt, -CH- en 3a, 1H du $-CH_2-$ en 3, -N-CO-CH-, -CH- en 7a) ; 4 à 4,21 (Mt, 1H du $-CH_2-$ en 3) ; 6,85 à 7,7 (Mt, 15H, aromatiques) ;

EXEMPLE 76

A une solution de 0,75 g d'acide [méthoxy-2 phényl]-2 propionique-(S), à 84 % de pureté optique, préparé selon T. Matsumoto et collaborateurs : Bull. Chem. Soc. Jpn., 58, 340 (1985) dans 15 $cm^3$ de diméthylformamide sec, on ajoute 0,59 g d'hydroxy-1 benzotriazole, puis refroidit la solution à 0°C. On ajoute 0,91 g de N,N'-dicyclohexylcarbodiimide, agite 1 heure à cette température puis on ajoute une solution de 1,44 g de chlorhydrate de diphényl-7,7 perhydroisoindolone-4-(3aR,7aR) et de 0,76 $cm^3$ de N,N diisopropyléthylamine dans 10 $cm^3$ de diméthylformamide. Le mélange réactionnel est agité à 20°C pendant 16 heures, dilué par 100 $cm^3$ d'acétate d'éthyle et concentré à sec sous pression réduite (2,7 kPa) après filtration du précipité. Le résidu est chromatographié sur une colonne de gel de silice (0,2-0,063 mm, diamètre 3 cm, hauteur 40 cm) en éluant

sous une pression de 0,7 bar d'azote, par un mélange de cyclohexane et d'acétate d'éthyle (50/50 en volumes) et en recueillant des fractions de 125 $cm^3$. Les fractions 4 à 7 sont réunies et concentrées à sec sous pression réduite (2,7 kPa). Le résidu est purifié par dissolution dans 60 $cm^3$ d'oxyde d'isopropyle bouillant additionné de 30 $cm^3$ d'hexane. La solution refroidie est filtrée et le filtrat concentré à sec sous pression réduite (2,7 kPa) pour donner 1,2 g de diphényl-7,7 [(méthoxy-2 phényl)-2 propionyl-(S)]-2 perhydroisoindolone-4-(3aR,7aR) sous la forme d'une meringue blanche contenant 10 % de diphényl-7,7 [(méthoxy-2 phényl)-2 propionyl-(R)]-2 perhydroisoindolone-4-(3aR,7aR);
$[\alpha]_D^{20}$= - 181° (c=0,81, chloroforme).
Spectre RMN du proton :
A température ambiante, on observe le mélange des deux rotamères de chacun des deux diastéréoisomères. Les deux diastéréoisomères étant dans les proportions 90/10.
1,10 et 1,20 (2 Mt, 3H en totalité, - $CH_3$) ; 1,9 à 2,4 (Mt, 2H, -$CH_2$- en 5 ou 6) ; 2,55 à 2,95 (Mt, -$CH_2$- en 1 et -$CH_2$- en 6 ou 5) ; 2,95 à 3,4 (Mt, 1H du -$CH_2$- en 3 et -CH- en 3a) ; 3,20-3,32-3,50 et 3,83 (4S, -$OCH_3$) ; 3,65 à 4,3 (Mt, -CH- en 7a, -N-CO-CH-, 1H du -$CH_2$- en 3) ; 6,7 à 7,65 (Mt, 14H, aromatiques).
Spectre infra-rouge (KBr), bandes caractéristiques ($cm^{-1}$):
3430, 3100-3000, 3000-2800, 1715, 1640, 1595, 1585, 1490, 1460, 1445, 1420, 1365, 1240, 1030, 755, 703.

## EXEMPLE 77

A une solution de 1 g d'acide [diméthylamino-2 phényl]-2 propionique-(RS) dans 30 $cm^3$ de dichlorométhane refroidie à 5°C, on ajoute 0,85 g de N,N'-carbonyldiimidazole puis agite 30 minutes à cette température. On ajoute une solution de 1,7 g de chlorhydrate de diphényl-7,7 perhydroisoindolone-4-(3aR,7aR) et de 1,4 $cm^3$ de triéthylamine dans 30 $cm^3$ de dichlorométhane. Le mélange réactionnel est agité à 20°C pendant 16 heures, lavé 2 fois par 100 $cm^3$ d'eau, séché sur sulfate de magnésium et concentré à sec sous pression réduite (2,7 kPa). Le résidu est chromatographié sur une colonne de gel de silice (0,2-0,063 mm, diamètre 3 cm, hauteur 50 cm) en éluant sous une pression de 0,5 bar d'azote, par de l'acétate d'éthyle et en recueillant des fractions de 125 $cm^3$. Les fractions 4 à 6 sont réunies et concentrées à sec sous pression réduite (2,7 kPa) pour donner 1,4 g de [(diméthylamino-2 phényl)-2 propionyl-(RS)]-2 diphényl-7,7 perhydroisoindolone-4-(3aR,7aR) sous la forme d'une meringue blanche.
Spectre RMN du proton :
A température ordinaire, on observe le mélange des deux rotamères de chacun des deux diastéréoisomères.
1,15 à 1,35 (Mt, 3H, -$CH_3$) ; 1,9 à 2,4 (Mt, -$CH_2$- en 5 ou 6); 2,1 - 2,19 - 2,62 - 2,64 (45, -$N(CH_3)_2$) ; 2,55 à 3,4 (Mt, -$CH_2$- en 6 ou 5, -$CH_2$- en 1, -CH- en 3a et 1H du -$CH_2$- en 3) ; 3,5 à 4,5 (Mt, -N-CO-CH-, 1H du -$CH_2$- en 3 et -CH en 7a) ; 7 à 7,7 (Mt, 15H, aromatiques).
Spectre infra-rouge (KBr) bandes caractéristiques ($cm^{-1}$):
3600-3300, 3100-3000, 3000-2780, 1715, 1640, 1595, 1580, 1490, 1460, 1445, 1410, 750, 702.

Une solution de 1,8 g d'acide [diméthylamino-2 phényl]-2 acétique dans 10 $cm^3$ de tétrahydrofuranne sec est ajoutée à 10°C à une solution de diisopropylamidure de lithium (préparée par action de 2,6 $cm^3$ de solution 1,6 M de butyllithium dans l'hexane sur une solution de 2,8 g de diisopropylamine dans 30 $cm^3$ de tétrahydrofuranne sec à 10°C). Le mélange réactionnel est agité 30 minutes à 20°C puis 30 minutes à 35°C. Après refroidissement à 20°C on ajoute 0,63 $cm^3$ d'iodure de méthyle et chauffe 1 heure à 35°C. On refroidit, dilue par 20 $cm^3$ d'eau et 100 $cm^3$ d'acétate d'éthyle. La phase aqueuse est lavée par 100 $cm^3$ d'acétate d'éthyle, acidifiée à pH 5 par de l'acide chlorhydrique et extraite 2 fois par 100 $cm^3$ d'acétate d'éthyle. Les phases organiques sont lavées à l'eau, séchées sur sulfate de magnésium et concentrées à sec sous pression réduite (2,7 kPa) pour donner 1 g d'acide [diméthylamino-2 phényl]-2 propionique-(RS) sous la forme d'une huile jaune.

## EXEMPLE 78

A une solution de 0,5 g d'acide phényl-2 butyrique-(S) dans 15 $cm^3$ de dichlorométhane sec, refroidie à +5°C, on ajoute 0,49 g de N,N'-carbonyldiimidazole. On agite 30 minutes à +5°C puis on ajoute une solution de 0,98 g de chlorhydrate de diphényl-7,7 perhydroisoindolone-4-(3aR,7aR) et de 0,42 $cm^3$ de triéthylamine dans 20 $cm^3$ de dichlorométhane. Le mélange réactionnel est agité à 20°C pendant 16 heures, puis lavé par 50 $cm^3$ d'eau distillée (2 fois), séché sur sulfate de magnésium, filtré et concentré à sec sous pression réduite (2,7 kPa). Le résidu est chromatographié sur une colonne de gel de silice (0,2-0,063 mm, diamètre 1,6 cm, hauteur 9,7 cm) en éluant avec un mélange d'acétate d'éthyle et de cyclohexane (80/20 en volumes) et en recueillant des fractions de 30 $cm^3$. La première fraction est concentrée à sec sous pression réduite (2,7 kPa). Le résidu est à nouveau chromatographié sur une colonne de gel de silice (0,2-0,063 mm, diamètre 1,3 cm, hauteur 14,5 cm) en éluant par un mélange de cyclohexane et d'acétate d'éthyle (70/30 en volumes) et en recueillant des fractions de 50 $cm^3$. Les fractions 1 à 3 sont réunies et concentrées à sec sous pression réduite (2,7 kPa). On obtient 0,54 g de diphényl-7,7 [phényl-2 butyryl-(S)]-2 perhydroisoindolone-4-(3aR,7aR) sous forme d'une meringue blanche.
$[\alpha]_D^{20}$ = -216° (c=1, méthanol).
Spectre RMN du proton :
A température ambiante, on observe le mélange des deux rotamères.
O,65 et O,77 (2T, 3H en totalité, $-CH_3$ éthyle) ; 1,4 à 2 (Mt, $-CH_2-$ de l'éthyle) ; 1,9 à 2,3 (Mt, 2H, $-CH_2-$ en 5 ou 6) ; 2,8 (Mt, 4H, $-CH_2-$ en 6 ou 5 et $-CH_2-$ en 1) ; 3,05 à 3,35 et 3,48 (Mt, -CH- en 3a, -N-CO-CH- et 1H du $-CH_2-$ en 3) ; 3,75 à 4,1 (Mt, 1H, -CH- en 7a) ; 4,2 à 4,45 (Mt, 1H, 1H du $-CH_2-$ en 3) ; 6,9 à 7,65 (Mt, 15H, aromatiques).
Spectre Infra-rouge (KBr) bandes caractéristiques ($cm^{-1}$):
3600-3300, 3100-3000, 3000-2830, 1715, 1640, 1600, 1580, 1492, 1450, 1445, 1420, 1375, 755 et 700.

## EXEMPLE 79

A une solution de 0,79 g d'acide tert-butoxycarbonylamino-3 phényl-2 propionique-(S) dans 20 $cm^3$ de dichlorométhane sec, refroidie à +5°C, on ajoute 0,5 g de N,N'-carbonyldiimidazole. On agite 1 heure 15 minutes à +5°C puis on ajoute une solution de 1 g de chlorhydrate de diphényl-7,7 perhydroisoindolone-4 -(3aR,7aR) et de 0,4 $cm^3$ de triéthylamine dans 25 $cm^3$ de dichlorométhane. Le mélange réactionnel est agité 15 minutes à +5°C, puis 16 heures à 20°C. Le mélange réactionnel est lavé par 100 $cm^3$ d'eau distillée (3 fois), séché sur sulfate de magnésium, filtré et concentré à sec sous pression réduite (2,7 kPa). Le résidu est chromatographié sur une colonne de gel de silice (0,2-0,06 mm, diamètre 3 cm, hauteur 30 cm) en éluant par un mélange de cyclohexane et d'acétate d'éthyle (50/50 en volumes) et en recueillant des fractions de 50 $cm^3$. Les fractions 2 à 7 sont réunies et concentrées à sec sous pression réduite (2,7 kPa). On obtient 1,2 g de [tert-butoxycarbonylaminométhyl-2 phényl-2 acétyl-(S)]-2 diphényl-7,7 perhydroisoindolone-4-(3aR,7aR) sous forme d'une meringue blanche.

On traite 1 g de [tert-butoxycarbonylaminométhyl-2 phényl-2 acétyl-(S)]-2 diphényl-7,7 perhydroisoindolone-4-(3aR,7aR) par 10 $cm^3$ d'une solution 5,7 N d'acide chlorhydrique dans le dioxanne sec pendant 2 heures à +5°C puis 3 heures à 20°C. Le mélange réactionnel est concentré à sec sous pression réduite (2,7 kPa) ; le résidu est cristallisé dans 5 $cm^3$ d'acétonitrile. Les cristaux obtenus sont essorés, lavés par 0,5 $cm^3$ d'acétonitrile et séchés. On obtient 0,76 g de chlorhydrate d'[(aminométhyl-2 phényl-2)acétyl-(S)]-2 diphényl-7,7 perhydroisoindolone-4-(3aR, 7aR), fondant à 220°C ;
$[\alpha]_D^{20}$= - 199° (c=0,2 , méthanol).

A une suspension de 8 g d'acide amino-3 phényl-2 propionique-(S) dans un mélange de 55 $cm^3$ d'eau et de 100 $cm^3$ de dioxanne, on ajoute, sous agitation, 10,3 g de carbonate de sodium, puis on verse une solution de 12,6 g de dicarbonate de ditert-butyle dans 16 $cm^3$ de dioxanne. Le mélange réactionnel est agité 20 heures à température ambiante, filtré et concentré à sec sous pression réduite (2,7 kPa). Le résidu est repris par 100 $cm^3$ d'eau distillée et 100 $cm^3$ d'acétate d'éthyle ; le mélange est refroidi à +5°C, puis acidifié par addition d'acide chlorhydrique 4N jusqu'à pH = 1, soit 35 $cm^3$. La phase organique est lavée par 100 $cm^3$ d'eau distillée (3 fois), séchée sur sulfate de magnésium puis concentrée à sec sous pression réduite (2,7 kPa). On obtient 11,3 g d'acide tert-butoxycarbonylamino-3 phényl-2 propionique-(S), fondant à 138°C ;
$[\alpha]_D^{20}$= + 94° (c=0,25 , méthanol).

L'acide amino-3 phényl-2 propionique-(S) peut être préparé selon une méthode décrite par J.A. Carbarino dans J. Chem. Soc. Perkin 1, 906 (1981).

## EXEMPLE 80

A une solution de 1,23 g d'acide α-méthoxy phénylacétique -(S) dans 20 $cm^3$ de diméthylformamide sec, refroidie à +5°C, on ajoute 1 g d'hydroxybenzotriazole, monohydrate ; on poursuit l'agitation 15 minutes, puis on ajoute 1,53 g de NN'-dicyclohexylcarbodiimide . On agite 1 heure à +5°C puis on ajoute une solution de 2,43 g de chlorhydrate de diphényl-7,7 perhydroisoindolone-4-(3aR,7aR) et de 1,28 $cm^3$ de diisopropyléthylamine dans 10 $cm^3$ de diméthylformamide sec. Le mélange réactionnel est agité à +5°C pendant 2 heures, puis à température ambiante pendant 2 heures. Le mélange réactionnel, repris par 100 $cm^3$ d'acétate d'éthyle, est filtré; le filtrat est lavé par 100 $cm^3$ d'eau distillée (2 fois), séché sur sulfate de magnésium, filtré et concentré à sec sous pression réduite (2,7 kPa). Le résidu est chromatographié sur une colonne de gel de silice (diamètre 2,2 cm, hauteur 27 cm) en éluant par un mélange de cyclohexane et d'acétate d'éthyle (80/20 en volumes) et en recueillant des fractions de 20 $cm^3$. Les fractions 68 à 177 sont réunies et concentrées à sec sous pression réduite (2,7 kPa). Le résidu est cristallisé dans 15 $cm^3$ d'oxyde d'isopropyle ; les cristaux obtenus sont essorés

puis séchés. On obtient 1,3 g de diphényl-7,7 [méthoxy-2 phényl-2 acétyl-(S)]-2 perhydroisoindolone-4-(3aR,7aR), sous la forme de cristaux blancs fondant à 130°C;
$[\alpha]_D^{20}$ = -230° (c=1 , méthanol).

EXEMPLE 81

A une solution de 1,55 g d'acide (S)-(+) O-acétylmandélique dans 60 $cm^3$ de dichlorométhane sec, refroidie à +5°C, on ajoute 1,3 g de N,N'-carbonyldiimidazole. On agite 90 minutes à +5°C puis on ajoute une solution de 2,6 g de chlorhydrate de diphényl-7,7 perhydroisoindolone-4-(3aR,7aR) et de 1,12 $cm^3$ de triéthylamine dans 80 $cm^3$ de dichlorométhane. Le mélange réactionnel est agité à 20°C pendant 16 heures, concentré à sec sous pression réduite (2,7 kPa). Le résidu est repris dans un mélange de 150 $cm^3$ d'acétate d'éthyle et de 100 $cm^3$ d'eau et la phase organique est lavée par 50 $cm^3$ d'eau puis par 50 $cm^3$ de solution saturée de chlorure de sodium, séchée sur sulfate de magnésium, filtrée et concentrée à sec sous pression réduite (2,7 kPa). Le résidu est chromatographié sur une colonne de gel de silice (0,4-0,063 mm, diamètre 4 cm, hauteur 23 cm) en éluant sous une pression de 0,5 bar d'azote par un mélange de cyclohexane et d'acétate d'éthyle (50/50 en volumes) et en recueillant des fractions de 60 $cm^3$. Les fractions 13 à 19 sont réunies et concentrées à sec sous pression réduite (2,7 kPa) pour donner 2,9 g d'[acétoxy-2 phényl-2 acétyl-(S)]-2 diphényl-7,7 perhydroisoindolone-4-(3aR,7aR) sous la forme d'une meringue blanche;
$[\alpha]_D^{20}$= -186,4° (c=1,0, méthanol).
Spectre RMN du proton :
A température ambiante, on observe le mélange des deux rotamères.
2 et 2,05 (2S, $-OCOCH_3$) ; 1,9 à 2,3 (Mt, $-CH_2-$ en 5 ou 6) ; 2,6 à 3 (Mt, 4H, $-CH_2-$ en 6 ou 5 et $-CH_2-$ en 1) ; 3,05 à 3,4 (Mt, 2H, 1H du $-CH_2-$ en 3 et $-CH_2-$ en 3a) ; 3,75 à 4,1 (Mt, 1H, -CH- en 7a) ; 4,15 à 4,5 (Mt, 1H, 1H du $-CH_2-$ en 6 ou 5) ; 5,65 et 6,14 (2S, 1H en totalité, -N-CO-CH-) ; 7,05 à 7,6 (Mt, 15H, aromatiques).
Spectre infra-rouge (KBr) bandes caractéristiques ($cm^{-1}$):
3090-3065, 2980-2880, 1735, 1720, 1660, 1600, 1585, 1495, 1460, 1445, 1435, 1375, 1240, 700.

EXEMPLE 82

Une solution de 2 g d'[acétoxy-2 phényl-2 acétyl-(S)]-2 diphényl-7,7 perhydroisoindolone-4-(3aR,7aR) dans un mélange de 20 $cm^3$ d'eau et 25 $cm^3$ d'éthanol est traitée par 5 $cm^3$ de soude normale et chauffée au reflux pendant 1 heure puis diluée par 100 $cm^3$ d'eau et 50 $cm^3$ d'acétate d'éthyle et neutralisée par addition de 5 $cm^3$ d'acide chlorhydrique normal. La phase organique est lavée à l'eau (50 $cm^3$) et par une solution saturée de chlorure de sodium (50 $cm^3$), séchée sur sulfate de magnésium et concentrée à sec sous pression réduite (2,7 kPa) pour donner 1,3 g d'[hydroxy-2 phényl-2 acétyl-(S)]-2 diphényl-7,7 perhydroisoindolone-4-(3aR,7aR), sous la forme d'une meringue blanche.
Spectre RMN du proton :
A température ambiante, on observe le mélange des deux rotamères.
1,95 à 2,45 (Mt, 2H, $-CH_2-$ en 5 ou 6) ; 2,6 à 3 (Mt, 4H, $-CH_2-$ en 6 ou 5 et $-CH_2-$ en 1) ; 3,8 à 4 (Mt, 1H, -CH- en 7a) ; 4,10 à 4,35 (Mt, 1H, 1H du $-CH_2-$ en 3) ; 4,90 et 5,25 (2D, J = 6, non échangeable, 1H en totalité, -N-CO-CH-O-) ; 5,47 et 5,6 (2D, J = 6, échangeable, 1 H en totalité, -OH) ; 7 à 7,7 (Mt, 15H, aromatiques).
Spectre infra-rouge (KBr) bandes caractéristiques ($cm^{-1}$):
3410, 3100-3000, 3000-2870, 1715, 1640, 1600, 1580, 1492, 1460, 1445, 1380, 1065, 755 et 700.

L'[acétoxy-2 phényl-2 acétyl-(S)]-2 diphényl-7,7 perhydroisoindolone-4-(3aR,7aR) peut être préparé comme décrit précédemment à l'exemple 81.

EXEMPLE 83

A une solution de 40,5 g d'ester monobenzylique de l'acide phénylmalonique dans 810 $cm^3$ de dichlorométhane sec, refroidie à +5°C, on ajoute 24,3 g de N,N'-carbonyldiimidazole. On agite 25 minutes à +5°C puis on ajoute une solution de 49,1 g de chlorhydrate de diphényl-7,7 perhydroisoindolone-4-(3aRS,7aRS) et de 21 $cm^3$ de triéthylamine dans 1000 $cm^3$ de dichlorométhane. Le mélange réactionnel est agité à 20°C pendant 16 heures, puis lavé par 1000 $cm^3$ d'eau distillée (3 fois), séché sur sulfate de magnésium, filtré et concentré à sec sous pression réduite (2,7 kPa). Le résidu est chromatographié sur une colonne de gel de silice (0,2-0,063 mm, diamètre 5,8 cm , hauteur 63 cm) en éluant avec un mélange d'acétate d'éthyle et de cyclohexane (90/10 en volumes) et en recueillant des fractions de 200 $cm^3$. Les fractions 14 à 17 sont réunies et concentrées à sec sous pression réduite (2,7 kPa). Le résidu est cristallisé dans 400 $cm^3$ d'oxyde d'isopropyle ; les cristaux obtenus sont essorés, lavés par 40 $cm^3$ d'oxyde d'isopropyle et séchés. 5 g des 35,6 g de cristaux obtenus

sont chromatographiés sur une colonne de gel de silice (0,2-0,063 mm, diamètre 4,6 cm, hauteur 30 cm) en éluant par un mélange de cyclohexane et d'acétate d'éthyle (60/40 en volumes) et en recueillant des fractions de 100 $cm^3$. Les fractions 4 à 10 sont réunies et concentrées à sec sous pression réduite (2,7 kPa) ; le résidu est recristallisé dans 5 $cm^3$ d'acétonitrile. Les cristaux obtenus sont essorés, lavés par 5 $cm^3$ d'oxyde d'isopropyle et séchés. On obtient 1,6 g de (benzyloxycarbonyl-2 phényl-2 acétyl)-2 diphényl-7,7 perhydroisoindolone-4-(3aRS,7aRS), fondant à 210°C.

EXEMPLE 84

A une suspension de 5 g (benzyloxycarbonyl-2 phényl-2 acétyl)-2 diphényl-7,7 perhydroisoindolone-4-(3aRS,7aRS) dans 50 $cm^3$ de méthanol, on ajoute, sous agitation, 1g de palladium sur charbon à 5%. Le mélange réactionel est hydrogéné, sous pression atmosphérique et à température ambiante pendant 3,5 heures puis filtré et concentré à sec sous pression réduite (2,7 kPa) ; le résidu est traité par 5 $cm^3$ d'une solution de soude agueuse 1N refroidie à +5°C, puis par 40 $cm^3$ d'eau distillée et 40 $cm^3$ d'acétate d'éthyle. La solution aqueuse est extraite par 40 $cm^3$ d'acétate d'éthyle ; les solutions organiques sont réunies, refroidies à +5°C et acidifiées par addition de 4 $cm^3$ d'acide chlorhydrique 4N ; le précipité obtenu est dissous dans 50 $cm^3$ d'acétate d'éthyle et la solution organique est lavée par 40 $cm^3$ d'eau distillée (3 fois), séchée sur sulfate de magnésium, filtrée et concentrée à sec sous pression réduite (2,7 kPa). Le résidu, repris par 5 $cm^3$ d'oxyde d'isopropyle est essoré et séché. On obtient 2 g de [(carboxy-2 phényl-2) acétyl)-2 diphényl-7,7 perhydroisoindolone-4-(3aRS,7aRS), fondant à 60°C avec décomposition.

La (benzyloxycarbonyl-2 phényl-2 acétyl)-2 diphényl-7,7 perhydroindolone-4-(3aRS,7aRS) peut être préparée comme décrit précédemment à l'exemple 83.

EXEMPLE 85

En opérant comme décrit précédemment à l'exemple 83, mais à partir de l'ester méthylique de l'acide phénylmalonique, on obtient 0,53 g de [(méthoxycarbonyl-2 phényl-2) acétyl]-2 diphényl-7,7 perhydroisoindolone-4-(3aRS,7aRS) sous forme d'une meringue blanche.

Spectre RMN du proton :

A température ambiante, on observe le mélange des deux rotamères de chacun des deux diastéréoisomères. 1,85 à 2,3 (Mt, 2H, $-CH_2-$ en 5 ou 6) ; 2,6 à 3,2 (Mt, 4H, $-CH_2-$ en 6 ou 5 et $-CH_2-$ en 1) ; 3 à 3,5 (Mt, 2H, 1H du $-CH_2-$ en 3 et -CH- en 3a) ; 3,48 - 3,58 - 3,6 et 3,62 (4S, 3H en totalité, $-COOCH_3$) ; 3,65 à 4,42 (Mt, -CH- en 7a et 1H du $-CH_2-$ en 3) ; 4,6 - 4,63 - 5 et 5,08 (4S, 1H en totalité, -N-COCH-COO-) ; 6,9 à 7,65 (Mt, 15H, aromatiques).

Spectre infra-rouge (KBr) bandes caractéristiques ($cm^{-1}$):

3600-3300, 3100-3000, 3000-2800, 1745, 1715, 1650, 1600, 1580, 1495, 1475, 1445, 1400, 1195, 755, 700.

EXEMPLE 86

A une solution de 1,53 g de (L)-N-α-tert-butoxycarbonyl phénylglycine dans 25 $cm^3$ de diméthylformamide sec, refroidie à +5°C, on ajoute 0,825 g d'hydroxy-1 benzotriazole, puis 1,25 g de N,N'-dicyclohexylcarbodiimide ; après 1 heure d'agitation à la même température on ajoute une solution de 2 g de chlorhydrate de diphényl-7,7 perhydroisoindolone-4-(3aR,7aR) et de 1,05 $cm^3$ de N,N diisopropyléthylamine dans 10 $cm^3$ de diméthylformamide. Le mélange réactionnel est agité 2 heures à + 5°C et 1,5 heure à 20°C, puis dilué par 250 $cm^3$ d'acétate d'éthyle et, après filtration du précipité, lavé par 100 $cm^3$ d'eau (2 fois) et par 100 $cm^3$ de solution saturée de chlorure de sodium et séché sur sulfate de magnésium. Après concentration à sec sous pression réduite (2,7 kPa) le résidu est chromatographié sur une colonne de gel de silice (0,2-0,063 mm, diamètre 3,8 cm, hauteur 31 cm) en éluant sous une pression de 0,5 bar d'azote, par un mélange de cyclohexane et d'acétate d'éthyle (75/25 en volumes) et en recueillant des fractions de 25 $cm^3$. Les fractions 9 à 20 sont réunies et concentrées à sec sous pression réduite (2,7 kPa) pour donner 5,3 g de [tert-butoxycarbonylamino-2 phényl-2 acétyl(S)]-2 diphényl-7,7 perhydroisoindolone-4-(3aR,7aR) sous la forme d'une meringue blanche.

On traite 5 g de [tert-butoxycarbonylamino-2 phényl-2 acétyl-(S)]-2 diphényl-7,7 perhydroisoindolone-4-(3aR,7aR) par 50 $cm^3$ d'une solution 5,7 N d'acide chlorhydrique dans le dioxanne sec pendant 30 minutes à +5°C puis 1 heure à 20°C. Le mélange réactionnel est concentré à sec sous pression réduite (2,7 kPa) et le résidu dissous dans 50 $cm^3$ d'eau et la solution obtenue est lavée par 60 $cm^3$ d'acétate d'éthyle (2 fois) et par 60 $cm^3$ d'éther éthylique puis filtrée et lyophilisée. Le solide est repris par 25 $cm^3$ d'eau et la suspension est de nouveau concentrée à sec sous pression réduite (2,7 kPa). Le solide est trituré avec 10 $cm^3$ d'acétone, essoré, lavé 2 fois par 2,5 $cm^3$ d'acétone et séché. On obtient 2,53 g de chlorhydrate de diphényl-7,7 [(L)-α-phénylglycyl]-2 perhydroisoindolone-4-(3aR,7aR) sous la forme d'un solide blanc partiellement hydraté ;
$[\alpha]_D^{20}$= - 234° (c=0,3 , eau).
Spectre RMN du proton :
A température ambiante, on observe le mélange des deux rotamères.
1,75 à 2,35 (Mt, 2H, $-CH_2-$ en 5 ou 6) ; 2,6 à 3 (Mt, 4H, $-CH_2-$ en 6 ou 5 et $-CH_2-$ en 1) ; 3,25 à 3,45 (Mt, en partie masqué, 1H du $-CH_2-$ en 3 et -CH- en 3a) ; 3,85 à 4,15 (Mt, 1H, -CH- en 7a) ; 4,25 à 4,45 (Mt, 1H, 1H du $-CH_2-$ en 3) ; 5,02 et 5,4 (2S, 1H en totalité, -N-CO-CH-N-) ; 6,95 à 7,65 (Mt, 14H, aromatiques) ; 8,65 (Mf, 3H, $-NH_3^+Cl^-$).
Spectre infra-rouge (KBr) bandes caractéristiques ($cm^{-1}$):
3600-3300, 3100-3000, 3000-2850, 3150-2500, 1715, 1655, 1595, 1580, 1495, 1475, 1445, 1440, 755, 700

EXEMPLE 87

En opérant de la même manière qu'à l'exemple 86, mais à partir de (L)-N-acétyl α-phénylglycine, on obtient 0,37 g d' [acétylamino-2 phényl-2 acétyl-(S)]-2 diphényl-7,7 perhydroisoindolone-4-(3aR,7aR), fondant à 190°C ;
$[\alpha]_D^{20}$ = -159° (c=1, méthanol).

EXEMPLE 88

A une solution de 2,35 g d'acide (cyclohexadièn-1,4 yl-1)acétique dans 50 $cm^3$ de dichlorométhane sec, refroidie à +5°C, on ajoute 3,6 g de N,N'-carbonyldiimidazole. On agite 30 minutes à +5°C puis on ajoute une solution de 6,55 g de chlorhydrate de diphényl-7,7 perhydroisoindolone-4 et de 5,6 $cm^3$ de triéthylamine dans 30 $cm^3$ de dichlorométhane. Le mélange réactionnel est agité 1 heure à +5°C, puis 16 heures à 20°C et lavé par 150 $cm^3$ d'acide chlorhydrique normal et 150 $cm^3$ d'une solution aqueuse saturée de chlorure de sodium. La phase organique est séchée sur sulfate de magnésium, filtrée et concentrée à sec sous pression réduite (2,7 kPa). Le résidu est chromatographié sur une colonne de gel de silice (granulométrie 0,2-0,063 mm, diamètre 6 cm, hauteur 30 cm) en éluant sous une pression de 0,5 bar d'azote par 3 litres de mélange de cyclohexane et d'acétate d'éthyle (50/50) puis par 2 litres d'acétate d'éthyle et en recueillant des fractions de 100 $cm^3$. Les fractions 10 à 48 sont réunies et concentrées à sec sous pression réduite (2,7 kPa). Le résidu est recristallisé dans 15 $cm^3$ d'acétonitrile. Les cristaux sont essorés, lavés par 5 $cm^3$ d'acétonitrile et 25 $cm^3$ d'oxyde d'isopropyle et séchés. On obtient 5,7 g (cyclohexadièn-1,4 yl-1)acétyl-2 diphényl-7,7 perhydroisoindolone-4-(3aRS,7aRS). P.F. =186°C .

EXEMPLE 89

Une solution de 3,7 g de (cyclohexadièn-1,4 yl-1)acétyl-2 diphényl-7,7 perhydroisoindolone-4-(3aRS,7aRS) dans 20 $cm^3$ de dichlorométhane sec est traitée par 1,9 g de tétrafluoroborate de triéthyloxonium et agitée pendant 22 heures à 20°C. Les cristaux formés sont essoré, lavés par 10 $cm^3$ de dichlorométhane et séchés pour donner 2 g de tétrafluoroborate d'[éthoxy-1 (cyclohexadièn-1,4 yl-1)-2 éthylidène]-2 oxo-4 diphényl-7,7 perhydroisoindolium-(3aRS,7aRS) sous la forme d'une poudre blanche utilisée à l'état brut pour la phase suivante.

A une suspension agitée et refoidie à -15°C de 2 g de tetrafluoroborate d'[éthoxy-1 (cyclohexadièn-1,4 yl-1)-2 éthylidène]-2 oxo-4 diphényl-7,7 perhydroisoindolium-(3aRS,7aRS) dans 30 $cm^3$ de dichlorométhane anhydre, on ajoute 0,7 $cm^3$ d'une solution 5,4N d'ammoniac dans l'éthanol. Après retour à la température ambiante le mélange réactionnel est agité 20 heures puis lavé 2 fois par 35 $cm^3$ d'une solution aqueuse à 20% de carbonate de potassium. La phase organique est séchée sur sulfate de magnésium et concentrée à sec sous pression réduite (2,7 kPa). Le résidu est cristallisé dans 5 $cm^3$ d'acétonitrile. Les cristaux obtenus sont essorés lavés à l'acétonitrile et séchés. On obtient 0,6 g de [(cyclohexadièn-1,4 yl-1)-2 imino-1 éthyl]-2 diphényl-7,7 perhydroisoindolone-4-(3aRS,7aRS) sous la forme de cristaux blancs. P.F. = 190°C.

EXEMPLE 90

A une solution de 2 g de chlorhydrate de diphényl-7,7 hexahydro-2,3,3a,4,7,7a 1H-isoindolone-4 et de 1,7 $cm^3$ de triéthylamine dans 20 $cm^3$ de dichlorométhane sec, refroidie à +5°C, on ajoute 0,82 $cm^3$ de chlorure de phénylacétyle. Le mélange réactionnel est agité 1 heure à +5°C et 1 heure à température ambiante ; il est lavé par 20 $cm^3$ d'eau distillée (2 fois), séché sur sulfate de magnésium, filtré et concentré à sec sous pression réduite (2,7 kPa). Le résidu est cristallisé dans 15 $cm^3$ d'acétonitrile. Les cristaux sont essorés, lavés par 10

$cm^3$ d'oxyde d'isopropyle, séchés, puis recristallisés dans 20 $cm^3$ d'acétonitrile. Les cristaux obtenus sont essorés et séchés. On obtient 2,7 g de diphényl-7,7 (phénylacétyl)-2 hexahydro-2,3,3a,4,7,7a 1H-isoindolone-4-(3aRS,7aRS), fondant à 188°C.

Le chlorhydrate de diphényl-7,7 hexahydro-2,3,3a,4,7,7a 1H-isoindolone-4 peut être préparé de la manière suivante :

Un mélange de 3,4 g de benzyl-2 diphényl-7,7 hexahydro-2,3,3a,4,7,7a 1H-isoindolone-4 et de 0,92 $cm^3$ de chloroformiate de vinyle dans 80 $cm^3$ de dichlororo-1,2 éthane est chauffé au reflux pendant 1 heure ; le mélange réactionnel est concentré à sec sous pression réduite (2,7kPa). Le résidu est cristallisé dans 20 $cm^3$ d'éther éthylique. On obtient 2,6 g de diphényl-7,7 vinyloxycarbonyl-2 hexahydro-2,3,3a,4,7,7a 1H-isoindolone-4, fondant à 162°C.

2,6 g de diphényl-7,7 vinyloxycarbonyl-2 hexahydro-2,3,3a,4,7,7a 1H-isoindolone-4 sont agités dans 30 $cm^3$ de dioxanne chlorhydrique 3N à température ambiante pendant 30 minutes ; le mélange est concentré à sec sous pression réduite (2,7 kPa). Le résidu est repris par 50 $cm^3$ d'éthanol et chauffé au reflux pendant 30 minutes ; le mélange est concentré à sec sous pression réduite (2,7 kPa). Le résidu est cristallisé dans 20 $cm^3$ d'éther éthylique ; les cristaux obtenus sont essorés et séchés. On obtient 2 g de chlorhydrate de diphényl-7,7 hexahydro-2,3,3a,4,7,7a 1H-isoindolone-4, fondant à une température supérieure à 260°C.

La benzyl-2 diphényl-7,7 hexahydro-2,3,3a,4,7,7a 1H-isoindolone-4-(3aRS,7aRS) peut être obtenue de la manière suivante :

A une solution de 7,7 g de diphényl-4,4 cyclohéxadiène-2,5 one-1 et de 11 $cm^3$ de N-butoxyméthyl N-triméthylsilylméthyl benzylamine dans 80 $cm^3$ de dichlorométhane sec, on ajoute 2 gouttes d'acide trifluoroacétique et chauffe le mélange réactionnel au reflux pendant 1 heure et demi. On ajoute 5 $cm^3$ de N-butoxyméthyl N-triméthylsilylméthyl benzylamine supplémentaires ainsi que 2 gouttes d'acide trifluoroacétique et chauffe le mélange réactionnel pendant 1 heure et demi. Le mélange réactionnel est traité par 3 g de carbonate de potassium, filtré et concentré à sec sous pression réduite (2,7 kPa). Le résidu est cristallisé dans 15 $cm^3$ d'oxyde d'isopropyle. Les cristaux obtenus sont essorés, lavés 5 $cm^3$ d'oxyde d'isopropyle (2 fois) et séchés ; on obtient 4,4 g de benzyl-2 diphényl-7,7 hexahydro-2,3,3a,4,7,7a 1H-isoindolone-4-(3aRS,7aRS), fondant à 132°C.

La diphényl-4,4 cyclohexadiène-2,5 one-1 peut être préparée selon la méthode de H.E. Zimmermann et D.I.Schuster J. Am. Chem. Soc., 84, 527 (1962).

EXEMPLE 91

Une suspension de 1,5 g de chlorhydrate de bis(fluoro-3 phényl)-7,7 perhydroisoindolone-4 dans 30 $cm^3$ de dichlorométhane refroidie à +4°C est traitée par 1,15 $cm^3$ de triéthylamine puis par 0,63 g de chlorure de phénylacétyle. Le mélange réactionnel est agité 5 heures à 25°C, puis lavé 3 fois par 100 $cm^3$ d'eau. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,3 cm, hauteur 25 cm) en éluant sous une pression de 0,5 bar d'azote par un mélange de cyclohexane et d'acétate d'éthyle (55/45) pour donner 1,21 g de meringue qui est cristallisée par addition de 10 $cm^3$ d'oxyde d'isopropyle. Les cristaux sont essorés, lavés par de l'oxyde d'isopropyle et séchés. On obtient 0,76 g de bis(fluoro-3 phényl)-7,7 (phénylacétyl)-2 perhydroisoindolone-4-(3aRS,7aRS). P.F. = 108°C.

Le chlorhydrate de bis(fluoro-3 phényl)-7,7 perhydroisoindolone-4 peut être obtenu de la manière suivante :

Une solution de 92,2 g de benzyl-2 bis(fluoro-3 phényl)-7,7 perhydroisoindolone-4-(3aRS,7aRS) dans 860 $cm^3$ de dichloro-1,2 éthane est traitée par 26,3 $cm^3$ de chloroformiate de vinyle et chauffée 3 heures au reflux puis concentrée sous pression réduite (2,7 kPa). Le résidu est chromatographié (en deux fois) sur gel de silice (granulomètrie 0,04-0,063 mm, colonnes de diamètre 8 cm et hauteur 35 cm) en éluant sous une pression de 0,5 bar d'azote par un mélange de cyclohexane et d'acétate d'éthyle (75/25). La meringue obtenue est cristallisée dans de l'oxyde d'isopropyle pour donner 50,3 g de bis(fluoro-3 phényl)-7,7 vinyloxycarbonyl-2 perhydroisoindolone-4-(3aRS,7aRS). P.F. = 152°C.

64,5 g de bis(fluoro-3 phényl)-7,7 vinyloxycarbonyl-2 perhydroisoindolone-4-(3aRS,7aRS) sont traités par 330 $cm^3$ d'une solution 6N d'acide chlorhydrique dans le dioxanne pendant 30 minutes à 25°C. La solution est concentrée à sec sous pression réduite (2,7 kPa) et le résidu repris par 500 $cm^3$ d'éthanol. La solution est chauffée à 60°C pendant 6 heures et agitée 16 heures à 25°C puis concentrée de moitié sous pression réduite (2,7 kPa) et les cristaux formés sont essorés et lavés par de l'oxyde d'isopropyle puis sèchés. On obtient 48,7 g de chlorhydrate de bis(fluoro-3 phényl)-7,7 perhydroisoindolone-4. P.F. = 264°C

La benzyl-2 bis(fluoro-3 phényl)-7,7 perhydroisoindolone-4-(3aRS,7aRS) peut être obtenue de la manière suivante :

A une solution de 90,3 g de bis(fluoro-3 phényl)-4,4 cyclohexènone et de 123 $cm^3$ de N-butoxyméthyl N-tri-

méthylsilylméthyl benzylamine dans 1000 $cm^3$ de dichlorométhane sec, on ajoute 3 $cm^3$ d'acide trifluoroacétique. Le mélange réactionnel est porté ou reflux puis agité 2 heures en laissant revenir la température à 25°C et encore 15 minutes après addition de 60 g de carbonate de potassium. Après filtration et concentration à sec sous pression réduite (2,7 kPa), le résidu cristallisé est battu avec de l'oxyde d'isopropyle, essoré lavé et recristallisé dans 300 $cm^3$ de cyclohexane. Les cristaux sont essorés, lavés 2 fois par 15 $cm^3$ de cyclohexane et séchés pour donner 92 g de benzyl-2 bis(fluoro-3 phényl)-7,7 perhydroisoindolone-4-(3aRS,7aRS) sous la forme de cristaux blancs. P.F. = 124°C.

La bis(fluoro-3 phényl)-4,4 cyclohexènone peut être obtenue de la manière suivante :
A une solution de 144,5 g de bis(fluoro-3 phényl)acétaldéhyde dans 500 $cm^3$ d'éther éthylique, on ajoute 50,4 $cm^3$ de butènone, puis après refroidissement à 0°C, goutte à goutte une solution de 13,9 g de potasse dans 89 $cm^3$ d'éthanol. Le mélange réactionnel est agité 2 h à 0°C puis 16 heures à 25 °C, dilué par 300 $cm^3$ d'acétate d'éthyle et 500 $cm^3$ d'eau. La phase aqueuse est lavée par 300 $cm^3$ d'acétate d'éthyle. Les phases organiques réunies sont lavées par 500 $cm^3$ de solution saturée de chlorure de sodium, séchées sur sulfate de magnésium et concentrées sous pression réduite (2,7 kPa). Le résidu est chromatographié (en 2 fois) sur gel de silice (granulomètrie 0,04-0,063 mm, colonnes diamètre 8,5 cm, hauteur 34 cm) en éluant sous une pression de 0,5 bar d'azote par un mélange de cyclohexane et d'acétate d'éthyle (90/10). On obtient 90,3 g de bis(fluoro-3 phényl)-4,4 cyclohexènone, sous forme de cristaux blancs. P.F. = 95°C

Le bis(fluoro-3 phényl)acétaldéhyde peut être obtenu de la manière suivante :
Une solution de 156,7 g de bis(fluoro-3 phényl)-1,1 méthoxy-2 éthanol (obtenu par réaction de bromure de (fluoro-3 phényl)magnésium sur du méthoxy-2 acétate de méthyle dans le THF) dans 160 $cm^3$ d'acide formique est chaufée au reflux 16 heures, refroidie et versée dans un mélange de 800 $cm^3$ de solution saturée de carbonate de sodium et de 500 $cm^3$ d'acétate d'éthyle. La phase organique est lavée 2 fois par 500 $cm^3$ d'eau et par 500 $cm^3$ de solution saturée de chlorure de sodium puis séchée et concentrée à sec sous pression réduite (2,7 kPa) pour donner 144,5 g de bis(fluoro-3 phényl)acétaldéhyde sous la forme d'une huile jaune.

EXEMPLE 92

Une solution de 0,46 g d'acide (méthoxy-2 phényl)acétique dans 15 $cm^3$ de dichlorométhane sec est refroidie à 0°C puis traitée par 0,45 g de N,N'-carbonyldiimidazole et agitée pendant 1 heure à 0°C. On ajoute goutte à goutte une solution de 1 g de chlorhydrate de bis(fluoro-3 phényl)-7,7 perhydroisoindolone-4 et 0,76 $cm^3$ de triéthylamine dans 20 $cm^3$ de dichlorométhane. Le mélange réactionnel est agité 3 heures à 25°C) puis lavé 2 fois par 50 $cm^3$ d'eau et par 50 $cm^3$ de solution saturée de chlorure de sodium. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,2 cm, hauteur 23 cm) en éluant sous une pression de 0,5 bar d'azote par un mélange de cyclohexane et d'acétate d'éthyle (70/30) et en recueillant des fractions de 15 $cm^3$. Les fractions 4 à 9 sont réunies et concentrées à sec sous pression réduite (2,7 kPa) et le produit obtenu est recristallisé dans de l'acétonitrile. Les cristaux sont essorés, lavés par de l'oxyde d'isopropyle et séchés. On obtient 0,76 g de bis(fluoro-3 phényl)-7,7 ((méthoxy-2 phényl)acétyl)-2 perhydroisoindolone-4-(3aRS,7aRS). P.F. = 194°C.

EXEMPLE 93

A une solution de 0,65 g d'acide (diméthylamino-2 phényl)acétique, dans 20 $cm^3$ de dichlorométhane sec refroidie à +4°C, on ajoute 0,59 g de N,N'-carbonyldiimidazole. Le mélange est agité 90 minutes à 25 °C puis on ajoute goutte à goutte une solution de 1,3 g de chlorhydrate de bis(fluoro-3 phényl)-7,7 perhydroisoindolone-4 et de 1,02 $cm^3$ de triéthylamine dans 25 $cm^3$ de dichlorométhane sec. Le mélange réactionnel est agité 16 heures à 25°C et lavé 2 fois par 250 $cm^3$ d'eau et par 250 $cm^3$ de solution saturée de chlorure de sodium. La phase organique est séchée sur sulfate de magnésium et concentrée à sec sous pression réduite (2,7 kPa). Le résidu est chromatographié sur une colonne de gel de silice (granulomètrie 0,04-0,063 mm, diamètre 2,3 cm, hauteur 23 cm) en éluant sous une pression de 0,5 bar d'azote par un mélange de cyclohexane et d'acétate d'éthyle (55/45) et en recueillant des fractions de 15 $cm^3$. Les fractions 6 à 18 sont réunies et concentrées à sec sous pression réduite (2,7 kPa). Le résidu est concrété par de l'oxyde d'isopropyle pour donner 0,6 g de bis(fluoro-3 phényl)-7,7 (diméthylamino-2 phényl)acétyl-2 perhydroisoindolone-4-(3aRS,7aRS) dont on prépare le chlorhydrate par dissolution dans 1 $cm^3$ d'acétate d'éthyle et addition d'une solution 3N d'acide chlorhydrique dans l'oxyde d'isopropyle. Le précipité est essoré, lavé par de l'oxyde d'isopropyle et séché. On obtient 0,48 g de chlorhydrate de bis(fluoro-3 phényl)-7,7 (diméthylamino-2 phényl)acétyl-2 perhydroisoindolone-4-(3aRS,7aRS) sous la forme d'un solide blanc.
Spectre RMN du proton ($DMSO-d_6$/AcOD 90/10).

A température ordinaire on observe le mélange des deux rotamères. 2 à 2,32 (m, 2H, $-CH_2-$ en 5) ; 2,37 et 2,6 (2s, 3H chacun, $-N(CH_3)_2$) ; 2,65 à 3 (m, 4H, $-CH_2-$ en 6 et $-CH_2-$ en 1) ; 3,15 à 3,3 (m, 1H, H en 3a) ; 3,35 et 3,47 (2m, 1H, 1H en 3) ; 3,35 et 3,5 (2d, J=15, $ArCH_2CO$ d'un rotamère) ; 3,67 (s, $ArCH_2CO$ de l'autre rotamère) ; 4 (m, 1H, H en 7a) ; 4,2 et 4,25 (2m, J=11, 1H , 1H en 3) ; 6,9 à 7,6 (m, 12H, aromatiques)
Spectre infrarouge (bandes caractéristiques en cm-1): 3500-3150, 3100-3000, 3000-2850, 1712, 1650, 1615, 1595, 1580, 1495, 1445, 1535, 755, 700.

EXEMPLE 94

A une solution de 0,49 g d'acide (diméthylamino-2 phényl)acétique, dans 20 $cm^3$ de dichlorométhane sec refroidie à +4°C, on ajoute 0,44 g de N,N'-carbonyle diimidazole. Le mélange est agité 1 heure à 25 °C puis on ajoute goutte à goutte une solution de 1 g de chlorhydrate de bis(fluoro-2 phényl)-7,7 perhydroisoindolone-4 et de 0,76 $cm^3$ de triéthylamine dans 25 $cm^3$ de dichlorométhane sec. Le mélange réactionnel est agité 20 heures à 25°C et lavé 2 fois par 100 $cm^3$ d'eau et par 100 $cm^3$ de solution saturée de chlorure de sodium. La phase organique est séchée sur sulfate de magnésium et concentrée à sec sous pression réduite (2,7 kPa). Le résidu est chromatographié sur une colonne de gel de silice (granulomètrie 0,04-0,063 mm, diamètre 2 cm, hauteur 23 cm) en éluant sous une pression de 0,5 bar d'azote par un mélange de cyclohexane et d'acétate d'éthyle (50/50) et en recueillant des fractions de 10 $cm^3$. Les fractions 14 à 36 sont réunies et concentrées à sec sous pression réduite (2,7 kPa) pour donner 1 g de bis(fluoro-2 phényl)-7,7 (diméthylamino-2 phényl)acétyl-2 perhydroisoindolone-4-(3aRS,7aRS) dont on prépare le chlorhydrate par dissolution dans 2 $cm^3$ d'acétate d'éthyle et addition d'une solution 3N d'acide chlorhydrique dans l'oxyde d'isopropyle. Le précipité est essoré, lavé par de l'oxyde d'isopropyle et séché. On obtient 0,87 g de chlorhydrate de bis(fluoro-2 phényl)-7,7 (diméthylamino-2 phényl)acétyl-2 perhydroisoindolone-4-(3aRS,7aRS) sous la forme d'un solide blanc.
Spectre RMN du proton (DMSO-$d_6$/AcOD 90/10) :
A température ambiante on observe le mélange des deux rotamères:
2,1 à 2,35 (m, 2H, $-CH_2-$ en 5) ; 2,8 à 3,4 (m, 10H, $-CH_2-$ en 1 et en 6 , $N(CH_3)_2$) ; 3,7 et 3,5 (2 dd larges, 1H, H en 3a) ; 3,8 (dd large, 1H, 1H en 3) ; 4,05 (s large, 2H, $-CH_2CO$) ; 4,1 (m large, 1H, H en 7a) ; 4,2 et 4,45 (d, 1H, 1H en 3) ; 7 à 8 (m,12H aromatiques).

Le chlorhydrate de bis(fluoro-2 phényl)-7,7 perhydroisoindolone-4-(3aRS,7aRS) peut être préparé de la manière suivante :
Une solution de 2,34 g de benzyl-2 bis(fluoro-2 phényl)-7,7 perhydroisoindolone-4-(3aRS,7aRS) dans 100 $cm^3$ de méthanol additionné de 6,2 $cm^3$ d'acide chlorhydrique N est hydrogénée sous pression atmosphérique en présence de 0,4 g de palladium sur charbon à 10 %, pendant 5 heures à 25 °C. Le mélange réactionnel est filtré et concentré sous pression réduite (2,7 kPa) pour donner 2 g de chlorhydrate de bis(fluoro-2 phényl)-7,7 perhydroisoindolone-4-(3aRS,7aRS) sous la forme d'un solide blanc.
Spectre RMN du proton (DMSO-$d_6$):
2 à 2,4 (m, 2H, $-CH_2-$ en 5) ; 2,7 à 3 (m, 4H, $-CH_2-$ en 1 et en 6) ; 3,5 (dd large, 1H, 1H en 3) ; 3,7 (dd large, 1H, H en 3a) ; 3,9 (d large, 1H, 1H en 3) ; 4,2 (m, 1H, H en 7a) ; 7,1 à 8 (m, 8H aromatiques).

La benzyl-2 bis(fluoro-2 phényl)-7,7 perhydroisoindolone-4-(3aRS,7aRS) peut être obtenue de la manière suivante :
A une solution de 4,3 g de bis(fluoro-2 phényl)-4,4 cyclohexènone et de 5,8 $cm^3$ de N-butoxyméthyl N-triméthylsilylméthyl benzylamine dans 30 $cm^3$ de dichlorométhane sec, on ajoute 3 gouttes d'acide trifluoroacétique. Le mélange réactionnel est porté au reflux puis agité 16 heures après avoir laissé revenir la température à 25°C. On ajoute 2,5 $cm^3$ de N-butoxyméthyl N-triméthylsilylméthyl benzylamine et 3 gouttes d'acide trifluoroacétique et agite 3 heures au reflux. Le mélange réactionnel est traité par 3 g de carbonate de potassium et agité 15 minutes. Après filtration et concentration à sec sous pression réduite (2,7 kPa), le résidu est chromatographié sur une colonne de gel de silice (granulomètrie 0,04-0,063 mm, diamètre 4 cm, hauteur 32 cm) en éluant sous une pression de 0,5 bar d'azote par un mélange de cyclohexane et d'acétate d'éthyle (85/15) et en recueillant des fractions de 20 $cm^3$. Les fractions 13 à 22 sont réunies et concentrées à sec sous pression réduite (2,7 kPa) pour donner 2,28 g de benzyl-2 bis(fluoro-2 phényl)-7,7 perhydroisoindolone-4-(3aRS,7aRS). P.F. = 138°C.

La bis(fluoro-2 phényl)-4,4 cyclohexènone peut être obtenue de la manière suivante :
A une solution de 30,8 g de bis(fluoro-2 phényl)acétaldéhyde dans 135 $cm^3$ de diméthoxy-1,2 éthane, on ajoute 26,9 g de carbonate de potassium puis goutte à goutte et après refroidissement à -50°C, 19,9 $cm^3$ de butènone. Le mélange réactionnel est agité 12 h à -50°C puis 6 heures à 25°C, dilué par 250 $cm^3$ d'acétate d'éthyle et 200 $cm^3$ d'eau. La phase organique est lavée 3 fois par 200 $cm^3$ d'eau puis par 200 $cm^3$ de solution saturée de chlorure de sodium, séchées sur sulfate de magnésium et concentrées sous pression réduite (2,7 kPa). Le résidu est chromatographié sur colonne de gel de silice (granulomètrie 0,04-0,063 mm, diamètre 5,5 cm, hauteur 50 cm) en éluant sous une pression de 0,5 bar d'azote par un mélange de cyclohexane et d'acétate d'éthyle (90/10). On obtient 9 g de bis(fluoro-2 phényl)-2,2 oxo-5 hexanal sous forme d'une huile jaune. Une solution de 6,65 g de ce composé dans 100 $cm^3$ de toluène contenant 1,5 g d'acide paratoluènesulfonique est chauffée au reflux pendant 3 heures lavée 2 fois par 100 $cm^3$ d'eau puis par 100 $cm^3$ de solution saturée de chlorure de sodium, séchée sur sulfate de magnésium et concentrée sous pression réduite (2,7 kPa). Le résidu est chromatographié sur une colonne de gel de silice (granulomètrie 0,04-0,063 mm, diamètre 4 cm, hauteur 30 cm) en éluant sous une pression de 0,5 bar d'azote par un mélange de cyclohexane et d'acétate d'éthyle (90/10) et en recueillant des fractions de 15 $cm^3$. Les fractions 21 à 26 sont réunies et concentrées à sec sous pression réduite (2,7 kPa) pour donner 2,83 g de bis(fluoro-2 phényl)-4,4 cyclohexènone, sous forme d'huile jaune.
Spectre RMN du proton (DMSO-$d_6$):
2,6 (m, 2H, -$CH_2$- en 5) ; 2,8 (dd large, 2H, -$CH_2$- en 6) ; 6,2 (d, 1H, H en 2) ; 6,9 à 7,4 (m, 9H aromatiques et H en 3).

Le bis(fluoro-2 phényl)acétaldéhyde peut être préparé de la manière suivante :
Une solution de 26,3 g de bis(fluoro-2 phényl)-1,2 oxirane dans 500 $cm^3$ de toluène est traitée goutte à goutte par 7 $cm^3$ d'éthèrate de trifluorure de bore et agitée 2 heures à 25°C puis lavée par 50 $cm^3$ d'eau et 50 $cm^3$ de solution saturée de bicarbonate de sodium. Après séchage sur sulfate de magnésium et concentration à sec sous pression réduite (2,7 kPa) on obtient 25 g de bis(fluoro-2 phényl)acétaldéhyde sous la forme d'une huile jaune.

Le bis(fluoro-2 phényl)-1,2 oxirane peut être préparé selon la méthode décrite par V. Mark (J. Am. Chem. Soc., 85, 1884 (1963))

EXEMPLE 95

A une solution de 1,06 g de chlorhydrate de bis(chloro-3 phényl)-7,7 perhydroisoindolone-4 dans 20 $cm^3$ de dichlorométhane refroidie à +4°C on ajoute 0,45 $cm^3$ de triéthylamine puis 0,49 g de chlorure de phénylacétyle. Le mélange réactionnel est agité 2 heures à 25°C, puis lavé 3 fois par 30 $cm^3$ d'eau et 3 par par 30 $cm^3$ de solution saturée de chlorure de sodium. La phase organique est séchée sur sulfate de magnésium, filtrée et concentrée à sec sous pression réduite (2,7 kPa). Le résidu est chromatographié sur une colonne de gel de silice (granulomètrie 0,04-0,063 mm, diamètre 2,2 cm, hauteur 23 cm) en éluant sous une pression de 0,5 bar d'azote par un mélange de cyclohexane et d'acétate d'éthyle (55/45 en volumes) et en recueillant des fractions de 15 $cm^3$. Les fractions 7 à 18 sont concentrées à sec sous pression réduite (2,7 kPa) et le résidu est cristallisé dans de l'acétonitrile. Les cristaux sont essorés, lavés par de l'oxyde d'isopropyle et séchés. On obtient 0,21 g de bis(chloro-3 phényl)-7,7 (phénylacétyl)-2 perhydroisoindolone-4-(3aRS,7aRS). P.F. = 160°C.

Le chlorhydrate de bis(chloro-3 phényl)-7,7 perhydroisoindolone-4 peut être préparé de la manière suivante :
Une solution de 11,5 g de benzyl-2 bis(chloro-3 phényl)-7,7 perhydroisoindolone-4-(3aRS,7aRS) dans 250 $cm^3$ de dichloro-1,2 éthane est traitée par 2,8 $cm^3$ de chloroformiate de vinyle et chauffée 16 heures au reflux puis concentrée sous pression réduite (2,7 kPa). Le résidu est chromatographié sur une colonne de gel de silice (granulomètrie 0,04-0,063 mm, diamètre 6 cm, hauteur 32 cm) en éluant sous une pression de 0,5 bar d'azote par un mélange de cyclohexane et d'acétate d'éthyle (80/20) et en recueillant des fractions de 25 $cm^3$. Les fractions 19 à 27 sont réunies et concentrées à sec sous pression réduite (2,7 kPa). La meringue obtenue est concrètée dans de l'oxyde d'isopropyle et le précipité est essoré, lavé par de l'oxyde d'isopropyle et sèché pour donner 6,7 g de bis(chloro-3 phényl)-7,7 vinyloxycarbonyl-2 perhydroisoindolone-4-(3aRS,7aRS) sous la forme d'un solide blanc.
Spectre RMN du proton (DMSO-$d_6$):
2,1 et 2,3 (2 ddd larges, 2H, -$CH_2$- en 5) ; 2,7 à 3 (m, 4H, -$CH_2$- en 1 et en 6) ; 3,3 (m, 1H , H en 3a) ; 3,45 (dd large, 1H, 1H en 3) ; 4,1 (m, 2H, H en 7a et 1H en 3)
4,45 et 4,70 (2 d larges, 2H, =$CH_2$ du vinyle) ; 7,05 ((dd, 1H, OCH= du vinyle)) ;7,2 à 7,7 (m, 8H aromatiques).
1,5 g de bis(chloro-3 phényl)-7,7 vinyloxycarbonyl-2 perhydroisoindolone-4-(3aRS,7aRS) sont traités par 7,4 $cm^3$ d'une solution 6N d'acide chlorhydrique dans le dioxanne pendant 2 heures à 25°C. La solution est concentrée à sec sous pression réduite (2,7 kPa) et le résidu est chauffé 1 heure en solution dans de l'éthanol à 60°C puis agitée pendant 6 heures à 25°C. La solution est concentrée à sec sous pression réduite (2,7 kPa) et la meringue obtenue concrètée dans de l'oxyde d'isopropyle. Le précipité est essoré et lavé par de l'oxyde d'isopropyle puis sèché pour donner 1 g de chlorhydrate de bis(chloro-3 phényl)-7,7 perhydroisoindolone-4.
Spectre RMN du proton (DMSO-$d_6$):
2 à 2,4 (m, 2H, -$CH_2$- en 5) ; 2,55 à 2,9 (m, 2H, -$CH_2$- en 6) ; 3,3 (dd large, 1H, 1H en 3) ; 3,5 (m, 1H, H en 3a) ; 3,85 (d large, 1H, 1H en 3) ; 3,95 (m, 1H, H en 7a) ; 7,1à 7,76 (m, 8H aromatiques).

La benzyl-2 bis(chloro-3 phényl)-7,7 perhydroisoindolone-4-(3aRS,7aRS) peut être obtenu de la manière

suivante :
A une solution de 26,8 g de bis(chloro-3 phényl)-4,4 cyclohexènone et de 33 $cm^3$ de N-butoxyméthyl N-triméthylsilylméthyl benzylamine dans 200 $cm^3$ de dichlorométhane sec, on ajoute 15 gouttes d'acide trifluoroacétique. Le mélange réactionnel est porté au reflux puis agité 16 heures après avoir laissé revenir la température à 25°C et encore 15 minutes après addition de 16 g de carbonate de potassium. Après filtration et concentration à sec sous pression réduite (2,7 kPa), le résidu est chromatographié sur une colonne de gel de silice (granulomètrie 0,04-0,063 mm, diamètre 7 cm, hauteur 40 cm) en éluant sous une pression de 0,5 bar d'azote par un mélange de cyclohexane et d'acétate d'éthyle (75/25 en volumes) et en recueillant des fractions de 500 $cm^3$. Les fractions 12 à 18 sont réunies et concentrées à sec sous pression réduite (2,7 kPa) pour donner 16,2 g de benzyl-2 bis(chloro-3 phényl)-7,7 perhydroisoindolone-4-(3aRS,7aRS) sous la forme d'une huile jaune.
Spectre RMN du proton: (DMSO-$d_6$):
1,75 (ddd, 1H) et 2,1 à 2,45 (m, 3H) : -$CH_2$- en 5 et en 6) ; 2,7 à 2,9 (m, 4H : -$CH_2$- en 1 et en 3) ; 3,1 (m, 1H , H en 3a) ; 3,5 (AB, 2H, -$CH_2$- benzylique) ; 3,8 (dd large, 1H, H en 7a) ; 7,1 à 7,5 (m, 13H aromatiques).

La bis(chloro-3 phényl)-4,4 cyclohexènone peut être obtenue de la manière suivante :
A une solution de 36,9 g de bis(chloro-3 phényl)acétaldéhyde dans 200 $cm^3$ d'éther éthylique, on ajoute 11,3 $cm^3$ de butènone, puis après refroidissement à 0°C, goutte à goutte une solution de 3,1 g de potasse dans 20 $cm^3$ d'éthanol. Le mélange réactionnel est agité 2 h à 0°C puis 16 heures à 25 °C, dilué par 100 $cm^3$ d'acétate d'éthyle et 200 $cm^3$ d'eau. La phase aqueuse est lavée par 100 $cm^3$ d'acétate d'éthyle. Les phases organiques réunies sont lavées 3 fois par 100 $cm^3$ de solution saturée de chlorure de sodium, séchées sur sulfate de magnésium et concentrées sous pression réduite (2,7 kPa). Le résidu est chromatographié sur colonne de gel de silice (granulomètrie 0,04-0,063 mm, diamètre 7 cm, hauteur 42 cm) en éluant sous une pression de 0,5 bar d'azote par un mélange de cyclohexane et d'acétate d'éthyle (90/10 en volumes). On obtient 27,7 g de bis(chloro-3 phényl)-4,4 cyclohexènone, sous forme d'huile jaune.
Spectre RMN du proton (DMSO-$d_6$):
2,3 (dd large, 2H, -$CH_2$- en 5) ; 2,7 (dd large, 2H, -$CH_2$- en 6) ; 6,2 (d, 1H, H en 2) ; 7,2 à 7,4 (m, 8H aromatiques) ; 7,6 (d, 1H, H en 3).

Le bis(chloro-3 phényl)acétaldéhyde peut être obtenu de la manière suivante :
Une solution de 47 g de bis(chloro-3 phényl)-1,1 méthoxy-2 éthanol (obtenu par réaction de bromure de (chloro-3 phényl)magnésium sur du méthoxy-2 acétate de méthyle dans le tétrahydrofuranne) dans 44 $cm^3$ d'acide formique est chauffée au reflux 5 heures, refroidie et versée dans un mélange de 500 $cm^3$ de solution saturée de carbonate de sodium et de 300 $cm^3$ d'acétate d'éthyle. La phase organique est lavée 3 fois par 250 $cm^3$ d'eau et par 200 $cm^3$ de solution saturée de chlorure de sodium puis séchée et concentrée à sec sous pression réduite (2,7 kPa) pour donner 36,9 g de bis(chloro-3 phényl)acétaldéhyde sous la forme d'une huile jaune.

## EXEMPLE 96

A une solution de 2,45 g de chlorhydrate de bis(chloro-3 phényl)-7,7 perhydroisoindolone-4 dans 50 $cm^3$ de dichlorométhane sec refroidie à +4°C on ajoute 1,1 g d'acide (diméthylamino-2 phényl)acétique, 0,09 g d'hydroxy-1 benzotriazole et 1,06 $cm^3$ de diisopropyléthylamine puis goutte à goutte une solution de 1,3 g de (diméthylamino-3 propyl)-1 éthyl-3 carbodiimide dans 100 $cm^3$ de dichlorométhane sec. Le mélange réactionnel est agité 5 heures à 0°C puis 16 heures à 25°C et lavé 2 fois par 250 $cm^3$ d'eau et par 250 $cm^3$ de solution saturée de chlorure de sodium. La phase organique est séchée sur sulfate de magnésium et concentrée à sec sous pression réduite (2,7 kPa). Le résidu est chromatographié sur une colonne de gel de silice (granulomètrie 0,04-0,063 mm, diamètre 3 cm, hauteur 22 cm) en éluant sous une pression de 0,5 bar d'azote par un mélange de cyclohexane et d'acétate d'éthyle (55/45 en volumes) et en recueillant des fractions de 20 $cm^3$. Les fractions 13 à 24 sont réunies et concentrées à sec sous pression réduite (2,7 kPa) pour donner 2,3 g de bis(chloro-3 phényl)-7,7 (diméthylamino-2 phényl)acétyl-2 perhydroisoindolone-4-(3aRS,7aRS) dont on prépare le chlorhydrate par dissolution dans 2 $cm^3$ d'acétate d'éthyle et addition d'une solution 3N d'acide chlorhydrique dans l'oxyde d'isopropyle. Le précipité est essoré, lavé par de l'oxyde d'isopropyle et séché. On obtient 1,94 g de chlorhydrate de bis(chloro-3 phényl)-7,7 (diméthylamino-2 phényl)acétyl-2 perhydroisoindolone-4-(3aRS,7aRS) sous la forme d'un solide blanc.
Spectre RMN du proton (DMSO-$d_6$/AcOD 90/10) ;
à température ambiante on observe le mélange des deux rotamères:
2,1 et 2,3 (m, 2H, -$CH_2$- en 5) ; 2,5 à 3,5 (m, 11H, -$CH_2$- en 1 et en 6, $N(CH_3)_2$ et H en 3a) ; 3,7 (m, 1H, 1H en 3) ; 4 (m, 3H, H en 7a et -$CH_2CO$) ; 4,4 et 4,2 (2 d, 1H, 1H en 3) ; 7,1 à 7,8 (m,12H aromatiques).

EXEMPLE 97

Une suspension de 1,5 g de chlorhydrate de bis(tolyl-3)-7,7 perhydroisoindolone-4-(3aRS,7aRS) dans 30 $cm^3$ de dichlorométhane refroidie à +4°C est traitée par 1,15 $cm^3$ de triéthylamine puis par 0,63 g de chlorure de phénylacétyle. Le mélange réactionnel est agité 5 heures à 25°C, puis lavé 3 fois par 100 $cm^3$ d'eau. La phase organique est séchée sur sulfate de magnésium, filtrée et concentrée à sec sous pression réduite (2,7 kPa). Le résidu est cristallisé 2 fois dans l'acétonitrile pour donner 0,36 g de bis(tolyl-3)-7,7 (phénylacétyl)-2 perhydroisoindolone-4-(3aRS,7aRS). P.F. = 207°C.

Le chlorhydrate de bis(tolyl-3)-7,7 perhydroisoindolone-4 peut être obtenu de la manière suivante :
Une solution de 13,7 g de benzyl-2 bis(tolyl-3)-7,7 perhydroisoindolone-4-(3aRS,7aRS) dans 150 $cm^3$ de dichloro-1,2 éthane est traitée par 3,7 $cm^3$ de chloroformiate de vinyle et chauffée 3 heures au reflux puis concentrée sous pression réduite (2,7 kPa). Le résidu est chromatographié sur gel de silice (granulomètrie 0,04-0,063 mm, colonne de diamètre 5,4 cm et hauteur 39 cm) en éluant sous une pression de 0,5 bar d'azote par un mélange de cyclohexane et d'acétate d'éthyle (80/20). Les fractions 23 à 39 sont réunies et concentré à sec sous pression réduite (2,7 kPa) pour donner 7,4 g de bis(tolyl-3)-7,7 vinyloxycarbonyl-2 perhydroisoindolone-4-(3aRS,7aRS). sous la forme d'une meringue blanche.
Spectre de RMN du proton(DMSO-$d_6$/AcOD 90/10):
A température ordinaire on observe le mélange des 2 rotamères.
1,95 à 2,4 (m, 2H, -$CH_2$- en 5) ; 2,27 et 2,32 (2s , 6H, $ArCH_3$) ; 2,4 à 2,95 (m, 4H, -$CH_2$- en 1 et en 6) ; 3,2 à 3,5 (m, 2H, H en 3a et 1H en 3) ; 4,03 (m, 1H H en 7a) ; 4,09 et 4,16 (2d larges, 1H, H en 3) ; 4,35 à 4.85 (4d larges, 2H, =$CH_2$ du vinyle) ; 6,9 à 7,5 (m, 9H, aromatiques et OCH= du vinyle).

7,4 g de bis(tolyl-3)-7,7 vinyloxycarbonyl-2 perhydroisoindolone-4-(3aRS,7aRS) sont traités par 39 $cm^3$ d'une solution 6N d'acide chlorhydrique dans le dioxanne pendant 30 minutes à 25°C. La solution est concentrée à sec sous pression réduite (2,7 kPa) et le résidu repris par 100 $cm^3$ d'éthanol. La solution est chauffée à 60°C pendant 2 heures et agitée 16 heures à 25°C puis concentrée à sec sous pression réduite (2,7 kPa). Le résidu est concrèté parde l'oxyde d'isopropyle, le solide est lavé essoré et séché. On obtient 6,36 g de chlorhydrate de bis(tolyl-3)-7,7 perhydroisoindolone-4, sous la forme d'un solide jaune.
Spectre de RMN du proton (DMSO-$d_6$/AcOD 90/10):
1,95 à 2,35 (m, 2H, -$CH_2$- en 5) ; 2,24 et 2,3 (2s , 6H, $ArCH_3$) ; 2,4 à 2,9 (m, 4H, -$CH_2$- en 6 et en 1) ; 3,3 (dd large, 1H, 1H en 3) ; 3,48 (m, 1H, H en 3a) ; 3,85 (d large, 1H, 1H en 3) ; 3,90 (m, 1H, H en 7a) ; 6,9 à 7,4 (m, 8H aromatiques).

La benzyl-2 bis(tolyl-3)-7,7 perhydroisoindolone-4-(3aRS,7aRS) peut être obtenue de la manière suivante :
A une solution de 16,7 g de bis(tolyl-3)-4,4 cyclohexènone et de 18,7 $cm^3$ de N-butoxyméthyl N-triméthylsilylméthyl benzylamine dans 150 $cm^3$ de dichlorométhane sec, on ajoute 12 gouttes d'acide trifluoroacétique. Le mélange réactionnel est porté au reflux puis agité 3 heures en laissant revenir la température à 25°C et encore 10 minutes après addition de 12 g de carbonate de potassium. Après filtration et concentration à sec sous pression réduite (2,7 kPa), le résidu est chromatographié sur une colonne de gel de silice (granulométrie 0,04-0,063 mm, diamètre 5 cm, hauteur 50 cm) en éluant sous une pression de 0,7 bar d'azote par un mélange de cyclohexane et d'acétate d'éthyle (85/15 en volumes) et en recueillant des fractions de 25 $cm^3$. Les fractions 14 à 30 sont réunies et concentré à sec sous pression réduite (2,7 kPa) pour donner 13,9 g de benzyl-2 bis(tolyl-3)-7,7 perhydroisoindolone-4-(3aRS,7aRS) sous la forme de cristaux blancs. sous la forme d'une huile incolore.
Spectre RMN du proton ($CDCl_3$):
1,98 (ddd, 1H) et 2,2 à 2,5 (m, 3H) : -$CH_2$- en 5 et en 6) ; (s , 6H, $ArCH_3$) ; 2,5 à 3,05 (m, 4H, -$CH_2$- en 1 et en 3) ; 3,2 (m, 1H, H en 3a) ; 3,45 et 3,65 (AB, 2H, -$CH_2$- Ar) ; 3,7 (m, 1H, H en 7a) ; 6,9 à 7,4 (m, 13H aromatiques).

La bis(tolyl-3)-4,4 cyclohexènone peut être obtenue de la manière suivante :
A une solution de 20,4 g de bis(tolyl-3)acétaldéhyde dans 110 $cm^3$ d'éther éthylique, on ajoute 7,23 $cm^3$ de butènone, puis après refroidissement à 0°C, goutte à goutte une solution de 2 g de potasse dans 12,7 $cm^3$ d'éthanol. Le mélange réactionnel est agité 2 heures à 0°C puis 16 heures à 25 °C, dilué par 200 $cm^3$ d'acétate d'éthyle et 200 $cm^3$ d'eau. La phase aqueuse est lavée 2 fois par 250 $cm^3$ d'acétate d'éthyle. Les phases organiques réunies sont lavées 2 fois par 250 d'eau puis par 250 $cm^3$ de solution saturée de chlorure de sodium, séchées sur sulfate de magnésium et concentrées sous pression réduite (2,7 kPa). Le résidu est chromatographié sur gel de silice (granulomètrie 0,04-0,063 mm, colonne de diamètre 5,4 cm et hauteur 40 cm) en éluant sous une pression de 0,5 bar d'azote par un mélange de cyclohexane et d'acétate d'éthyle (85/15 en volumes). On obtient 16,7 g de bis(tolyl-3)-4,4 cyclohexènone, sous forme d'huile jaune.

Le bis(tolyl-3)acétaldéhyde peut être obtenu de la manière suivante : Une solution de 24,66 g de bis(tolyl-3)-1,1 méthoxy-2 éthanol (obtenu par réaction de bromure de (tolyl-3)magnésium sur du méthoxy-2 acétate

de méthyle dans le tétrahydrofuranne) dans 30 $cm^3$ d'acide formique est chaufée au reflux 12 heures, refroidie et versée dans un mélange de 400 $cm^3$ de solution saturée de carbonate de sodium et de 400 $cm^3$ d'acétate d'éthyle. La phase organique est lavée 3 fois par 300 $cm^3$ d'eau et par 300 $cm^3$ de solution saturée de chlorure de sodium puis séchée et concentrée à sec sous pression réduite (2,7 kPa) pour donner 20,45 g de bis(tolyl-3)acétaldéhyde sous la forme d'une huile jaune.

EXEMPLE 98

Une solution de 25 g de diphényl-4,4 cyclohexène-2 one-1 et de 1,65 g de tris triméthylsilylméthyl-1,3,5 triazine-1,3,5 dans 17 $cm^3$ de trichloro-1,1,2 éthane est traitée par une solution de 1,8 g de fluorure de phénylacétyle dans 5 $cm^3$ de trichloro-1,1,2 éthane puis chauffée à 120°C pendant 22 heures. Le mélange réactionnel est dilué par 100 $cm^3$ de dichlorométhane, lavé par 100 $cm^3$ de solution saturée de bicarbonate de sodium et par 100 $cm^3$ de solution saturée de chlorure de sodium, séché sur sulfate de magnésium, puis concentré à sec sous pression réduite (2,7 kPa). Le résidu est chromatographié sur une colonne de gel de silice (0,2-0,063 mm, diamètre 3 cm, hauteur 27 cm) en éluant par un mélange de cyclohexane et d'acétate d'éthyle (90/10 en volumes) et en recueillant des fractions de 35 $cm^3$. Les fractions 33 à 36 sont réunies et concentrées à sec sous pression réduite (2,7 kPa) pour donner 0,25 g de diphényl-7,7 (phénylacétyl)-2 perhydroisoindolone-4-(3aRS,7aRS) sous la forme de cristaux blancs, fondant à 212°C.

La tris triméthylsilylméthyl-1,3,5 triazine-1,3,5 peut être préparée selon la méthode de T.Morimoto, Y. Nezu et K. Achiwa: Chem. Pharm. Bull. 33, 4596 (1985).

Le fluorure de phénylacétyle peut être obtenu par la méthode de G. Olah, S. Kuhn, et S. Beke: Chem. Ber. 89, 862, (1956).

EXEMPLE 99

A une solution de 1,3 g d'[(hydroxy-2 phényl) acétyl]-2 diphényl-7,7 perhydroisoindolone-4-(3aRS,7aRS) dans 26 $cm^3$ de butanone et 1,3 $cm^3$ de diméthylformamide, on ajoute 0,34 $cm^3$ d'iodure d'isopropyle et 0,89 g de carbonate de potassium. On chauffe six heures au reflux puis refroidit à température ambiante. Le solide est éliminé par filtration et lavé par 2 fois 10 $cm^3$ de butanone. Les fractions organiques sont rassemblées, séchées sur sulfate de magnésium, et concentrées sous pression réduite. Le résidu est chromatographié sur une colonne de 150 g de gel de silice en éluant par un mélange de cyclohexane et d'acétate d'éthyle (80/20 en volumes). Le résidu est repris par 5 $cm^3$ de chlorure de méthylène et 5 $cm^3$ d'eau. La phase organique est décantée, lavée par deux fois 5 $cm^3$, séchée sur sulfate de sodium et évaporée sous pression réduite. Le résidu est cristallisé dans 50 $cm^3$ d'éther de pétrole (Eb. 40°C-60°C). Les cristaux sont essorés, lavés par de l'éther de pétrole et séchés.

On obtient 0,41 g d'[(isopropoxy-2 phényl) acétyl]-2 diphényl-7,7 perhydroisoindolone-4-(3aRS,7aRS) sous la forme de cristaux blancs, fondant à 177°C.

EXEMPLE 100

A une suspension de 0,26 g d'hydrure de sodium (dispersion à 50 % dans l'huile) dans 3 $cm^3$ de toluène on ajoute 2,1 g d'[(hydroxy-2 phényl) acétyl]-2 diphényl-7,7 perhydroisoindolone-4-(3aRS,7aRS) et agite 30 minutes à 50°C. La solution refroidie à 25°C est traitée par une solution de N,N diméthyl chloro-2 éthylamine dans 4 $cm^3$ de toluène sec (obtenue par libération de 2,16 g de chlorhydrate correspondant par action de lessive de potasse). Le mélange réactionnel est chauffé 21 heures au reflux puis traité par 0,35 $cm^3$ d'acide acétique, dilué par 20 $cm^3$ d'eau et 20 $cm^3$ d'acétate d'éthyle. La phase organique est extraite 2 fois par 30 $cm^3$ d'acide chlorhydrique 0,2 N. La phase aqueuse acide est lavée par de l'acétate d'éthyle, alcalinisée par addition de 25 $cm^3$ de soude N et réextraite par de l'acétate d'éthyle. La phase organique est lavée à l'eau à neutralité, séchée sur sulfate de magnésium et concentrée à sec sous pression réduite (2,7 kPa). Après deux chromatographies sur une colonne de 100 g de gel de silice (0,2-0,045 mm) en éluant par un mélange de toluène et de diéthylamine (90/10 en volumes) on isole 1,3 g du produit attendu qui est trituré avec de l'éther de pétrole et séché. On obtient 0,47 g de [((diméthylamino-2 éthoxy)-2 phényl) acétyl]-2 diphényl-7,7 perhydroisoindolone-4-(3aRS,7aRS), fondant à 90°C.

EXEMPLE 101

A une solution de 2,1 g d'[(hydroxy-2 phényl) acétyl]-2 diphényl-7,7 perhydroisoindolone-4-(3aRS,7aRS) dans 20 $cm^3$ de toluène on ajoute 0,18 g d'hydrure de sodium (dispersion à 80 % dans l'huile) et agite 30 minutes à 20°C. La suspension est traitée par une solution de N,N diméthyl chloro-3 propylamine dans 20 $cm^3$ de toluène sec (obtenue par libération de 2,3 g de chlorhydrate correspondant par action de lessive de potasse). Le mélange réactionnel est chauffé 16 heures au reflux puis traité par 0,5 $cm^3$ d'acide acétique, dilué par 20 $cm^3$ d'eau et 50 $cm^3$ d'acétate d'éthyle. La phase organique est extraite 2 fois par 25 $cm^3$ d'acide chlorhydrique 0,2 N. La phase aqueuse acide est lavée par de l'acétate d'éthyle, filtrée et concentrée à sec sous pression réduite (2,7 kPa). Le résidu est dissous dans 20 $cm^3$ d'acétate d'éthyle et traité par 1,2 $cm^3$ de solution 3M d'acide chlorhydrique dans le dioxanne sec. Le produit attendu précipite sous la forme de chlorhydrate impur. Ce solide jaune est redissous dans de l'eau, la solution est lavée par de l'acétate d'éthyle, alcalinisée à pH 10 par action de soude 0,1 N et extraite par de l'acétate d'éthyle. La phase aqueuse est extraite par de l'acétate d'éthyle. Cette phase organique est séchée sur sulfate de magnésium et concentré à sec sous pression réduite (2,7 kPa). Le produit, obtenu sous forme de base, est converti en chlorhydrate par dissolution dans 10 $cm^3$ d'acétate d'éthyle et traitement par 0,5 $cm^3$ de solution 3M d'acide chlorhydrique dans le dioxanne sec. On obtient 0,45 g de chlorhydrate de [[(diméthylamino-3 propoxy)-2 phényl] acétyl]-2 diphényl-7,7 perhydroisoindolone-4-(3aRS,7aRS) sous la forme d'un solide jaune.
Spectre RMN du proton :
2,05 à 2,35 (Mt, 4H, $-CH_2-$ en 5 ou 6 et $-CH_2-$ central du propoxy diméthylamino-3) ; 2,65 à 3 (Mt, $-CH_2-$ en 6 ou 5 et $-CH_2-$ en 1) ; 2,83 (s, - $N(CH_3)_2$) ; 3,2 à 3,4 (Mt, 3H, -CH- en 3a et $-N-CH_2-$) ; 3,4, 3,65 (Mt, 3H, -N-CO-$CH_2$- et 1H du $-CH_2-$ en 3) ; 3,9 à 4,5 (Mt, 3H, -CH- en 7a et $-O-CH_2-$) ; 4,28 (D, J = 11Hz, 1H, 1H du $-CH_2-$ en 3) ; 6,8 à 7,65 (Mt, 14H, aromatiques).
Spectre infra-rouge (KBr) bandes caractéristiques ($cm^{-1}$):
3600-3250, 3100-3000, 3000-2850, 2750, 2250, 1715, 1640, 1600, 1495, 1475, 1455, 1445, 1440, 1245, 1050, 755, 705.

EXEMPLE 102

A une suspension de 0,32 g d'hydrure de sodium (dispersion à 50 % dans l'huile) dans 10 $cm^3$ de toluène on ajoute 2,55 g d'[(hydroxy-2 phényl)acétyl]-2 diphényl-7,7 perhydroisoindolone-4-(3aRS,7aRS) puis à 25°C, une solution de (méthyl-4 pipérazinyl-1)-1 chloro-2 éthane dans 10 $cm^3$ de toluène sec (obtenue par libération de 1,14 g de dichlorhydrate correspondant par action de lessive de potasse). Le mélange réactionnel est chauffé 18 heures au reflux puis traité par 20 $cm^3$ d'eau et 30 $cm^3$ d'acétate d'éthyle. La phase aqueuse est extraite par 10 $cm^3$ d'acétate d'éthyle et les phases organiques réunies sont lavées à l'eau à neutralité, séchées sur sulfate de sodium et concentrées à sec sous pression réduite (2,7 kPa). Le résidu est lavé par de l'éther de pétrole puis dissous dans un mélange d'acétate d'éthyle et d'acide méthanesulfonique dilué (pH 2). La phase organique est lavée à l'eau, les phases aqueuses acides réunies sont lavées par de l'acétate d'éthyle, neutralisées par action de soude normale. On extrait par de l'acétate d'éthyle puis lave la phase organique obtenue par de l'eau, sèche sur sulfate de sodium et concentre à sec sous pression réduite (2,7 kPa). Après chromatographie sur une colonne de 50 g de gel de silice (0,2-0,045 mm) en éluant par un mélange de toluène et de diéthylamine (90/10 en volumes) on isole 0,6 g du produit attendu qui est trituré dans 15 $cm^3$ d'éther de pétrole et séché. On obtient 0,36 g de {[(méthyl-4 pipérazinyl-1)-2 éthoxy]-2 phénylacétyl}-2 diphényl-7,7 perhydroisoindolone-4-(3aRS,7aRS), fondant à 78°C.

Le dichlorhydrate de (méthyl-4 pipérazinyl-1)-1 chloro-2 éthane est préparé selon G. Emptoz et coll., Chim. Thér., 4 (4), 283 (1969).

EXEMPLE 103

En opérant comme à l'exemple 102, à partir de diphényl-7,7 (hydroxy-2 phénylacétyl)-2 perhydroisoindolone-4-(3aRS,7aRS) et de benzyloxy-1 chloro-2 éthane, on obtient O,5 g de diphényl-7,7 {[(hydroxy-2 éthoxy)-2 phényl] acétyl}-2 perhydroisoindolone-4-(3aRS,7aRS) sous forme de cristaux blancs, fondant à 150°C.

EXEMPLE 104

A une suspension agitée de 1,6 g de chlorhydrate de diphényl-7,7 - perhydroisoindolone-4-(3aRS,7aRS)dans 40 $cm^3$ de dichloro-1,2 éthane, on ajoute 1,2 g de bromhydrate de phénylacétimidate d'éthyle et 1,4 $cm^3$ de triéthylamine. Le mélange réactionnel est laissé sous agitation 20 heures à température ambiante, puis 6 heures au reflux. Après retour à température ambiante le mélange réctionnel est traité par 50 $cm^3$ d'une solution aqueuse saturée en carbonate de potassium ; la phase organique est séchée sur sulfate de magnésium, filtrée et concentrée à sec sous pression réduite (2,7 kPa). Le résidu est cristallisé dans 20 $cm^3$ d'acétonitrile. Les cristaux obtenus sont essorés et séchés. On obtient 0,9 g d'($\alpha$-iminophénéthyl)-2 diphényl-7,7 perhydroisoindolone-4-(3aRS,7aRS), fondant à 195°C.

Le bromhydrate de phénylacétimidate d'éthyle peut être préparé selon une méthode décrite par D.J. Morgan, Chem. and Ind., 854 (1959)

EXEMPLE 105

A une suspension agitée de 6,6 g de (méthoxy-2 phényl)-2 acétamide dans 20 $cm^3$ de dichlorométhane anhydre, on ajoute 8,4 g de tétrafluoroborate de triéthyloxonium. Le mélange réactionnel est laissé 20 heures, sous agitation, à température ambiante. Il est refroidit à +5°C, puis on ajoute une solution de 9,8 g de chlorhydrate de diphényl-7,7 perhydroisoindolone-4-(3aR,7aR) et de 10,4 $cm^3$ de triéthylamine dans 60 $cm^3$ de dichlorométhane. Après retour à température ambiante, le mélange réactionnel est chauffé 4 heures au reflux. Il est ensuite traité, après retour à température ambiante, par 40 $cm^3$ d'une solution aqueuse de carbonate de potassium à 10% ; la phase organique est lavée par 20 $cm^3$ d'eau distillée, séchée sur sulfate de magnésium et concentrée à sec sous pression réduite (2,7 kPa). Le résidu est cristallisé dans 20 $cm^3$ d'acétonitrile ; les cristaux obtenus sont essorés, lavés par 5 $cm^3$ d'acétonitrile, par 10 $cm^3$ d'oxyde d'isopropyle et séchés. Les cristaux sont recristallisés dans 45 $cm^3$ d'acétonitrile; les cristaux obtenus sont essorés et séchés. On obtient 3,8 g d'[imino-1 (méthoxy-2 phényl)-2 éthyl]-2 diphényl-7,7 perhydroisoindolone-4-(3aR,7aR), fondant à 191°C.

$[\alpha]_D^{20} = -255°$ (c=1 , méthanol).

Le (méthoxy-2 phényl)-2 acétamide peut être préparé selon la méthode décrite par U.S. Seth et S.S. Deshapande, J. Indian Chem. Soc., 27, 429 (1950).

EXEMPLE 106

Une suspension de 0,9 g de (méthoxy-2 phényl)acétamide dans 3 $cm^3$ de dichlorométhane sec est traitée par 1,14 g de tétrafluoroborate de triéthyloxonium et la solution obtenue agitée pendant 20 heures à 25°C. Après refroidissement à 0°C on ajoute au milieu réactionnel une solution de 1,5 g de chlorhydrate de bis(fluoro-3 phényl)-7,7 perhydroisoindolone-4 et 1,4 $cm^3$ de triéthylamine dans 9 $cm^3$ de dichlorométhane. Le mélange réactionnel est agité 30 minutes à 25°C, puis chauffé au reflux pendant 5 heures et enfin agité encore 16 heures à 25°C. On ajoute 50 $cm^3$ de solution saturée de carbonate de potassium, agite, filtre et lave la phase organique 2 fois par 50 $cm^3$ d'eau. Après séchage sur sulfate de magnésium, filtration et concentration à sec sous pression réduite (2,7 kPa), le résidu est chromatographié sur une colonne d'alumine (diamètre 2,6 cm, hauteur 24 cm) en éluant sous une pression de 0,5 bar d'azote par un mélange de dichloro-1,2 éthane et de méthanol (95/5 en volumes) et en recueillant des fractions de 15 $cm^3$. Les fractions 7 à 25 sont réunies et concentrées à sec sous pression réduite (2,7 kPa) pour donner 0,54 g de bis(fluoro-3 phényl)-7,7 [imino-1 (méthoxy-2 phényl)-2 éthyl]-2 perhydroisoindolone-4-(3aRS,7aRS) sous la forme d'une meringue jaune pâle.

Spectre RMN du proton ($CDCl_3$):

2,20 et 2,45 (2m, 2H, $-CH_2-$ en 5) ; 2,8 (m, 2H, $-CH_2-$ en 6) ; 3,08 (m, 2H, $-CH_2-$ en 1) ; 3,23 (m, 1H, H en 3a) ; 3,53 (dd,J=11 et 6,5, 1H, 1H du $-CH_2-$ en 3) ; 3,6 (s, 2H, $-CH_2-Ar$) ; 3,8 (m, 1H, H en 7a) ; 3,8 (s, 3H, OCH3) ; 4,43 (d, J=11, 1H, 1H du $-CH_2-$ en 3) ; 6,8 à 7,5 (m, 14H aromatiques).

Spectre infrarouge (bandes caractéristiques): 3425, 3100-3000, 3000-2850, 2835, 1715, 1592, 1610, 1595, 1460, 1250, 1030, 780, 755, 695.

EXEMPLE 107

A une suspension agitée de 5,4 g de (méthoxy-2 phényl)-2 propionamide-(RS) dans 60 $cm^3$ de dichlorométhane anhydre, on ajoute 6,34 g de tétrafluoroborate de triéthyloxonium. Le mélange réactionnel est laissé 20 heures sous agitation. On ajoute une solution de 6,65 g de chlorhydrate de diphényl-7,7 perhydroisoindolone-4-(3aR,7aR) et de 2,8 $cm^3$ de triéthylamine dans 30 $cm^3$ de dichlorométhane. Le mélange réactionnel est chauffé 5 heures au reflux. Il est ensuite refroidi à +5°C puis est traité par 20 $cm^3$ d'une solution aqueuse de carbonate de potassium à 10%. Après filtration, la phase organique est lavée par 20 $cm^3$ d'eau distillée, séchée sur sulfate de magnésium et concentrée à sec sous pression réduite (2,7 kPa). Le résidu est chromatographié sur une colonne d'alumine neutre (0,05 mm-0,160 mm, diamètre 5 cm, hauteur 20 cm), en éluant par un mélange de cyclohexane et d'acétate d'éthyle (50/50) et en recueillant des fractions de 250 $cm^3$. La première fraction est concentrée à sec sous pression réduite (2,7 kPa). Le résidu est chromatographié sur une colonne de gel de silice (0,04 mm-0,06 mm, diamètre 2 cm, hauteur 35 cm), en éluant par un mélange de n-butanol-acide acétique-pyridine-eau (90/4/4/2 en volumes) et en recueillant des fractions de 20 $cm^3$. Les fractions 14 à 19 sont concentrées à sec sous pression réduite (0,13 kPa). Le résidu est repris par 40 $cm^3$ de dichlorométhane

et lavé par 10 $cm^3$ de solution aqueuse de carbonate de potassium. La phase organique est séchée sur sulfate de magnésium et concentrée à sec sous pression réduite (2,7 kPa). Le résidu est cristallisé dans l'oxyde de diisopropyle. Les cristaux sont essorés, séchés sous pression réduite (0,13 kPa).
On obtient 0,12 g d'[imino-1 (méthoxy-2 phényl)-2 méthyl-2 éthyl]-2 diphényl-7,7 perhydroisoindolone-4-(3aR,7aR),forme A, sous la forme d'une meringue blanche.
Spectre RMN du proton ($CDCl_3$, 323°K):
1,34(D, J = 7, 3H); 2,05 à 2,45 (m, 2H, 2H en 5); 2,7 à 3 (m, 4H, 2H en 6 et 2H en 1); 3,2 (m,1H, 1H en 3a); 3,58 (DD, J = 11 et 7, 1H du $-CH_2-$ en 3); 3,7 (S, 3H, $-OCH_3$); 3,75 (m, 1H, H en 7a); 4,04 ((Q, J = 7, 1H, Ar-<u>CH</u>-$CH_3$); 4,37 (D, J = 11, 1H, 1H du $-CH_2-$ en 3); 6 6,7 à 7,6 (m, 14H, aromatiques).
Spectre infra-rouge (KBr), bandes caractéristiques ($cm^{-1}$):
3400, 3100-3000, 3000-2850, 2835, 1715, 1595, 1495, 1445, 1245, 1030, 750, 700.

## EXEMPLE 108

En opérant comme décrit précédemment à l'exemple 107, pour la préparation de la forme A, on obtient, à partir des fractions chromatographiques 21 à 30 réunies, 0,1 g d'[imino-1 (méthoxy-2 phényl)-2 méthyl-2 éthyl]-2 diphényl-7,7 perhydroisoindolone-4-(3aR,7aR) forme B, sous la forme d'une meringue blanche.
Spectre RMN du proton ($CDCl_3$, 333°K)
2,20(m, 1H, 1H du $-CH_2-$ en 5); 2,45(m, 1H, 1H du $-CH_2-$ en 5); 2,8(m, 2H, $-CH_2-$ en 6); 3,08(m, 2H, $-CH_2-$ en 1); 3,23(m, 1H, 1H en 3a); 3,53(DD, J = 11 et 6,5, 1H, 1H du $-CH_2-$en 3); 3,6(S, 3H, $-OCH_3$); 4,43(D, J = 11, 1H, 1H en 7a); 3,8(S, 3H, $-OCH_3-$); 4,43(D, J = 11, 1H du $-CH_2-$ en 3); 6,8 à 7,5(m, 14H, aromatiques).
Spectre infra-rouge (KBr) : bandes identiques à celles de la forme A.

Le (méthoxy-2 phényl)-2 propionamide (RS) peut être préparé de la façon suivante:
A une solution refroidie à +5°C de 17,5 g d'acide (méthoxy-2 phényl)-2 propionique dans 200 $cm^3$ de dichlorométhane sec, on ajoute 16,2 g de N,N'-carbonyldiimidazole. On agite 30 minutes à +5°C puis on fait passer dans la solution un courant d'ammoniac pendant 1 heure, en maintenant la même température. Le mélange réactionnel, après agitation 2 heures à 20°C, est lavé par 200 $cm^3$ d'eau, séché sur sulfate de magnésium puis concentré à sec sous pression réduite (2,7 kPa). Le résidu est cristallisé dans l'oxyde d'isopropyle, les cristaux sont essorés, séchés sous pression réduite (2,7 kPa).On obtient 12,1 g de (méthoxy-2 phényl)-2 propionamide (RS) sous la forme d'un solide blanc fondant à 138°C.

L'acide (méthoxy-2 phényl)-2 propionique (RS) peut être préparé de la façon suivante:
A une solution refroidie à +10°C de 45,5 $cm^3$de diisopropylamine dans 250 $cm^3$de trétrahydrofuranne, on ajoute en 20 minutes 20 $cm^3$d'une solution 1,6M de n-butyllithium dans le tétrahydrofuranne. Après 10 minutes d'agitation, le mélange est amené à 0°C et on ajoute en 20 minutes une solution de (méthoxy-2 phényl)-2 acétique dans 100 $cm^3$ de tétrahydrofuranne. Après 30 minutes à 35°C, on ajoute 10 $cm^3$ d'iodure de méthyle et agite 1 heure à 35°C. Le mélange réactionnel est additionné de 150 $cm^3$ d'eau, dilué par de l'acétate d'éthyle. La phase aqueuse est séparée, acidifiée par de l'acide chlorhydrique 4N puis extraite par 2 fois 100 $cm^3$ d'acétate d'éthyle. La phase organique est séchée sur sulfate de magnésium, concentrée à sec sous pression réduite (2,7 kPa). Le résidu est cristallisé dans 100 $cm^3$ d'oxyde d'isopropyle, les cristaux sont essorés, séchés sous pression réduite (2,7 kPa).
On obtient 17,5 g d'acide (méthoxy-2 phényl)-2 propionique (RS) sous la forme d'un solide blanc fondant à 108°C.

## EXEMPLE 109

A une solution agitée de 5,2 g de N-(thiényl-2)méthyl (méthoxy-2 phényl)-2 acétamide dans 50 $cm^3$ de dichlorométhane anhydre, on ajoute 4 g de tétrafluoroborate de triéthyloxonium. Le mélange réactionnel est laissé 7 heures sous agitation, à température ambiante. Il est refroidit à +5°C, puis on ajoute une solution de 4,6 g de chlorhydrate de diphényl-7,7 perhydroisoindolone-4-(3aR,7aR) et de 5 $cm^3$ de triéthylamine dans 50 $cm^3$ de dichlorométhane. Le mélange réactionnel est ensuite laissé sous agitation 20 heures à température ambiante, puis chauffé 1 heure au reflux. Il est ensuite traité, après retour à température ambiante, par 100 $cm^3$ d'une solution aqueuse de carbonate de potassium à 10% ; la phase organique est lavée par 40 $cm^3$ d'eau distillée, séchée sur sulfate de magnésium et concentrée à sec sous pression réduite (2,7 kPa). Le résidu est chromatographié sur une colonne de gel d'alumine neutre Péchiney CBT1 (diamètre 5 cm, hauteur 40 cm) en éluant par un mélange de cyclohexane et d'acétate d'éthyle (50/50 en volumes) et en recueillant des fractions de 100 $cm^3$. Les fractions 4 à 13 sont réunies et concentrée à sec sous pression réduite (2,7 kPa). Le résidu est cristallisé dans 20 $cm^3$ d'isopropanol; les cristaux obtenus sont essorés,lavés par 10 $cm^3$ d'oxyde d'isopropyle et séchés. On obtient 1,9 g de[(méthoxy-2 phényl)-2 (thiényl-2 méthyl) imino-1 éthyl]-2 diphényl-7,7 perhydroisoindolone-4-(3aR,7aR), fondant à 88°C ;
$[\alpha]_D^{20}$ = -170° (c=1, méthanol).

Le N-(thiényl-2)méthyl (méthoxy-2 phényl)-2 acétamide peut être préparé de la façon suivante :
A une solution agitée et refroidie à +5°C de 8,3 g d'acide (méthoxy-2 phényl)-2 acétique dans 80 $cm^3$ de dichlorométhane anhydre, on ajoute 8,1 g de N,N'-carbonyldiimidazole ; le mélange réactionnel est laissé sous agitation 1 heure et demi, puis on ajoute 5,1 $cm^3$ de (thiényl-2)méthylamine. Le mélange réactionnel est laissé sous agitation 1 heure à +5°C, puis 2 heures à température ambiante. Il est ensuite lavé par 40 $cm^3$ d'eau distillée (2 fois) ; la solution organique est séchée sur sulfate de magnésium et concentrée à sec sous pression réduite (2,7 kPa). Le résidu solide obtenu est lavé par 30 $cm^3$ d'oxyde d'isopropyle, essoré et séché. On obtient 10,5 g de N-(thiényl-2)méthyl (méthoxy-2 phényl)-2 acétamide, fondant à 84°C.

EXEMPLE 110 à 136

En opérant comme décrit à l'exemple 4 à partir de la diphényl-7,7 phénylacétyl-2 perhydroisoindolone-4-(3aRS,7aRS) ou (3aR,7aR), de la diphényl-7,7 (fluoro-2 phényl)acétyl-2 perhydroisoindolone-4-(3aRS,7aRS) ou de la diphényl-7,7 (méthoxy-2 phényl)acétyl-2 perhydroisoindolone(3aRS,7aRS) ou (3aR,7aR), on prépare les produits suivants :

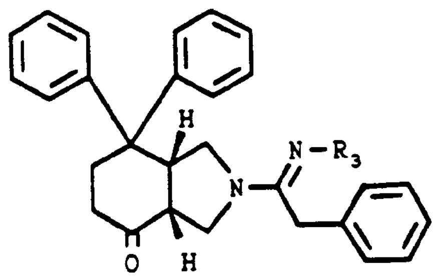

| N° de l'exemple | R3 | Forme | Point de fusion | R % |
|---|---|---|---|---|
| 110 | $-CH_2-C_6H_5$ p-$CH_3$ | (3aRS,7aRS) | 182°C | 63 |
| 111 | $-(CH_2)_2-N(CH_3)_2$ | (3aRS,7aRS) | 147°C | 53 |
| 112 | $-CH_2$ 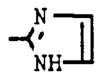 | (3aRS,7aRS) | 143°C | 50 |
| 113 | $-(CH_2)_6-N(CH_3)CH_2C_6H_5$ | (3aRS,7aRS) | 172°C | 56 |
| 114 | $-(CH_2)_6-N(CH_3)_2$ | (3aRS,7aRS) | 104°C | 5,6 |
| 115 | $-CH_2-CON(CH_3)-(CH_2)_2-N(CH_3)_2$ | (3aRS,7aRS) | 75°C | 58 |
| 116 | $-(CH_2)_5-CONH_2$ | (3aRS,7aRS) | 174°C | 48 |
| 117 | $-(CH_2)_5-COO(CH_2)_2-N(CH_3)_2$ | (3aRS,7aRS) | 102°C | 6,8 |
| 118 | $-(CH_2)_5-CON(CH_3)_2$ | (3aRS,7aRS) | 130°C | 27 |
| 119 | $-(CH_2)_4-N(CH_3)_2$ | (3aRS,7aRS) | 134°C | 32,5 |
| 120 | $-(CH_2)_5-N(CH_3)_2$ | (3aRS,7aRS) | 112°C | 28,6 |
| 121 | $-(CH_2)_4-NHCOCH_3$ | (3aRS,7aRS) | 114°C | 20,2 |
| 122 | $-(CH_2)_3-NHCOCH_3$ | (3aRS,7aRS) | 180°C | 20 |

| N° de l'exemple | R3 | Forme | R % | Spectre infra-rouge |
|---|---|---|---|---|
| 123 | $-(CH_2)_8-NHCOCH_3$ | (3aRS,7aRS) | 26 | 3300 $cm^{-1}$ ν NH<br>3100 - 3000 $cm^{-1}$ ν CH aromatiques<br>2925 $cm^{-1}$ $\nu_a CH_2$<br>2850 $cm^{-1}$ $\nu_s CH_2$<br>1715 $cm^{-1}$ ν C=O cetone<br>1655 $cm^{-1}$ ν C=O amide<br>1600;1585;1495;1445 $cm^{-1}$ noyaux aromatiques<br>1550 $cm^{-1}$ δ NH<br>750;700 $cm^{-1}$ Γ CH |
| 124 | $-(CH_2)_{10}-NHCOCH_3$ | (3aRS,7aRS) | 14,5 | 3400 $cm^{-1}$ ν NH<br>3100-3000 $cm^{-1}$ ν CH aromatiques<br>2925 $cm^{-1}$ $\nu_a CH_2$<br>2850 $cm^{-1}$ $\nu_s CH_2$<br>1715 $cm^{-1}$ ν C=O cetone<br>1650 $cm^{-1}$ ν C=O amide<br>1620 $cm^{-1}$ ν C=N<br>1600;1580;1495;1450 $cm^{-1}$ noyaux aromatiques<br>1550 $cm^{-1}$ δ NH<br>750;700 $cm^{-1}$ Γ CH |

| N° de l'exemple | R3 | Forme | R % | Spectre infra-rouge |
|---|---|---|---|---|
| 125 | $-(CH_2)_{12}-NHCOCH_3$ | (3aRS,7aRS) | 30 | 3300 $cm^{-1}$ ν NH<br>3100 - 3000 $cm^{-1}$ ν CH aromatiques<br>2925 $cm^{-1}$ $\nu_a CH_2$<br>2850 $cm^{-1}$ $\nu_s CH_2$<br>1715 $cm^{-1}$ ν C=O cetone<br>1655 $cm^{-1}$ ν C=O amide<br>1600;1585;1495;1445 $cm^{-1}$ noyaux aromatiques<br>1550 $cm^{-1}$ δ NH<br>750;700 $cm^{-1}$ γ CH |
| 126 | $-(CH_2)_6-NHCOOC(CH_3)_3$ | (3aRS,7aRS) | 79,8 | 3500 - 2250 $cm^{-1}$ ν OH+νNH+$\nu N^+H$<br>3100 - 3000 $cm^{-1}$ ν CH aromatiques<br>3000 - 2850 $cm^{-1}$ $\nu_a+\nu_s$ $CH_2+CH_3$<br>1715 $cm^{-1}$ ν C=O cetone + carbamate<br>1640 $cm^{-1}$ ν C=N + $\nu_a$ C=O carboxylate<br>1600;1585;1495;1445 $cm^{-1}$ noyaux aromatiques<br>1510 $cm^{-1}$ δ NH<br>1235 $cm^{-1}$ ν CO phenols<br>1165 $cm^{-1}$ ν CO carbamate<br>750;700 $cm^{-1}$ γ CH |

| N° de l'exemple | R3 | Forme | R % | Spectre infra-rouge |
|---|---|---|---|---|
| 127 | $-(CH_2)_6-NHCHO$ | (3aRS,7aRS) | 46,5 | 3240 $cm^{-1}$ ν NH<br>3100 - 3000 $cm^{-1}$ ν CH aromatiques<br>3000 - 2825 $cm^{-1}$ $\nu_a+\nu_s CH_2$<br>1710 $cm^{-1}$ ν C=O cetone<br>1670 $cm^{-1}$ ν C=O amide<br>1610 $cm^{-1}$ ν C=N<br>1595;1580;1495;1445 $cm^{-1}$ noyaux aromatiques<br>750;700 $cm^{-1}$ γ CH |
| 128 | $-(CH_2)_4-CON(CH_3)_2$ | (3aRS,7aRS) | 34,8 | 3100 - 3000 $cm^{-1}$ ν CH aromatiques<br>3000 - 2825 $cm^{-1}$ $\nu_a+\nu_s CH_2+CH_3$<br>1715 $cm^{-1}$ ν C=O cetone<br>1645 $cm^{-1}$ ν C=O amide<br>1615 $cm^{-1}$ ν C=N<br>1580;1495;1445 $cm^{-1}$ noyaux aromatiques<br>750;700 $cm^{-1}$ γ CH |

| N° de l'exemple | R3 | Forme | R % | Spectre infra-rouge |
|---|---|---|---|---|
| 129 | $-CH-CON-(CH_2)_2-N(CH_3)_2$ (S)<br>$\;\;|\;\;\;\;\;\;\;|$<br>$C_6H_5\;\;CH_3$ | (3aR, 7aR) | 35 | 3100 - 3000 $cm^{-1}$ $\nu$ CH aromatiques<br>3000 - 2850 $cm^{-1}$ $\nu_a+\nu_s$ $CH_2+CH_3$<br>2825;2770 $cm^{-1}$ $\nu$ CH $N(CH_3)_2$<br>1715 $cm^{-1}$ $\nu$ C=O cetone<br>1635 $cm^{-1}$ $\nu$ C=O amide<br>1605 $cm^{-1}$ $\nu$ C=N<br>1580;1495;1445 $cm^{-1}$ noyaux aromatiques<br>755;700 $cm^{-1}$ $\gamma$ CH |

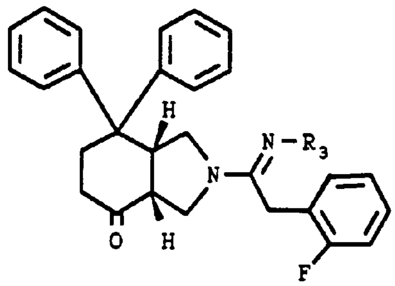

| N° de l'exemple | R3 | Forme | Point de fusion | R % |
|---|---|---|---|---|
| 130 | $-CH_2-C_6H_5$ | (3aRS,7aRS) | 162°C | 27 |
| 131 | -H | (3aRS,7aRS) | 170°C | 10 |

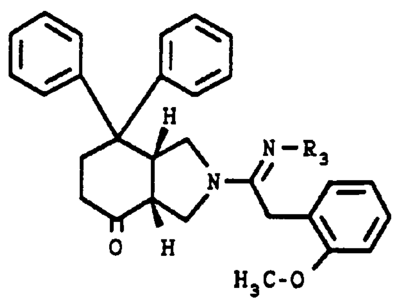

| N° de l'exemple | R3 | Forme | Point de fusion | R % |
|---|---|---|---|---|
| 132 | $-CH_2-C_6H_5$ | (3aR,7aR) | 110°C | 24 |
| 133 | $-CH_2$ 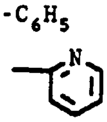 | (3aR,7aR) | 168°C | 9 |
| 134 | $-(CH_2)_6-NHCOCH_3$ | (3aRS,7aRS) | 152°C | 17,2 |

| N° de l'exemple | R3 | Forme | R % | Spectre infra-rouge |
|---|---|---|---|---|
| 135 | $-CH_2-CON(CH_3)-(CH_2)_2N(CH_3)_2$ | (3aRS,7aRS) | 47 | 3100 - 3000 $cm^{-1}$ ν CH aromatiques<br>3000 - 2750 $cm^{-1}$ $\nu_a+\nu_s$ $CH_2+CH_3$<br>2835 $cm^{-1}$ ν $CH_3$ O-$CH_3$<br>1715 $cm^{-1}$ ν C=O cetone<br>1640 $cm^{-1}$ ν C=O amide<br>1610 $cm^{-1}$ ν C=N<br>1495 $cm^{-1}$ noyaux aromatiques<br>1245 $cm^{-1}$ $\nu_a$CO ether<br>1030 $cm^{-1}$ $\nu_s$CO ether<br>755 - 750 $cm^{-1}$ γ CH |
| 136 | $-CH(C_6H_5)-COOH$ (S) | (3aR,7aR) | 4,8 | 3100 - 3000 $cm^{-1}$ ν CH aromatiques<br>3000 - 2850 $cm^{-1}$ $\nu_a+\nu_s$ $CH_2$<br>2840 $cm^{-1}$ ν CH φ-O-$CH_3$<br>1715 $cm^{-1}$ ν C=O cetone<br>1625 $cm^{-1}$ ν C=N + ν C=O carboxylate<br>1495;1450 $cm^{-1}$ noyaux aromatiques<br>1350 $cm^{-1}$ $\nu_s$ C=O carboxylate<br>1250 $cm^{-1}$ $\nu_a$ CO ether<br>1025 $cm^{-1}$ $\nu_s$ CO ether<br>750;730;700 $cm^{-1}$ CH γ |

EXEMPLE 137

En opérant comme décrit à l'exemple 104, à partir de chlorhydrate de diphényl-7,7 perhydroisoindolone-4-(3aR,7aR), on obtient la diphényl-7,7 [imino-1 (diméthylamino-2 phényl)-2 éthyl]-2 perhydroisoindolone-4-(3aR,7aR) fondant à 188°C, avec un rendement de 55 %.

La présente invention concerne également les compositions pharmaceutiques constituées par un produit

de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention peuvent être particulièrement utiles dans le traitement des douleurs d'origine traumatique, postchirurgicale, menstruelle, céphaliques, dans les traitements de l'anxiété, des psychoses, de la maladie de Parkinson, de la schizophrénie, de la maladie d'Alzeimer, dans les traitements myorelaxants, dans les traitements des manifestations spasmodiques, douloureuses et inflammatoires des voies digestives (colites ulcéreuses, syndrome du colon irritable, maladie de Crohn), des voies urinaires (cystites) et des voies respiratoires (asthme, rhinites) ou en gynécologie et dans les traitements des migraines.

Les nouveaux dérivés de l'isoindolone sont également utiles dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses et dans les troubles inflammatoires occulaires ou dentaires.

Les produits selon l'invention peuvent également trouver une application dans les traitements des troubles cardiovasculaires tels de l'hypotension.

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

Exemple

On prépare, selon la technique habituelle, des comprimés de produit actif ayant la composition suivante :

- [imino-1 (méthoxy-2 phényl)-2 éthyl]-2 diphényl-7,7 perhydroisoindolone-4-(3aR,7aR) 25 mg
- amidon 83 mg
- silice 30 mg
- stéarate de magnésium 3 mg

## Claims

### Revendications pour les Etats contractants suivants : AT, BE, CH, DE, DK, FR, GB, IT, LI, LU, NL, SE

1. Dérivé de l'isoindolone caractérisé en ce qu'il répond à la formule générale :

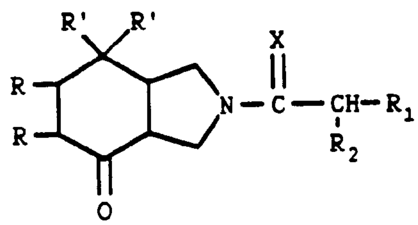

dans laquelle
   - les radicaux R sont identiques et représentent des atomes d'hydrogène ou forment ensemble une liaison,
   - les symboles R' sont identiques et représentent des radicaux phényle éventuellement substitués par un atome d'halogène ou par un radical méthyle en position 2 ou 3
   - le symbole X représente un atome d'oxygène, de soufre ou un radical N-$R_3$ pour lequel $R_3$ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces radicaux pouvant elles même porter un substituant dialcoylamino ou phényle) ou substitué par des radicaux phényle, phényle substitué (par halogène, alcoyle, alcoyloxy ou dialcoylamino), naphtyle, thiényle, furyle, pyridyle ou imidazolyle] ou un radical dialcoylamino,
   - le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 5 à 6 chaînons pouvant contenir un autre hétéroatome choisi parmi l'oxygène, le soufre ou l'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre et
   - le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino

   les radicaux alcoyle et acyle cités ci-dessus étant droits ou ramifiés et contenant 1 à 4 atomes de carbone, sous ses formes (3aR,7aR) ou (3aRS,7aRS) ou leurs mélanges ainsi que ses sels lorsqu'ils existent.

2. Un dérivé de l'isoindolone selon la revendication 1 caractérisé en ce que :
   - les radicaux R sont identiques et représentent des atomes d'hydrogène ou forment ensemble une liaison,
   - les symboles R' sont identiques et représentent des radicaux phényle éventuellement substitués par un atome de fluor ou de chlore ou par un radical méthyle en position 2 ou 3,
   - le symbole X représente un atome d'oxygène, de soufre ou un radical N-$R_3$ pour lequel $R_3$ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces radicaux pouvant elles même porter un substituant dialcoylamino ou phényle) ou substitué par des radicaux phényle, phényle substitué (par un atome de fluor ou de chlore, ou par un radical alcoyle,

alcoyloxy ou dialcoylamino), naphtyle, thiényle-2, furyle-2, pyridyle ou imidazolyle] ou un radical di-alcoylamino,

- le symbole $R_1$ représente un radical phényle éventuellement substitué par un ou plusieurs atomes de fluor, de chlore ou de brome ou radicaux hydroxy, alcoyle pouvant être éventuellement substitués (par des atomes d'halogène ou des radicaux amino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 6 chaînons pouvant contenir un autre atome d'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle saturé à 5 ou 6 chaînons) pouvant contenir en outre un autre atome d'oxygène, ou représente un radical cyclohexadiènyle, naphtyle ou hétérocyclyle mono ou polycyclique) saturé ou insaturé contenant 5 à 9 atomes de carbone et un ou plusieurs hétéroatomes choisis parmi l'azote ou le soufre, et
- le symbole $R_2$ représente un atome d'hydrogène ou de fluor ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino

les radicaux alcoyle et acyle cités ci-dessus étant droits ou ramifiés et contenant 1 à 4 atomes de carbone, sous ses formes (3aR,7aR) ou (3aRS,7aRS) ou leurs mélanges ainsi que ses sels lorsqu'ils existent.

3. Selon la revendication 1, l'[imino-1 (méthoxy-2 phényl)-2 éthyl]-2 diphényl-7,7 perhydroisoindolone-4 sous ses formes (3aR,7aR) ou (3aRS,7aRS) ainsi que ses sels.

4. Selon la revendication 1, la diphényl-7,7 [(diméthylamino-2 phényl)-2 acétyl]-2 perhydroisoindolone-4 sous ses formes (3aR,7aR) ou (3aRS,7aRS) ainsi que ses sels.

5. Selon la revendication 1, la diphényl-7,7 [(méthoxy-2 phényl)-2 propionyl-(R)]-2 perhydroisoindolone-4 sous ses formes (3aR,7aR) ou (3aRS,7aRS).

6. Selon la revendication 1, la {[(diméthylamino-3 propoxy)-2 phényl] acétyl}-2 diphényl-7,7 perhydroisoindolone4-sous ses formes (3aR,7aR) ou (3aRS,7aRS) ainsi que ses sels.

7. Selon la revendication 1, la [(S)-α-carboxy benzylimino-1 (méthoxy-2 phényl)-2 éthyl]-2 diphényl-7,7 perhydroisoindolone-4 sous ses formes (3aR,7aR) ou (3aRS,7aRS) ainsi que ses sels.

8. Procédé de préparation d'un nouveau dérivé de l'isoindolone selon la revendication 1, caractérisé en ce que l'on fait agir un acide ou un dérivé réactif de l'acide de formule générale

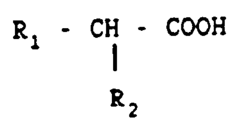

dans laquelle $R_1$ et $R_2$ sont définis comme dans la revendication 1, sur un dérivé de l'isoindolone de formule générale

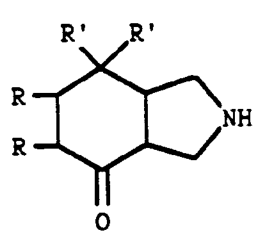

dans laquelle les symboles R et R' sont définis comme dans la revendication 1, puis transforme éventuellement l'amide obtenu en un thioamide ou en une amidine pour laquelle X représente un radical N-$R_3$, $R_3$ étant défini comme précédemment dans la revendication 1, et transforme éventuellement le produit

obtenu en un sel lorsqu'ils existent.

9. Procédé de préparation d'un dérivé de l'isoindolone selon la revendication 1, pour lequel $R_1$ et $R_2$ sont définis comme dans la revendication 1 à l'exception de représenter ou de porter des substituants hydroxy, amino, alcoylamino, dialcoylamino ou carboxy et R, R', $R_3$ et X sont définis comme dans la revendication 1, caractérisé en ce que l'on fait agir la tristriméthylsilylméthyl-1,3,5 triazine-1,3,5 et un fluorure d'acide de formule générale

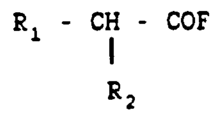

dans laquelle $R_1$ et $R_2$ sont définis comme ci-dessus, sur un dérivé de la cyclohexénone de formule générale

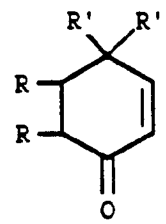

dans laquelle R et R' sont définis comme dans la revendication 1, puis éventuellement transforme l'amide obtenu en un thioamide ou en une amidine pour laquelle X est un radical N-$R_3$, $R_3$ étant défini comme dans la revendication 1.

10. Un procédé de préparation d'un dérivé de l'isoindolone selon la revendication 1, pour lequel le symbole $R_1$ représente un radical alcoyloxyphényle dont la partie alcoyle est éventuellement substituée, le symbole $R_2$ est autre qu'un radical hydroxy et les symboles R, R', $R_3$ et X sont définis comme dans la revendication 1, caractérisé en ce que l'on fait agir un dérivé halogéné de formule générale

$R_4$ - Hal

dans laquelle $R_4$ est un radical alcoyle, éventuellement substitué par un radical hydroxy protégé ou dialcoylamino dont les parties alcoyle peuvent éventuellement former avec l'atome d'azote auquel elles sont rattachées, un hétérocycle tel que défini dans la revendication 1 et Hal est un atome d'halogène, sur un dérivé de l'isoindolone selon la revendication 1, pour lequel $R_1$ est un radical hydroxyphényle, puis élimine le cas échéant le radical protecteur d'hydroxy.

11. Procédé de préparation d'un dérivé de l'isoindolone selon la revendication 1 pour lequel X est un radical N-$R_3$ et les symboles R, R', $R_1$, $R_2$ et $R_3$ sont définis comme dans la revendication 1, caractérisé en ce que l'on fait agir un produit de formule générale :

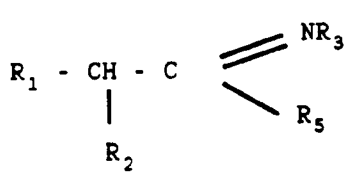

éventuellement à l'état de sel, dans laquelle $R_1$, $R_2$ et $R_3$ sont définis comme dans la revendication 1 et $R_5$ représente un radical alcoyloxy droit ou ramifié contenant 1 à 4 atomes de carbone ou un radical méthylthio, éthylthio, benzylthio ou alcoyloxycarbonylméthylthio ou, si $R_3$ est autre que l'atome d'hydrogène, $R_5$ représente un radical acyloxy contenant 1 à 4 atomes de carbone ou un atome de chlore, sur un dérivé de l'isoindolone de formule générale :

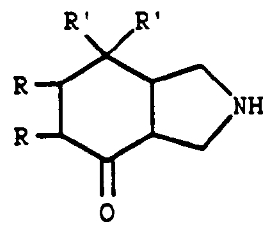

dans laquelle R et R' sont définis comme dans la revendication 1, puis, lorsqu'ils existent, transforme éventuellement le produit en un sel.

**12.** Composition pharmaceutique caractérisée en ce qu'elle contient au moins un produit selon la revendication 1, à l'état pur ou sous forme d'association avec un ou plusieurs adjuvants ou diluants compatibles et pharmaceutiquement acceptables.

**Revendication pour les Etats contractants suivants : ES, GR**

**1.** Procédé de préparation d'un nouveau dérivé de l'isoindolone de formule générale :

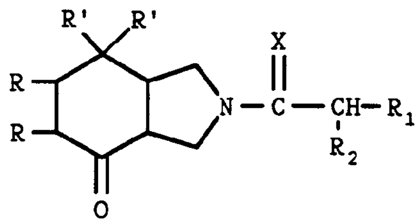

dans laquelle

- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3
- le symbole X représente un atome d'oxygène, de soufre ou un radical $N-R_3$ pour lequel $R_3$ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces radicaux pouvant elles même porter un substituant dialcoylamino ou phényle) ou substitué par des radicaux phényle, phényle substitué (par halogène, alcoyle, alcoyloxy ou dialcoylamino), naphtyle, thiényle, furyle, pyridyle ou imidazolyle] ou un radical dialcoylamino,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino), alcoyloxy ou alcoylthio pouvant être éventuellement substitués (par des radicaux hydroxy ou dialcoylamino dont les parties alcoyle peuvent former avec l'atome d'azote auquel elles sont rattachées, un hétérocycle à 5 à 6 chaînons pouvant contenir un autre hétéroatome choisi parmi l'oxygène, le soufre ou l'azote éventuellement substitué par un radical alcoyle), ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre et
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,

dans lequel les radicaux alcoyle et acyle sont droits ou ramifiés et contiennent 1 à 4 atomes de carbone,

sous ses formes (3aR,7aR) ou (3aRS,7aRS) ou leurs mélanges ainsi que ses sels lorsqu'ils existent caractérisé en ce que l'on fait agir un acide ou un dérivé réactif de l'acide de formule générale

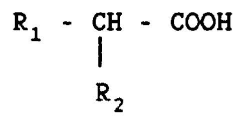

dans laquelle $R_1$ et $R_2$ sont définis comme ci-dessus, sur un dérivé de l'isoindolone de formule générale

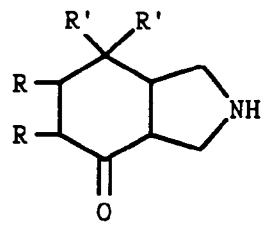

dans laquelle les symboles R et R' sont définis comme ci-dessus, puis transforme éventuellement l'amide obtenu en un thioamide ou en une amidine pour laquelle X représente un radical N-$R_3$, $R_3$ étant défini comme précédemment, ou bien
pour préparer un dérivé de l'isoindolone pour lequel $R_1$ et $R_2$ sont définis comme précédemment à l'exception de représenter ou de porter des substituants hydroxy, amino, alcoylamino, dialcoylamino ou carboxy et R, R', $R_3$ et X sont définis comme précédemment, caractérisé en ce que l'on fait agir la tristriméthylsilylméthyl-1,3,5 triazine-1,3,5 et un fluorure d'acide de formule générale

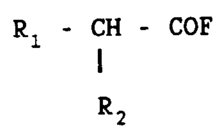

dans laquelle $R_1$ et $R_2$ sont définis comme ci-dessus, sur un dérivé de la cyclohexénone de formule générale

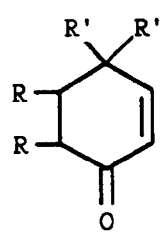

dans laquelle R et R' sont définis comme ci-dessus, puis éventuellement transforme l'amide obtenu en un thioamide ou en une amidine pour laquelle X est un radical N-$R_3$, $R_3$ étant défini comme ci-dessus, ou bien
pour la préparation d'un dérivé de l'isoindolone pour lequel le symbole $R_1$ représente un radical alcoyloxyphényle dont la partie alcoyle est éventuellement substituée, le symbole $R_2$ est autre qu'un radical hydroxy et les symboles R, R', $R_3$ et X sont définis comme précédemment, caractérisé en ce que l'on fait agir un dérivé halogéné de formule générale

$$R_4 - Hal$$

dans laquelle $R_4$ est un radical alcoyle, éventuellement substitué par un radical hydroxy protégé ou dialcoylamino dont les parties alcoyle peuvent éventuellement former avec l'atome d'azote auquel elles sont rattachées, un hétérocycle tel que défini précédemment et Hal est un atome d'halogène, sur le dérivé correspondant de l'isoindolone pour lequel $R_1$ est un radical hydroxyphényle, puis élimine le cas échéant le

radical protecteur d'hydroxy, ou bien
pour la préparation d'un dérivé de l'isoindolone pour lequel X est un radical N-$R_3$ et les symboles R, R', $R_1$, $R_2$ et $R_3$ sont définis comme précédemment, caractérisé en ce que l'on fait agir un produit de formule générale :

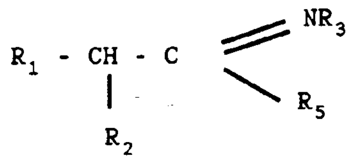

éventuellement à l'état de sel, dans laquelle $R_1$, $R_2$ et $R_3$ sont définis comme ci-dessus et $R_5$ représente un radical alcoyloxy droit ou ramifié contenant 1 à 4 atomes de carbone ou un radical méthylthio, éthylthio, benzylthio ou alcoyloxycarbonylméthylthio ou, si $R_3$ est autre que l'atome d'hydrogène, $R_5$ représente un radical acyloxy contenant 1 à 4 atomes de carbone ou un atome de chlore, sur un dérivé de l'isoindolone de formule générale :

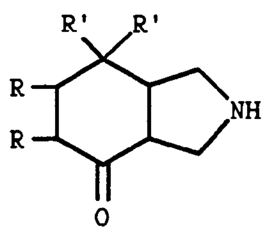

dans laquelle R et R' sont définis comme ci-dessus,
puis, lorsqu'ils existent, transforme éventuellement le produit obtenu en un sel.

**Patentansprüche**

**Patentansprüche für folgende Vertragsstaaten : AT, BE, CH, DE, DK, FR, GB, IT, LI, LU, NL, SE**

**1.** Isoindolonderivat, dadurch gekennzeichnet, daß es der allgemeinen Formel

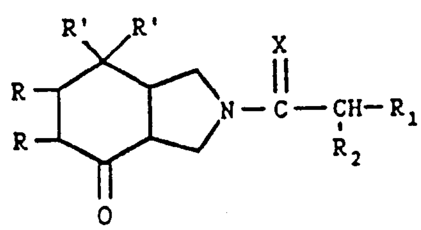

entspricht, worin

- die Reste R identisch sind und Wasserstoffatome darstellen oder gemeinsam eine Bindung bilden,
- die Symbole R' identisch sind und gegebenenfalls durch ein Halogenatom oder durch einen Methylrest in 2- oder 3-Stellung substituierte Phenylreste bedeuten,
- das Symbol X ein Sauerstoffatom, ein Schwefelatom oder einen Rest N-$R_3$, worin $R_3$ ein Wasserstoffatom, einen Alkylrest mit 1 bis 12 Kohlenstoffatomen und gegebenenfalls substituiert [durch einen oder mehrere Carboxy-, Dialkylamino, Acylamino-, Alkyloxycarbonyl-, Alkyloxycarbonylamino-, Carbamoyl-, Alkylcarbamoyl-, Dialkylcarbamoylreste (wobei die Alkylteile dieser Reste ihrerseits einen Dialkylamino- oder Phenylsubstituenten aufweisen können) oder substituiert durch Phenyl-, substituierte (durch Halogen, Alkyl, Alkyloxy oder Dialkylamino) Phenyl-, Naphthyl-, Thienyl-, Furyl-, Pyridyl- oder Imidazolylreste] oder einen Dialkylaminorest darstellt, bedeutet,
- das Symbol $R_1$ einen Phenylrest darstellt, der gegebenenfalls substituiert ist durch ein oder mehrere

Halogenatome oder Hydroxyreste, Alkylreste, die gegebenenfalls substituiert sein können (durch Halogenatome oder Aminoreste, Alkylamino- oder Dialkylaminoreste), Alkyloxy- oder Alkylthioreste, die gegebenenfalls substituiert sein können (durch Hydroxy- oder Dialkylaminoreste, deren Alkylteile mit dem Stickstoffatom, an das sie gebunden sind, einen Heterocyclus mit 5 bis 6 Kettengliedern bilden können, der ein weiteres Heteroatom, ausgewählt unter Sauerstoff, Schwefel oder Stickstoff, enthalten kann und gegebenenfalls durch einen Alkylrest substituiert ist), oder substituiert ist durch Amino-, Alkylamino-, Dialkylaminoreste, deren Alkylteile mit dem Stickstoffatom, an das sie gebunden sind, einen Heterocyclus, wie vorstehend definiert, bilden können, oder einen Cyclohexadienyl-, Naphthyl- oder gesättigten oder ungesättigten, mono- oder polycyclischen, heterocyclischen Rest mit 5 bis 9 Kohlenstoffatomen und einem oder mehreren Heteroatomen, ausgewählt unter Sauerstoff, Stickstoff oder Schwefel, bedeutet, und

- das Symbol $R_2$ ein Wasserstoff- oder Halogenatom oder einen Hydroxy-, Alkyl, Aminoalkyl-, Alkylaminoalkyl-, Dialkylaminoalkyl-, Alkoxy-, Alkylthio-, Acyloxy-, Carboxy-, Alkoxycarbonyl-, Dialkylaminoalkyloxycarbonyl-, Benzyloxycarbonyl-, Amino-, Acylamino- oder Alkyloxycarbonylaminorest bedeutet,

wobei die vorstehend genannten Alkyl- und Acylreste geradkettig oder verzweigt sind und 1 bis 4 Kohlenstoffatome aufweisen,
in ihren (3aR,7aR)- oder (3aRS,7aRS)-Formen oder in Form von deren Gemischen sowie ihre Salze, soweit sie existieren.

**2.** Isoindolonderivat gemäß Anspruch 1, dadurch gekennzeichnet, daß

- die Reste R identisch sind und Wasserstoffatome darstellen oder gemeinsam eine Bindung bilden,
- die Symbole R' identisch sind und gegebenenfalls durch ein Fluor- oder Chloratom oder durch einen Methylrest in 2- oder 3-Stellung substituierte Phenylreste bedeuten,
- das Symbol X ein Sauerstoffatom, ein Schwefelatom oder einen Rest N-$R_3$ darstellt, worin $R_3$ ein Wasserstoffatom, einen Alkylrest mit 1 bis 12 Kohlenstoffatomen, der gegebenenfalls substituiert ist [durch einen oder mehrere Carboxy-, Dialkylamino-, Acylamino-, Alkoxycarbonyl-, Alkoxycarbonylamino-, Carbamoyl-, Alkylcarbamoyl-, Dialkylcarbamoylreste (wobei die Alkylteile dieser Reste ihrerseits einen Dialkylamino- oder Phenylsubstituenten aufweisen können) oder substituiert ist durch Phenylreste, (durch ein Fluor- oder Chloratom oder durch einen Alkyl-, Alkoxy- oder Dialkylaminorest) substituierte Phenylreste, Naphthyl-, Thien-2-yl-, Fur-2-yl-, Pyridyl- oder Imidazolylreste] oder einen Dialkylaminorest bedeutet,
- das Symbol $R_1$ einen Phenylrest, gegebenenfalls substituiert durch ein oder mehrere Fluor-, Chlor- oder Bromatome oder Hydroxyreste, Alkylreste, die gegebenenfalls substituiert sein können (durch Halogenatome oder Aminoreste), Alkoxy- oder Alkylthioreste, die gegebenenfalls substituiert sind (durch Hydroxy- oder Dialkylaminoreste, deren Alkylteile mit dem Stickstoffatom, an das sie gebunden sind, einen Heterocyclus mit 6 Ringgliedern bilden können, der ein weiteres Stickstoffatom, welches gegebenenfalls durch einen Alkylrest substituiert ist, enthalten kann), oder substituiert durch Amino-, Alkylamino-, Dialkylaminoreste, deren Alkylteile mit dem Stickstoffatom, an das sie gebunden sind, einen gesättigten Heterocyclus mit 5 oder 6 Kettengliedern, der außerdem ein weiteres Sauerstoffatom enthalten kann, bilden können,oder einen Cyclohexadienyl-, Naphthyl- oder gesättigten oder ungesättigten, mono- oder polycyclischen, heterocyclischen Rest mit 5 bis 9 Kohlenstoffatomen und einem oder mehreren Heteroatomen, ausgewählt unter Stickstoff oder Schwefel, bedeutet, und
- das Symbol $R_2$ ein Wasserstoff- oder Fluoratom oder einen Hydroxy-, Alkyl-, Aminoalkyl-, Alkylaminoalkyl-, Dialkylaminoalkyl-, Alkyloxy-, Alkylthio-, Acyloxy-, Carboxy-, Alkyloxycarbonyl-, Dialkylaminoalkyloxycarbonyl-, Benzyloxycarbonyl-, Amino-, Acylamino- oder Alkyloxycarbonylaminorest bedeutet,

wobei die vorstehend genannten Alkyl- und Acylreste geradkettig oder verzweigt sind und 1 bis 4 Kohlenstoffatome besitzen,
in ihren (3aR,7aR)- oder (3aRS,7aRS)-Formen oder in Form von deren Gemischen sowie ihre Salze, soweit sie existieren.

**3.** Gemäß Anspruch 1 das 2-[1-Imino-2-(2-methoxyphenyl)-ethyl]-7,7-diphenyl-4-perhydroisoindolon in seinen (3aR, 7aR)- oder (3aRS,7aRS)-Formen sowie ihre Salze.

**4.** Gemäß Anspruch 1 das 7,7-Diphenyl-2-[2-(2-dimethylaminophenyl)-acetyl]-4-perhydroisoindolon in seinen (3aR,7aR)- oder (3aRS,7aRS)-Formen sowie ihre Salze.

5. Gemäß Anspruch 1 das 7,7-Diphenyl-2-[2-(2-methoxyphenyl)-(R)-propionyl]-4-perhydroisoindolon in seinen (3aR,7aR)- oder (3aRS,7aRS)-Formen.

6. Gemäß Anspruch 1 das 2-{[2-(3-Dimethylaminopropoxy)-phenyl]-acetyl}-7,7-diphenyl-4-perhydroisoindolon in seinen (3aR,7aR)- oder (3aRS,7aRS)-Formen sowie ihre Salze.

7. Gemäß Anspruch 1 das 2-[1-(S)-α-Carboxybenzylimino-2- (2-methoxyphenyl)-ethyl]-7,7-diphenyl-4-perhydroisoindolon in seinen (3aR,7aR)- oder (3aRS,7aRS)-Formen sowie ihre Salze.

8. Verfahren zur Herstellung eines neuen Isoindolonderivats gemäß Anspruch 1, dadurch gekennzeichnet, daß man eine Säure oder ein reaktives Derivat der Säure der allgemeinen Formel

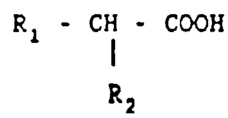

worin $R_1$ und $R_2$ die in Anspruch 1 angegebene Bedeutung besitzen, mit einem Isoindolonderivat der allgemeinen Formel

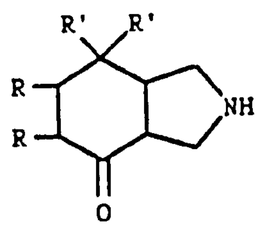

worin die Symbole R und R' wie in Anspruch 1 definiert sind, umsetzt, hiernach gegebenenfalls das erhaltene Amid in ein Thioamid oder in ein Amidin überführt, worin X einen Rest N-$R_3$ darstellt, wobei $R_3$ wie vorstehend in Anspruch 1 definiert ist, und gegebenenfalls das erhaltene Produkt in ein Salz überführt, soweit es existiert.

9. Verfahren zur Herstellung eines Isoindolonderivats gemäß Anspruch 1, worin $R_1$ und $R_2$ wie in Anspruch 1 definiert sind, mit der Ausnahme, daß sie Hydroxy-, Amino-, Alkylamino-, Dialkylamino- oder Carboxysubstituenten darstellen oder enthalten, und R, R', $R_3$ und X wie in Anspruch 1 definiert sind, dadurch gekennzeichnet, daß man 1,3,5-Tristrimethylsilylmethyl-1,3,5-triazin und ein Säurefluorid der allgemeinen Formel

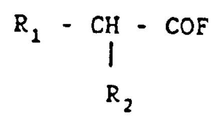

worin $R_1$ und $R_2$ wie vorstehend definiert sind, mit einem Cyclohexenonderivat der allgemeinen Formel

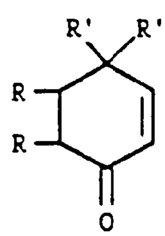

worin R und R' wie in Anspruch 1 definiert sind, umsetzt, wonach man gegebenenfalls das erhaltene Amid in ein Thioamid oder in ein Amidin überführt, worin X einen Rest N-$R_3$ darstellt, wobei $R_3$ wie in Anspruch 1 definiert ist.

**10.** Verfahren zur Herstellung eines Isoindolonderivats gemäß Anspruch 1, worin das Symbol $R_1$ einen Alkoxyphenylrest darstellt, dessen Alkylteil gegebenenfalls substituiert ist, das Symbol $R_2$ von einem Hydroxyrest verschieden ist und die Symbole R, R', $R_3$ und X wie in Anspruch 1 definiert sind, dadurch gekennzeichnet, daß man ein Halogenderivat der allgemeinen Formel

$$R_4 - Hal$$

worin $R_4$ einen Alkylrest, gegebenenfalls substituiert durch einen geschützten Hydroxyrest oder Dialkylaminorest, deren Alkylteile gegebenenfalls mit dem Stickstoffatom, an das sie gebunden sind, einen Heterocyclus, wie in Anspruch 1 definiert, bilden können, bedeutet und Hal für ein Halogenatom steht, mit einem Isoindolonderivat gemäß Anspruch 1 umsetzt, worin $R_1$ einen Hydroxyphenylrest darstellt, wonach man gegebenenfalls die Hydroxyschutzgruppe entfernt.

**11.** Verfahren zur Herstellung eines Isoindolonderivats gemäß Anspruch 1, worin X einen Rest N-$R_3$ darstellt und die Symbole R, R', $R_1$, $R_2$ und $R_3$ wie in Anspruch 1 definiert sind, dadurch gekennzeichnet, daß man ein Produkt der allgemeinen Formel

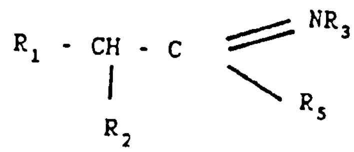

gegebenenfalls in Form des Salzes, worin $R_1$, $R_2$ und $R_3$ wie in Anspruch 1 definiert sind, und $R_5$ einen geradkettigen oder verzweigten Alkoxyrest mit 1 bis 4 Kohlenstoffatomen oder einen Methylthio-, Ethylthio-, Benzylthio- oder Alkoxycarbonylmethylthiorest darstellt, oder wenn $R_3$ von einem Wasserstoffatom verschieden ist, $R_5$ einen Acyloxyrest mit 1 bis 4 Kohlenstoffatomen oder ein Chloratom bedeutet, mit einem Isoindolonderivat der allgemeinen Formel

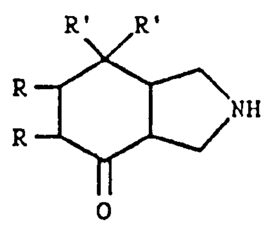

worin R und R' wie in Anspruch 1 definiert sind, umsetzt, wonach man gegebenenfalls das Produkt in ein Salz, soweit es existiert, überführt.

**12.** Pharmzeutische Zusammensetzung, dadurch gekennzeichnet, daß sie zumindest ein Produkt gemäß Anspruch 1 in reiner Form oder in Form einer Assoziation mit einem oder mehreren verträglichen und pharmazeutisch annehmbaren Adjuvanzien oder Verdünnungsmitteln enthält.

**Patentanspruch für folgende Vertragsstaaten : ES, GR**

**1.** Verfahren zur Herstellung eines Isoindolonderivats der allgemeinen Formel

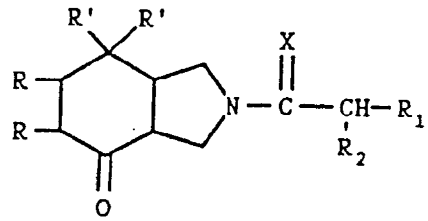

worin

- die Reste R identisch sind und Wasserstoffatome darstellen oder gemeinsam eine Bindung bilden,
- die Symbole R' identisch sind und gegebenenfalls durch ein Halogenatom oder durch einen Methylrest in 2- oder 3-Stellung substituierte Phenylreste bedeuten,
- das Symbol X ein Sauerstoffatom, ein Schwefelatom oder einen Rest N-$R_3$, worin $R_3$ ein Wasserstoffatom, einen Alkylrest mit 1 bis 12 Kohlenstoffatomen und gegebenenfalls substituiert [durch einen oder mehrere Carboxy-, Dialkylamino, Acylamino-, Alkyloxycarbonyl-, Alkyloxycarbonylamino-, Carbamoyl-, Alkylcarbamoyl-, Dialkylcarbamoylreste (wobei die Alkylteile dieser Reste ihrerseits einen Dialkylamino- oder Phenylsubstituenten aufweisen können) oder substituiert durch Phenyl-, substituierte (durch Halogen, Alkyl, Alkyloxy oder Dialkylamino) Phenyl-, Naphthyl-, Thienyl-, Furyl-, Pyridyl- oder Imidazolylreste] oder einen Dialkylaminorest darstellt, bedeutet,
- das Symbol $R_1$ einen Phenylrest darstellt, der gegebenenfalls substituiert ist durch ein oder mehrere Halogenatome oder Hydroxyreste, Alkylreste, die gegebenenfalls substituiert sein können (durch Halogenatome oder Aminoreste, Alkylamino- oder Dialkylaminoreste), Alkyloxy- oder Alkylthioreste, die gegebenenfalls substituiert sein können (durch Hydroxy- oder Dialkylaminoreste, deren Alkylteile mit dem Stickstoffatom, an das sie gebunden sind, einen Heterocyclus mit 5 bis 6 Kettengliedern bilden können, der ein weiteres Heteroatom, ausgewählt unter Sauerstoff, Schwefel oder Stickstoff, enthalten kann und gegebenenfalls durch einen Alkylrest substituiert ist), oder substituiert ist durch Amino-, Alkylamino-, Dialkylaminoreste, deren Alkylteile mit dem Stickstoffatom, an das sie gebunden sind, einen Heterocyclus, wie vorstehend definiert, bilden können, oder einen Cyclohexadienyl-, Naphthyl- oder gesättigten oder ungesättigten, mono- oder polycyclischen, heterocyclischen Rest mit 5 bis 9 Kohlenstoffatomen und einem oder mehreren Heteroatomen, ausgewählt unter Sauerstoff, Stickstoff oder Schwefel, bedeutet, und
- das Symbol $R_2$ ein Wasserstoff- oder Halogenatom oder einen Hydroxy-, Alkyl, Aminoalkyl-, Alkylaminoalkyl-, Dialkylaminoalkyl-, Alkoxy-, Alkylthio-, Acyloxy-, Carboxy-, Alkoxycarbonyl-, Dialkylaminoalkyloxycarbonyl-, Benzyloxycarbonyl-, Amino-, Acylamino- oder Alkyloxycarbonylaminorest bedeutet,

wobei die vorstehend genannten Alkyl- und Acylreste geradkettig oder verzweigt sind und 1 bis 4 Kohlenstoffatome aufweisen,

in ihren (3aR,7aR)- oder (3aRS,7aRS)-Formen oder in Form von deren Gemischen sowie von deren Salzen, soweit sie existieren, dadurch gekennzeichnet, daß man eine Säure oder ein reaktives Derivat der Säure der allgemeinen Formel

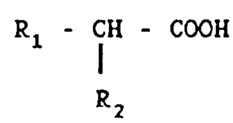

worin $R_1$ und $R_2$ wie vorstehend definiert sind, mit einem Isoindolonderivat der allgemeinen Formel

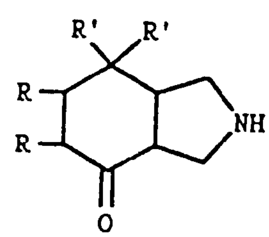

worin die Symbole R und R' wie vorstehend definiert sind, umsetzt, wonach man gegebenenfalls das erhaltene Amid in ein Thioamid oder in ein Amidin überführt, worin X einen Rest N-$R_3$ bedeutet, wobei $R_3$ wie vorstehend definiert ist, oder

zur Herstellung eines Isoindolonderivats, worin $R_1$ und $R_2$ wie vorstehend definiert sind, mit Ausnahme dessen, daß sie Hydroxy-, Amino-, Alkylamino-, Dialkylamino- oder Carboxysubstituenten darstellen oder enthalten, und R, R', $R_3$ und X wie vorstehend definiert sind, dadurch gekennzeichnet, daß man das 1,3,5-Tristrimethylsilylmethyl-1,3,5-triazin und ein Säurefluorid der allgemeinen Formel

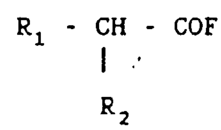

worin $R_1$ und $R_2$ wie vorstehend definiert sind, mit einem Cyclohexenonderivat der allgemeinen Formel

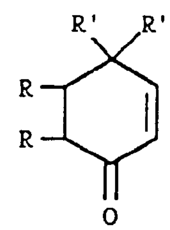

worin R und R' wie vorstehend definiert sind, umsetzt, wonach man gegebenenfalls das erhaltene Amid in ein Thioamid oder in ein Amidin überführt, worin X einen Rest N-$R_3$ bedeutet, wobei $R_3$ wie vorstehend definiert ist, oder

zur Herstellung eines Isoindolonderivats, worin das Symbol $R_1$ einen Alkoxyphenylrest darstellt, dessen Alkylteil gegebenenfalls substituiert ist, das Symbol $R_2$ von einem Hydroxyrest verschieden ist und die Symbole R, R', $R_3$ und X wie vorstehend definiert sind, dadurch gekennzeichnet, daß man ein Halogenderivat der allgemeinen Formel

$$R_4 - Hal$$

worin $R_4$ einen Alkylrest, gegebenenfalls substituiert durch einen geschützten Hydroxyrest oder Dialkylaminorest, dessen Alkylteile gegebenenfalls mit dem Stickstoffatom, an das sie gebunden sind, einen Heterocyclus, wie vorstehend definiert, bilden können, bedeutet und Hal für ein Halogenatom steht, mit dem entsprechenden Isoindolonderivat, worin $R_1$ einen Hydroxyphenylrest darstellt, umsetzt und hiernach gegebenenfalls die Hydroxyschutzgruppe entfernt, oder

zur Herstellung eines Isoindolonderivats, worin X einen Rest N-$R_3$ darstellt und die Symbole R, R', $R_1$, $R_2$ und $R_3$ wie vorstehend definiert sind, dadurch gekennzeichnet, daß man ein Produkt der allgemeinen Formel

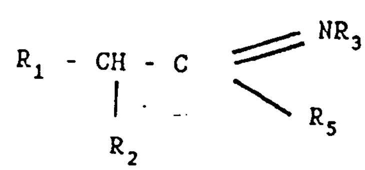

gegebenenfalls in Form des Salzes, worin $R_1$, $R_2$ und $R_3$ wie vorstehend definiert sind und $R_5$ einen geradkettigen oder verzweigten Alkoxyrest mit 1 bis 4 Kohlenstoffatomen oder einen Methylthio-, Ethylthio-, Benzylthio- oder Alkoxycarbonylmethylthiorest darstellt, oder wenn $R_3$ von einem Wasserstoffatom verschieden ist, $R_5$ einen Acyloxyrest mit 1 bis 4 Kohlenstoffatomen oder ein Chloratom bedeutet, mit einem Isoindolonderivat der allgemeinen Formel

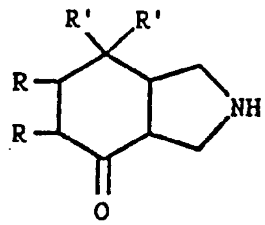

worin R und R' wie vorstehend definiert sind, umsetzt,
wonach man gegebenenfalls das erhaltene Produkt, soweit es existiert, in ein Salz überführt.

## Claims

### Claims for the following Contracting States : AT, BE, CH, DE, DK, FR, GB, IT, LI, LU, NL, SE

1. Isoindolone derivative characterized in that it corresponds to the general formula:

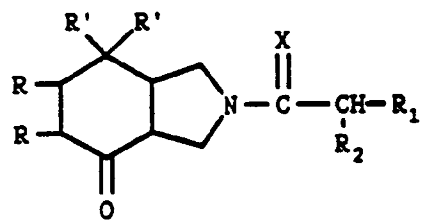

in which
- the radicals R are identical and represent hydrogen atoms or together form a bond,
- the symbols R' are identical and represent phenyl radicals optionally substituted by a halogen atom or by a methyl radical in position 2 or 3,
- the symbol X represents an oxygen or sulphur atom or a radical N-$R_3$, for which $R_3$ is a hydrogen atom, an alkyl radical containing 1 to 12 carbon atoms and optionally substituted [by one or more carboxyl, dialkylamino, acylamino, alkoxycarbonyl, alkoxycarbonylamino, carbamoyl, alkylcarbamoyl or dialkylcarbamoyl radicals (the alkyl portions of these radicals being able themselves to carry a dialkylamino or phenyl substituent) or substituted by phenyl, substituted phenyl (substituted by halogen, alkyl, alkoxy or dialkylamino), naphthyl, thienyl, furyl, pyridyl or imidazolyl radicals] or a dialkylamino radical,
- the symbol $R_1$ represents a phenyl radical optionally substituted by one or more halogen atoms or hydroxyl or alkyl radicals which can optionally be substituted (by halogen atoms or amino, alkylamino or dialkylamino radicals) or alkoxy or alkylthio radicals, which can optionally be substituted (by hydroxyl or dialkylamino radicals in which the alkyl parts can form, with the nitrogen atom to which they are attached, a 5- to 6-membered heterocycle which can contain another hetero-atom chosen from oxygen, sulphur or nitrogen, optionally substituted by an alkyl radical), or substituted by amino, alkylamino or dialkylamino radicals in which the alkyl parts can form, with the nitrogen atom to which they are attached, a heterocycle as defined above, or represents a cyclohexadienyl or naphthyl radical or a saturated or unsaturated monocyclic or polycyclic heterocycle containing 5 to 9 carbon atoms and one or more hetero-atoms chosen from oxygen, nitrogen or sulphur, and
- the symbol $R_2$ represents a hydrogen or halogen atom or a hydroxyl, alkyl, aminoalkyl, alkylaminoalkyl, dialkylaminoalkyl, alkoxy, alkylthio, acyloxy, carboxyl, alkoxycarbonyl, dialkylaminoalkoxycarbonyl, benzyloxycarbonyl, amino, acylamino or alkoxycarbonylamino radical,

the abovementioned alkyl and acyl radicals being straight-chain or branched and containing 1 to 4 carbon atoms,
in its (3aR,7aR) or (3aRS,7aRS) forms or their mixtures as well as its salts where such exist.

2. An isoindolone derivative according to claim 1, characterized in that:
- the radicals R are identical and represent hydrogen atoms or together form a bond,
- the symbols R' are identical and represent phenyl radicals optionally substituted by a fluorine or chlorine atom or by a methyl radical in position 2 or 3,

- the symbol X represents an oxygen or sulphur atom or a radical N-$R_3$ for which $R_3$ is a hydrogen atom, an alkyl radical containing 1 to 12 carbon atoms and optionally substituted [by one or more carboxyl, dialkylamino, acylamino, alkoxycarbonyl, alkoxycarbonylamino, carbamoyl, alkylcarbamoyl or dialkylcarbamoyl radicals (the alkyl portions of these radicals being able themselves to carry a dialkylamino or phenyl substitutent) or substituted by phenyl, substituted phenyl (substituted by a fluorine or chlorine atom or by an alkyl, alkoxy or dialkylamino radical), naphthyl, 2-thienyl, 2-furyl, pyridyl or imidazolyl radicals] or a dialkylamino radical,
- the symbol $R_1$ represents a phenyl radical optionally substituted by one or more fluorine, chlorine or bromine atoms or hydroxyl radicals, or alkyl radicals, which can optionally be substituted (by halogen atoms or amino radicals), or alkoxy or alkylthio radicals, which can optionally be substituted (by hydroxyl or dialkylamino radicals in which the alkyl parts can form, with the nitrogen atom to which they are attached, a 6-membered heterocycle which can contain another nitrogen atom optionally substituted by an alkyl radical), or substituted by amino, alkylamino or dialkylamino radicals, in which the alkyl parts can form, with the nitrogen atom to which they are attached, a saturated 5- or 6-membered heterocycle, which can also contain another oxygen atom, or represents a cyclohexadienyl or naphthyl radical or a saturated or unsaturated monocyclic or polycyclic heterocyclic radical containing 5 to 9 carbon atoms and one or more hetero-atoms chosen from nitrogen or sulphur, and
- the symbol $R_2$ represents a hydrogen or fluorine atom or a hydroxyl, alkyl, aminoalkyl, alkylaminoalkyl, dialkylaminoalkyl, alkoxy, alkylthio, acyloxy, carboxyl, alkoxycarbonyl, dialkylaminoalkoxycarbonyl, benzyloxycarbonyl, amino, acylamino or alkoxycarbonylamino radical,

the abovementioned alkyl and acyl radicals being straight-chain or branched and containing 1 to 4 carbon atoms,

in its (3aR,7aR) or (3aRS,7aRS) forms or their mixtures as well as its salts where such exist.

3. According to claim 1, 2-[1-imino-2-(2-methoxyphenyl)ethyl]-7,7-diphenyl-4-perhydroisoindolone in its (3aR,7aR) or (3aRS,7aRS) forms as well as its salts.

4. According to claim 1, 7,7-diphenyl-2-[2-(2-dimethylaminophenyl)acetyl]-4-perhydroisoindolone in its (3aR,7aR) or (3aRS,7aRS) forms as well as its salts.

5. According to claim 1, 7,7-diphenyl-2-[(R)-2-(2-methoxyphenyl)propionyl]-4-perhydroisoindolone in its (3aR,7aR) or (3aRS,7aRS) forms.

6. According to claim 1, 2-{[2-(3-dimethylaminopropoxy)phenyl]acetyl}-7,7-diphenyl-4-perhydroisoindolone in its (3aR,7aR) or (3aRS,7aRS) forms as well as its salts.

7. According to claim 1, 2-[(S)-α-1-carboxybenzylimino-2-(2-methoxyphenyl)ethyl]-7,7-diphenyl-4-perhydroisoindolone in its (3aR,7aR) or (3aRS,7aRS) forms as well as its salts.

8. Process for the preparation of a new isoindolone derivative according to claim 1, characterized in that an acid or a reactive derivative of the acid of general formula

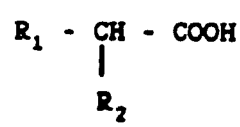

in which $R_1$ and $R_2$ are defined as in claim 1, is allowed to act on an isoindolone derivative of general formula

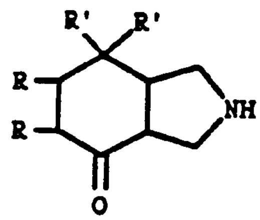

in which the symbols R and R' are defined as in claim 1, and, if necessary, the amide obtained is then converted to a thioamide or to an amidine for which X represents a radical N-$R_3$, $R_3$ being defined as above in claim 1, and, if necessary, the product obtained is converted to a salt where such exist.

**9.** Process for the preparation of an isoindolone derivative according to claim 1, for which $R_1$ and $R_2$ are defined as in claim 1, with the exception of representing or carrying hydroxyl, amino, alkylamino, dialkylamino or carboxyl substituents, and R, R', $R_3$ and X are defined as in claim 1, characterized in that 1,3,5-tris-trimethylsilylmethyl-1,3,5-triazine and an acid fluoride of general formula

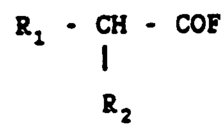

in which $R_1$ and $R_2$ are defined as above, are allowed to act on a cyclohexenone derivative of general formula

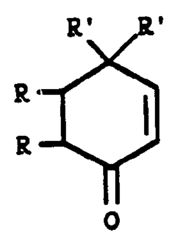

in which R and R' are defined as in claim 1, and, if necessary, the amide obtained is then converted to a thioamide or to an amidine for which X is a radical N-$R_3$, $R_3$ being defined as in claim 1.

**10.** A process for the preparation of an isoindolone derivative according to claim 1, for which the symbol $R_1$ represents an alkoxyphenyl radical in which the alkyl part is optionally substituted, the symbol $R_2$ is other than a hydroxyl radical and the symbols R, R', $R_3$ and X are defined as in claim 1, characterized in that a halogenated derivative of general formula

$$R_4 - Hal$$

in which $R_4$ is an alkyl radical optionally substituted by a protected hydroxyl radical or dialkylamino in which the alkyl parts can optionally form, with the nitrogen atom to which they are attached, a heterocycle as defined in claim 1, and Hal is a halogen atom, is allowed to act on an isoindolone derivative according to claim 1, for which $R_1$ is a hydroxyphenyl radical, and, if necessary, the hydroxyl protective radical is then removed.

**11.** Process for the preparation of an isoindolone derivative according to claim 1, for which X is a radical N-$R_3$ and the symbols R, R', $R_1$, $R_2$ and $R_3$ are defined as in claim 1, characterized in that a product of general formula:

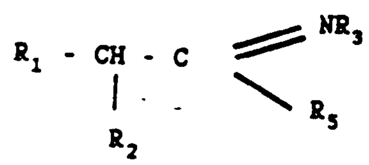

optionally in the form of a salt, in which $R_1$, $R_2$ and $R_3$ are defined as in claim 1 and $R_5$ represents a straight-chain or branched alkoxy radical containing 1 to 4 carbon atoms or a methylthio, ethylthio, benzylthio or alkoxycarbonylmethylthio radical or, if $R_3$ is other than a hydrogen atom, $R_5$ represents an acyloxy radical containing 1 to 4 carbon atoms or a chlorine atom, is allowed to act on an isoindolone derivative of general formula:

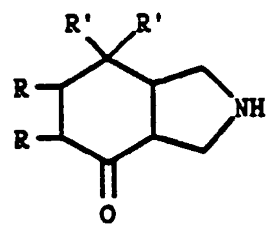

in which R and R' are defined as in claim 1, and, if necessary, the product is then converted to a salt, if such exists.

**12.** Pharmaceutical composition, characterized in that it contains at least one product according to claim 1, in the pure state or in the form of a combination with one or more compatible and pharmaceutically acceptable adjuvants or diluents.

**Claim for the following Contracting States : ES, GR**

**1.** Process for the preparation of an isoindolone derivative of the general formula:

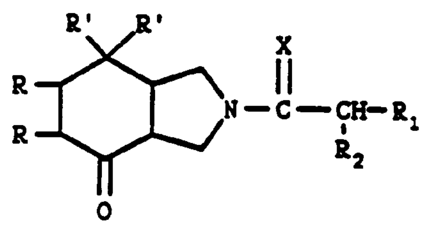

in which

- the radicals R are identical and represent hydrogen atoms or together form a bond,
- the symbols R' are identical and represent phenyl radicals optionally substituted by a halogen atom or by a methyl radical in position 2 or 3,
- the symbol X represents an oxygen or sulphur atom or a radical N-$R_3$, for which $R_3$ is a hydrogen atom, an alkyl radical containing 1 to 12 carbon atoms and optionally substituted [by one or more carboxyl, dialkylamino, acylamino, alkoxycarbonyl, alkoxycarbonylamino, carbamoyl, alkylcarbamoyl or dialkylcarbamoyl radicals (the alkyl portions of these radicals being able themselves to carry a dialkylamino or phenyl substituent) or substituted by phenyl, substituted phenyl (substituted by halogen, alkyl, alkoxy or dialkylamino), naphthyl, thienyl, furyl, pyridyl or imidazolyl radicals] or a dialkylamino radical,
- the symbol $R_1$ represents a phenyl radical optionally substituted by one or more halogen atoms or hydroxyl or alkyl radicals which can optionally be substituted (by halogen atoms or amino, alkylamino or dialkylamino radicals) or alkoxy or alkylthio radicals, which can optionally be substituted (by hydroxyl or dialkylamino radicals in which the alkyl parts can form, with the nitrogen atom to which they are attached, a 5- to 6-membered heterocycle which can contain another hetero-atom chosen from

oxygen, sulphur or nitrogen, optionally substituted by an alkyl radical), or substituted by amino, alkylamino or dialkylamino radicals in which the alkyl parts can form, with the nitrogen atom to which they are attached, a heterocycle as defined above, or represents a cyclohexadienyl or naphthyl radical or a saturated or unsaturated monocyclic or polycyclic heterocycle containing 5 to 9 carbon atoms and one or more hetero-atoms chosen from oxygen, nitrogen or sulphur, and

- the symbol $R_2$ represents a hydrogen or halogen atom or a hydroxyl, alkyl, aminoalkyl, alkylaminoalkyl, dialkylaminoalkyl, alkoxy, alkylthio, acyloxy, carboxyl, alkoxycarbonyl, dialkylaminoalkoxycarbonyl, benzyloxycarbonyl, amino, acylamino or alkoxycarbonylamino radical,

in which the alkyl and acyl radicals are straight-chain or branched and contain 1 to 4 carbon atoms, in its (3aR,7aR) or (3aRS,7aRS) forms or their mixtures as well as its salts where such exist characterized in that an acid or a reactive derivative of the acid of general formula

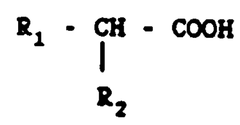

in which $R_1$ and $R_2$ are defined as above, is allowed to act on an isoindolone derivative of general formula

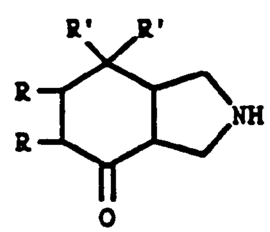

in which the symbols R and R' are defined as above, and, if necessary, the amide obtained is then converted to a thioamide or to an amidine for which X represents a radical $N-R_3$, $R_3$ being defined as above, or else,

to prepare an isoindolone derivative for which $R_1$ and $R_2$ are defined as above, with the exception of representing or carrying hydroxyl, amino, alkylamino, dialkylamino or carboxyl substituents, and R, R', $R_3$ and X are defined as above, characterized in that 1,3,5-tris-trimethylsilylmethyl-1,3,5-triazine and an acid fluoride of general formula

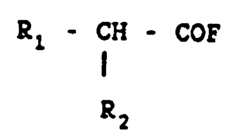

in which $R_1$ and $R_2$ are defined as above, are allowed to act on a cyclohexenone derivative of general formula

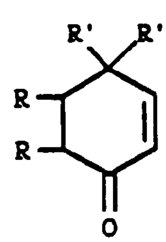

in which R and R' are defined as above, and, if necessary, the amide obtained is then converted to a thioamide or to an amidine for which X is a radical $N-R_3$, $R_3$ being defined as above, or else

for the preparation of an isoindolone derivative for which the symbol $R_1$ represents an alkoxyphenyl radical

in which the alkyl part is optionally substituted, the symbol $R_2$ is other than a hydroxyl radical and the symbols R, R', $R_3$ and X are defined as above, characterized in that a halogenated derivative of general formula

$$R_4 - Hal$$

in which $R_4$ is an alkyl radical optionally substituted by a protected hydroxyl radical or dialkylamino in which the alkyl parts can optionally form, with the nitrogen atom to which they are attached, a heterocycle as defined above, and Hal is a halogen atom, is allowed to act on the corresponding isoindolone derivative for which $R_1$ is a hydroxyphenyl radical, and, if necessary, the hydroxyl protective radical is then removed, or else

for the preparation of an isoindolone derivative for which X is a radical N-$R_3$ and the symbols R, R', $R_1$, $R_2$ and $R_3$ are defined as above, characterized in that a product of general formula:

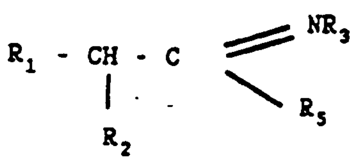

optionally in the form of a salt, in which $R_1$, $R_2$ and $R_3$ are defined as above and $R_5$ represents a straight-chain or branched alkoxy radical containing 1 to 4 carbon atoms or a methylthio, ethylthio, benzylthio or alkoxycarbonylmethylthio radical or, if $R_3$ is other than a hydrogen atom, $R_5$ represents an acyloxy radical containing 1 to 4 carbon atoms or a chlorine atom, is allowed to act on an isoindolone derivative of general formula:

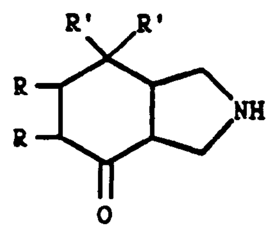

in which R and R' are defined as above, and, if necessary, the product is then converted to a salt, if such exists.